# EUROPEAN PATENT APPLICATION

(11) **EP 4 710 922 A2**
(43) Date of publication of application: **18.03.2026**
(21) Application number: 26154854.9
(22) Date of filing: 30.07.2020
(51) Int. Cl.: A61K 31/4184

(54) **ARYL HYDROCARBON RECEPTOR ACTIVATORS**

(30) Priority: 30.07.2019 US 201962880478 P
(62) Divisional of application: 20847828.9
(71) Applicant: Oregon State University, Corvallis, OR 97331 (US)
(72) Inventor: KOLLURI, Siva Kumar, Corvallis, OR 97331 (US); KERKVLIET, Nancy I., Corvallis, OR 97331 (US); BERNALES, Sebastian, Corvallis, OR 97331 (US); CHAKRAVARTY, Jit, Corvallis, OR 97331 (US); PUJALA, Brahmam, Corvallis, OR 97331 (US); KHAN, Pasha, Corvallis, OR 97331 (US); KUMAR, Varun, Corvallis, OR 97331 (US); DANODIA, Abhinandan, Corvallis, OR 97331 (US); URETA, Gonzalo, Corvallis, OR 97331 (US)
(74) Representative: Mathys & Squire

(57) **Abstract**

Small molecule AhR ligands are disclosed. The ligands can induce the differentiation of Tr1 cells to suppress pathogenic immune responses without inducing nonspecific immune suppression. Methods of treatment of autoimmune diseases using the AhR ligands are also disclosed.

## Description

### CROSS-REFERENCE TO RELATED APPLICATION

This application claims the benefit of U.S. Provisional Application No. 62/880,478 filed July 30, 2019 expressly incorporated hereby in its entirety.

### STATEMENT OF GOVERNMENT LICENSE RIGHTS

This invention was made with Government support under R01ES016651 awarded by National Institutes of Health. The Government has certain rights in the invention.

### BACKGROUND OF THE INVENTION

Autoimmune disease is caused by a failure of the immune system to recognize the difference between healthy cells and cells that have been altered as a result of infectious disease or mutations leading to cancer. When the immune system attacks healthy cells, the resulting damage may affect one or several tissue types or organs. For example, type 1 diabetes (T1D), also known as diabetes mellitus type 1, is an autoimmune disease in which cytotoxic T-lymphocytes (CTL) attack and destroy the insulin-producing beta cells (β-cells) in the pancreas. Current management of T1D involves administration of insulin and various formulations of insulin. Currently, an estimated 80,000 children develop TIDM each year and approximately 3 million people have TID in the United States. Complications from TID include heart disease, stroke, kidney failure, foot ulcers, and diabetic retinopathy. In addition, insulin treatment can lead to low blood sugar, or hypoglycemia, which can result in coma and death. Another immune-mediated disease, graft versus host disease (GVHD), can occur after a tissue transplant or blood transfusion. GVHD develops when grafted donor T cells recognize the recipient's cells as foreign and differentiate into CTL that attack a recipient's healthy cells. GVHD can cause a range of symptoms from mild to severe, including death.

Current immune-suppressing drug therapies for GVHD, TID, and other autoimmune disorders act by nonspecifically inhibiting cellular proliferation or by suppressing inflammatory responses; the intended target cells (e.g. CTL) that are responsible for the autoimmune disease are suppressed as well. However, such nonspecific immune suppression results in undesirable side effects including an increased risk of infection and certain cancers. Thus, conventional immunosuppressive treatments of autoimmune diseases fail to provide long-term remission without severe side effects.

Targeting T cells is a promising therapeutic strategy for the prevention or treatment of autoimmune diseases. The aryl hydrocarbon receptor (AhR) represents a potential drug target as a ligand-activated transcription factor that specifically targets T cell differentiation rather than inhibiting cellular proliferation. Activation of the AhR has been shown to prevent the development of T1D in the NOD mouse model, and to suppress the development of murine GVHD, implicating the AhR as a novel therapeutic target.

Two potent AhR ligands, 10- and 11-chloro-7H-benzimidazo[2,1-a]benzo[de]-Isoquinolin-7-one (11-Cl-BBQ) have been identified that suppress the development of T1D and GVHD in murine models. Acute or chronic treatment of mice with these compounds produced no overt toxicity at the therapeutic dose. Furthermore, extensive studies have shown that activation of the AhR by these compounds in T cells drives their differentiation into type 1 regulatory T cells (Tr1 cells) that suppress pathogenic T cell responses.

A need exists for non-toxic, small molecule AhR ligands with favorable pharmacokinetic properties that can induce the differentiation of Tr1 cells to suppress pathogenic immune responses without inducing nonspecific immune suppression.

### SUMMARY

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

In one aspect, provided herein is a compound of the formula: 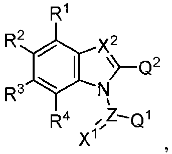 a tautomer thereof, a stereoisomer thereof, a hydrate thereof, or a pharmaceutically acceptable salt thereof, wherein:
Q¹ is an optionally substituted C6-C10 aryl; optionally substituted C5-C10 heteroaryl; optionally substituted C5-C10 heterocyclyl; optionally substituted C1-C10 alkyl, or optionally substituted C3-C10 cycloalkyl;
Q² is an optionally substituted C6-C14 aryl; optionally substituted C5-C10 heteroaryl; optionally substituted C5-C10 heterocyclyl; optionally substituted C1-C10 alkyl, or optionally substituted C3-C10 cycloalkyl;
Z is C, S(O), 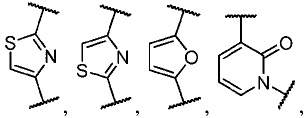 or Z is CH when X1 is absent;
X¹ is absent, O, NH, S, or X1 is 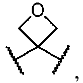 wherein the wavy lines denote points of attachment to Z;
X² is N, CCl, CF, CBr, CI, CCN, CCONH2, CCOOH, or CH;
R¹, R², R³, and R⁴ are independently H, halogen, CN, optionally substituted C1-C10 alkyl, optionally substituted C3-C10 cycloalkyl, optionally substituted C1-C6 alkoxy, SO₂R⁵, CO2R⁵, or CONR⁵R⁶, or any one of R¹ and R², R² and R³, and R³ and R⁴ pairs, together with the carbon atoms to which they are attached, forms an optionally substituted a five- or six-membered cycloalkenyl, heterocyclenyl, aryl or heteroaryl; and
R⁵ and R⁶ are independently H, optionally substituted C1-C10 alkyl, or optionally substituted C3-C10 cycloalkyl, or R⁵ and R⁶, together with the nitrogen atom to which they are attached, form an optionally substituted 5-membered ring or an optionally substituted 6-membered ring.

In another aspect, provided herein is a compound of the formula: 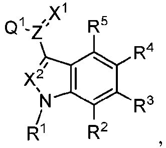 a tautomer thereof, a stereoisomer thereof, a hydrate thereof, or a pharmaceutically acceptable salt thereof, wherein:
Q¹ is an optionally substituted C6-C10 aryl; optionally substituted C5-C10 heteroaryl; optionally substituted C5-C10 heterocyclyl; optionally substituted C1-C10 alkyl, or optionally substituted C3-C10 cycloalkyl;
Z is C, S(O), 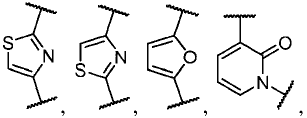 or Z is CH when X¹ is absent, or - Z(X¹)Q¹ is absent;
X¹ is absent, O, NH, S, or X1 is 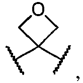 wherein the wavy lines denote points of attachment to Z;
X² is N or CQ²;
Q² is H, halogen, CN, CONH₂, COOH, optionally substituted C1-C10 alkyl, optionally substituted C3-C10 cycloalkyl, optionally substituted C3-C10 heteroalkyl, optionally substituted C3-C10 heterocyclyl, optionally substituted C6-C14 aryl, or optionally substituted C5-C14 heteroaryl;
R¹ is H, optionally substituted C1-C10 alkyl, optionally substituted C3-C10 cycloalkyl, optionally substituted C3-C10 heteroalkyl, optionally substituted C3-C10 heterocyclyl, optionally substituted C6-C10 aryl, optionally substituted C5-C10 heteroaryl, or C1-C12 acyl;
R², R³, R⁴, and R⁵ are independently H, halogen, CN, optionally substituted C1-C10 alkyl, optionally substituted C3-C10 cycloalkyl, optionally substituted C1-C6 alkoxy, SO₂R⁷, CO2R⁷, or CONR⁷R⁶, or any one of R² and R³, R³ and R⁴, and R⁴ and R⁵ pairs, together with the carbon atoms to which they are attached, forms an optionally substituted a five- or six-membered cycloalkenyl, heterocyclenyl, aryl or heteroaryl; and
R⁶ and R⁷ are independently H, optionally substituted C1-C10 alkyl, or optionally substituted C3-C10 cycloalkyl, or R⁶ and R⁷, together with the nitrogen atom to which they are attached, form an optionally substituted 5-membered ring or an optionally substituted 6-membered ring.

In another aspect, provided herein is a compound of the formula: 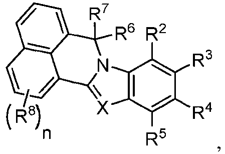 a tautomer thereof, a stereoisomer thereof, a hydrate thereof, or a pharmaceutically acceptable salt thereof, wherein:
R⁶ and R⁷ are independently H, halogen, OH, or optionally substituted C1-C6 alkyl, or R⁶ and R⁷ taken together are =O or =S;
X is N or CR¹;
R¹ is H, optionally substituted C6-C10 aryl; optionally substituted C5-C10 heteroaryl, or optionally substituted C1-C6 alkyl;
R², R³, R⁴, and R⁵ are independently H, halogen, CN, optionally substituted C1-C10 alkyl, optionally substituted C3-C10 cycloalkyl, optionally substituted C1-C6 alkoxy, SO₂R⁹, CO₂R⁹, or CONR⁹R¹⁰, or any one of R² and R³, R³ and R⁴, and R⁴ and R⁵ pairs, together with the carbon atoms to which they are attached, forms an optionally substituted a five- or six-membered cycloalkenyl, heterocyclenyl, aryl or heteroaryl; and;
R⁸, at each occurrence, is independently CN, optionally substituted C1-C6 alkyl, or halogen;
R⁹ and R¹⁰ are independently H, optionally substituted C1-C10 alkyl, or optionally substituted C3-C10 cycloalkyl, or R⁹ and R¹⁰, together with the nitrogen atom to which they are attached, form an optionally substituted 5-membered ring or an optionally substituted 6-membered ring; and
m is 0, 1, 2, 3, 4, 5, or 6.

In another aspect, provided herein is a compound of the formula: 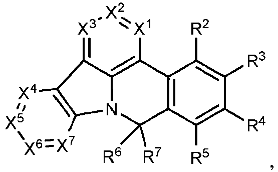 a tautomer thereof, a stereoisomer thereof, a hydrate thereof, or a pharmaceutically acceptable salt thereof, wherein:
R⁶ and R⁷ are independently H, halogen, OH, or optionally substituted C1-C6 alkyl, or R⁶ and R⁷ taken together are =O or =S;
R², R³, R⁴, and R⁵ are independently H, halogen, CN, optionally substituted C1-C10 alkyl, optionally substituted C3-C10 cycloalkyl, optionally substituted C1-C6 alkoxy, SO₂R⁹, CO₂R⁹, or CONR⁹R¹⁰, or any one of R² and R³, R³ and R⁴, and R⁴ and R⁵ pairs, together with the carbon atoms to which they are attached, forms an optionally substituted a five- or six-membered cycloalkenyl, heterocyclenyl, aryl or heteroaryl; and;
each of X¹, X², X³, X⁴, X⁵, X⁶, and X⁷ is, independently N or CR⁸, provided that no more than two of X¹, X², X³, X⁴, X⁵, X⁶, and X⁷ are N;
each of R⁸ is, independently, H, CN, halogen, optionally substituted C1-C10 alkyl, optionally substituted C3-C10 cycloalkyl, optionally substituted C1-C6 alkoxy, SO₂R⁹, CO₂R⁹, or CONR⁹R¹⁰; and
R⁹ and R¹⁰ are independently H, optionally substituted C1-C10 alkyl, or optionally substituted C3-C10 cycloalkyl, or R⁹ and R¹⁰, together with the nitrogen atom to which they are attached, form an optionally substituted 5-membered ring or an optionally substituted 6-membered ring.

In another aspect, provided herein is a method of treating an autoimmune disease treatable through induction of regulatory T-cells comprising administering a therapeutically effective amount of an aryl hydrocarbon receptor (AhR) ligand to a subject in need thereof, wherein the aryl hydrocarbon receptor (AhR) ligand is a compound disclosed herein.

In some embodiments, the autoimmune disease is diabetes mellitus type 1.

In some embodiments, the autoimmune disease is graft versus host disease, Celiac disease, autoimmune hepatitis, autoimmune pancreatitis, Crohn's disease, interstitial cystitis, microscopic colitis, or ulcerative colitis.

In some embodiments, the autoimmune disease is alopecia areata, atopic dermatitis, cicatricial pemphigoid, dermatomyositis, dermatitis herpetiformis, lichen planus, pemphigus vulgaris, or psoriasis.

In some embodiments, the aryl hydrocarbon receptor (AhR) ligand is administered topically. In other embodiments, the aryl hydrocarbon receptor (AhR) ligand is administered orally, transdermally, intravenously, subcutaneously, or with a nanoparticle.

In some embodiments, the method further includes administering the AhR ligand with a pharmaceutically acceptable carrier.

In another aspect, provided herein is a pharmaceutical composition comprising an AhR ligand of the disclosure.

### DETAILED DESCRIPTION

A need exists for a non-toxic therapy to suppress an autoimmune response without inducing general immune suppression. Accordingly, in one aspect, provided herein is an AhR ligand compound of the Formula I: 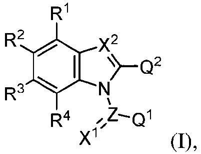 a tautomer thereof, a stereoisomer thereof, a hydrate thereof, or a pharmaceutically acceptable salt thereof, wherein:
Q¹ is an optionally substituted C6-C14 aryl; optionally substituted C5-C14 heteroaryl; optionally substituted C5-C14 heterocyclyl; optionally substituted C1-C10 alkyl, or optionally substituted C3-C10 cycloalkyl; optionally substituted C2-C10 alkenyl, or optionally substituted C2-C10 alkynyl;
Q² is an optionally substituted C6-C14 aryl; optionally substituted C5-C14 heteroaryl; optionally substituted C5-C10 heterocyclyl; optionally substituted C1-C10 alkyl, or optionally substituted C3-C10 cycloalkyl;
Z is C, S(O), 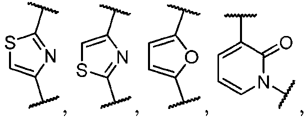 or Z is 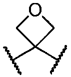 when X¹ is absent;
X¹ is absent, O, NH, or S;
X² is N, CCl, CF, CBr, CI, CCN, CCONH₂, CCOOH, or CH;
R¹, R², R³, and R⁴ are independently H, halogen, CN, OCF₃, optionally substituted C1-C10 alkyl, optionally substituted C3-C8 cycloalkyl, optionally substituted C3-C8 heterocycloalkyl, optionally substituted C1-C6 alkoxy, SO₂R⁵, CO₂R⁵, or CONR⁵R⁶, or any one of R¹ and R², R² and R³, and R³ and R⁴ pairs, together with the carbon atoms to which they are attached, forms an optionally substituted five- or six-membered cycloalkenyl, heterocyclenyl, aryl or heteroaryl; and
R⁵ and R⁶ are independently H, optionally substituted C1-C10 alkyl, or optionally substituted C3-C10 cycloalkyl, or R⁵ and R⁶, together with the nitrogen atom to which they are attached, form an optionally substituted 5-membered ring or an optionally substituted 6-membered ring.

In some embodiments of Formula I, X² is N. In certain embodiments of Formula I, Z is C.

In some embodiments, the compound is represented by the Formula IA: 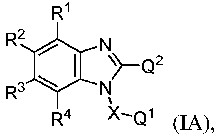 a tautomer thereof, a stereoisomer thereof, a hydrate thereof, or a pharmaceutically acceptable salt thereof, wherein:
Q¹ is an optionally substituted C6-C10 aryl; optionally substituted C5-C10 heteroaryl; optionally substituted C5-C10 heterocyclyl; optionally substituted C1-C10 alkyl, or optionally substituted C3-C10 cycloalkyl;
Q² is an optionally substituted C6-C14 aryl; optionally substituted C5-C10 heteroaryl; optionally substituted C5-C10 heterocyclyl; optionally substituted C1-C10 alkyl, or optionally substituted C3-C10 cycloalkyl;
X is CO or S(O)₂;
R¹, R², R³, and R⁴ are independently H, halogen, CN, optionally substituted C1-C10 alkyl, optionally substituted C3-C10 cycloalkyl, optionally substituted C1-C6 alkoxy, SO₂R⁵, CO2R⁵, or CONR⁵R⁶, or any one of R¹ and R², R² and R³, and R³ and R⁴ pairs, together with the carbon atoms to which they are attached, forms an optionally substituted a five- or six-membered cycloalkenyl, heterocyclenyl, aryl or heteroaryl; and
R⁵ and R⁶ are independently H, optionally substituted C1-C10 alkyl, or optionally substituted C3-C10 cycloalkyl, or R⁵ and R⁶, together with the nitrogen atom to which they are attached, form an optionally substituted 5-membered ring or an optionally substituted 6-membered ring.

In some embodiments of Formula IA, X is CO. In some embodiments of Formula IA, X is SO₂.

In some embodiments of Formula IA, the compound is a compound of the formula IB or IC:

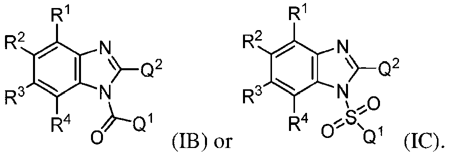

In some embodiments of Formula IA, IB, or IC, Q² is a 1-naphthyl.

In some embodiments of Formulae I, IA, IB, or IC, the compound is a compound of the formula ID: 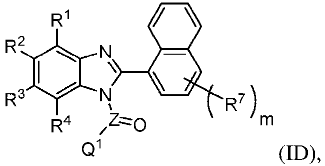 a tautomer thereof, a stereoisomer thereof, a hydrate thereof, or a pharmaceutically acceptable salt thereof, wherein:
Q¹ is an optionally substituted C6-C10 aryl; optionally substituted C5-C10 heteroaryl; optionally substituted C5-C10 heterocyclyl; optionally substituted C1-C10 alkyl, or optionally substituted C3-C10 cycloalkyl;
Z is C or SO;
R¹, R², R³, and R⁴ are independently H, halogen, CN, optionally substituted C1-C10 alkyl, optionally substituted C3-C10 cycloalkyl, optionally substituted C1-C6 alkoxy, SO₂R⁵, CO₂R⁵, or CONR⁵R⁶, or any one of R¹ and R², R² and R³, and R³ and R⁴ pairs, together with the carbon atoms to which they are attached, forms an optionally substituted a five- or six-membered cycloalkenyl, heterocyclenyl, aryl or heteroaryl;
R⁵ and R⁶ are independently H, optionally substituted C1-C10 alkyl, or optionally substituted C3-C10 cycloalkyl, or R⁵ and R⁶, together with the nitrogen atom to which they are attached, form an optionally substituted 5-membered ring or an optionally substituted 6-membered ring;
R⁷, at each occurrence, is independently H, halogen, CN, optionally substituted C1-C10 alkyl, optionally substituted C2-C6 alkenyl, optionally substituted C1-C10 heteroalkyl, optionally substituted C1-C10 heterocyclyl, optionally substituted C6-C10 aryl, optionally substituted C5-C10 heteroaryl, optionally substituted C1-C6 alkoxy, optionally substituted C1-C6 cycloalkyloxy, OCF₃, NR⁵R⁶, SCF₃, or C(O)NR⁵R⁶; and m is an integer ranging from 1 to 7.

In some embodiments of Formulae I, IA, IB, IC, or ID, Q¹ is an optionally substituted phenyl, an optionally substituted naphthyl, an optionally substituted optionally substituted C3-C6 cycloalkyl or an optionally substituted quinolinyl. In some embodiments, Q¹ is a phenyl optionally substituted with one, two, or three substituents independently selected from F, Cl, Br, OCH₃, CN, OCF₃, SCF₃, t-Bu, NMe₂, CONH₂, piperazyl, piperidyl, OCH₂CH₂OH, OCH₂CH₂NMe₂, and 1-naphthyl.

In some embodiments of Formulae I, IA, IB, IC, or ID, R¹ is H or halogen, such as F, Cl, or Br. In some embodiments of Formulae I, IA, IB, IC, or ID, R⁴ is H or halogen, such as F, Cl, or Br. In some embodiments of Formulae I, IA, IB, IC, or ID, all R⁷ are H.

In some embodiments of Formulae I, IA, IB, IC, or ID, the compound is a compound of formula IE: 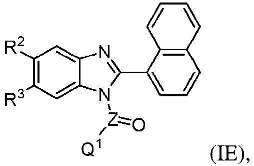
a tautomer thereof, a stereoisomer thereof, a hydrate thereof, or a pharmaceutically acceptable salt thereof,
wherein R² and R³, independently, F, Cl, Br, O(C1-C5 alkyl), SCF₃, OCF₃, CO₂H, CO₂(C1-C5 alkyl), or CONR⁵R⁶, wherein R⁵ and R⁶ are independently H, optionally substituted C1-C10 alkyl, optionally substituted C3-C10 cycloalkyl, or R⁵ and R⁶, together with the nitrogen atom to which they are attached, form an optionally substituted morpholinyl; and
Q¹ is an optionally substituted C6-C10 aryl; optionally substituted C5-C10 heteroaryl; optionally substituted C5-C10 heterocyclyl; optionally substituted C1-C10 alkyl, or optionally substituted C3-C10 cycloalkyl.

In some embodiments of Formula IE, Q¹ is a phenyl, cyclopropyl, naphthyl, benzodioxanyl, or quinolinyl, each of which is optionally substituted with one, two, or three substituents independently selected from the group consisting of F, Cl, Br, CF₃, SCF₃, CN, and OCH₃.

In some embodiments of Formulae I, IA, IB, IC, ID, or IE, the compound is a compound of Table 1.

In a second aspect, provided herein is an AhR ligand compound represented by Formula II: 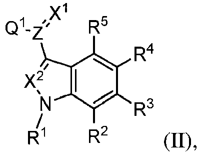 a tautomer thereof, a stereoisomer thereof, a hydrate thereof, or a pharmaceutically acceptable salt thereof, wherein:
Q¹ is an optionally substituted C6-C14 aryl; optionally substituted C5-C14 heteroaryl; optionally substituted C5-C14 heterocyclyl; optionally substituted C1-C10 alkyl, or optionally substituted C3-C10 cycloalkyl; optionally substituted C2-C10 alkenyl, or optionally substituted C2-C10 alkynyl;
Z is C, S(O), 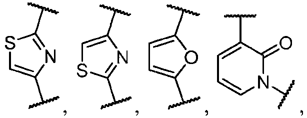 or Z is 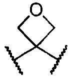 when X¹ is absent, or -Z(X¹)Q¹ is H;
X¹ is absent, O, NH, S,
X² is N, CCl, CF, CBr, CI, CCN, CCONH₂, CCOOH, CH, or CQ², wherein Q² is optionally substituted C6-C10 aryl, optionally substituted C5-C10 heteroaryl, optionally substituted C6-C10 aryl, optionally substituted C3-C10 cycloalkyl, or optionally substituted C3-C10 heterocyclyl;
R¹ is H, optionally substituted C1-C10 alkyl, optionally substituted C3-C10 cycloalkyl, optionally substituted C3-C10 heteroalkyl, optionally substituted C3-C10 hetercyclyl, optionally substituted C6-C10 aryl, optionally substituted C5-C10 heteroaryl, or C1-C12 acyl;
R², R³, R⁴, and R⁵ are independently H, halogen, CN, optionally substituted C1-C10 alkyl, optionally substituted C3-C10 cycloalkyl, optionally substituted C1-C6 alkoxy, SO₂R⁷, CO₂R⁷, or CONR⁷R⁶, or any one of R² and R³, R³ and R⁴, and R⁴ and R⁵ pairs, together with the carbon atoms to which they are attached, form an optionally substituted five-membered or six-membered cycloalkenyl, heterocyclenyl, aryl, or heteroaryl; and
R⁶ and R⁷ are independently H, optionally substituted C1-C10 alkyl, or optionally substituted C3-C10 cycloalkyl, or R⁶ and R⁷, together with the nitrogen atom to which they are attached, form an optionally substituted 5-membered ring or an optionally substituted 6-membered ring.

In some embodiments of Formula II, X² is CH, CF, CBr, or CCl. In some embodiments of Formula II, Z is CH.

In some embodiments of Formula II, the compound is represented by Formula IIA: 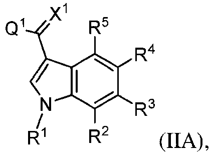 wherein Q¹, X¹, R¹, R², R³, R⁴, and R⁵ are as defined above for Formula II.

In some embodiments of Formulae II or IIA, X¹ is O.

In some embodiments of Formulae II or IIA, the compound is represented by the Formula IIC: 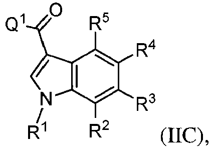 wherein all substituents are as defined for Formula II.

In some embodiments of Formulae II, IIA, IIB, or IIC, Q¹ is selected from optionally substituted pyridyl, optionally substituted naphthyl, optionally substituted benzodioxanyl, optionally substituted cyclopropyl, optionally substituted benzyl, optionally substituted phenyl, optionally substituted cyclohexyl, optionally substituted piperidinyl, optionally substituted quinolinyl, optionally substituted benzofuryl, optionally substituted benzomorpholinyl, and optionally substituted benzimidazolyl.

In some embodiments of Formulae II or IIA, Q¹ is a C5 heterocyclyl. In certain embodiments, Q¹ is thiazolyl, imidazolyl, pyrrolyl, pyrazolyl, thiophenyl, triazolyl, or furyl, each of which can be optionally substituted. In some embodiments of Formulae II or IIA, Q¹ is a C6 heterocyclyl. In some embodiments of Formulae II or IIA, Q¹ is pyridyl, pyrimidinyl, phenyl optionally substituted with alkyl or halogen, or pyridonyl, each of which can be optionally substituted. In other embodiments, Q¹ is indolyl, indazolyl, benzimidazolyl, or benzthiazolyl, each of which can be optionally substituted.

In some embodiments of Formulae II, IIA, IIB, or IIC, Q¹ is: 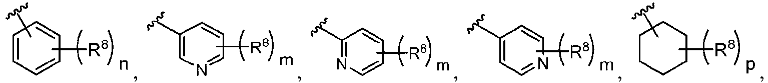 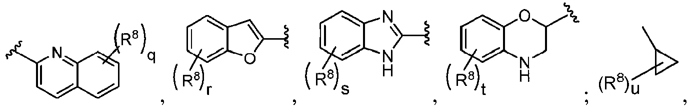 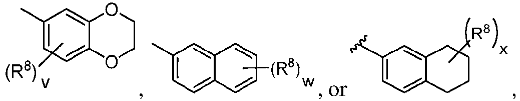
wherein R⁸, at each occurrence, is independently H, F, Cl, Br, I, CN, optionally substituted C1-C10 alkyl, optionally substituted C3-C8 cycloalkyl, optionally substituted C2-C6 alkenyl, optionally substituted C2-C6 alkynyl, optionally substituted C3-C10 heteroalkyl, optionally substituted C3-C10 heterocyclyl, optionally substituted C6-C10 aryl, optionally substituted C5-C10 heteroaryl, optionally substituted C1-C6 alkoxy, optionally substituted C3-C8 cycloalkyloxy, OCF₃, CF₃, NR'R", SCF₃, or C(O)NR'R";
R' and R" are H, optionally substituted C1-C10 alkyl, optionally substituted C3-C8 cycloalkyl, optionally substituted C2-C6 alkenyl, optionally substituted C2-C6 alkynyl, or optionally substituted C3-C10 heteroalkyl; or R' and R", together with the nitrogen atom to which they are attached, form an optionally substituted 5-membered ring or an optionally substituted 6-membered ring; and
n is an integer ranging from 1 to 5; m is an integer ranging from 1 to 4; p is an integer ranging from 1 to 11; q is an integer ranging from 1 to 6; r is an integer ranging from 1 to 5; s is an integer ranging from 1 to 4; t is an integer ranging from 1 to 8; u is an integer ranging from 1 to 5; v is an integer ranging from 1 to 7; w is an integer ranging from 1 to 7; and x is an integer ranging from 1 to 11.

In some embodiments of Formulae II, IIA, IIB, or IIC, Q¹ is morpholinyl, piperazinyl, pyrrolidinyl, piperidinyl, or alkylamino.

In certain embodiments of Formulae II, IIA, IIB, or IIC, Q¹ is a phenyl optionally substituted with one or two substituents independently selected from F, Cl, Br, I, OCH₃, CN, OCF₃, SCF₃, t-Bu, NMe₂, CO₂H, CO₂(C1-C10 alkyl), CONH₂, piperazyl, piperidyl, OCH₂CH₂OH, OCH₂CH₂NMe₂, and 1-naphthoyl.

In certain embodiments of Formulae II, IIA, IIB, or IIC, Q¹ is: 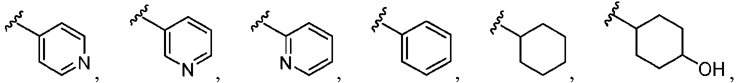 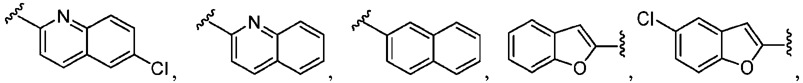 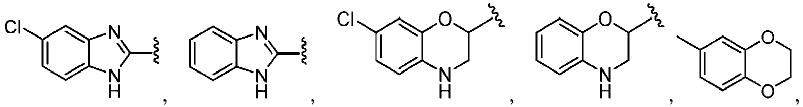 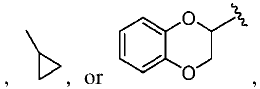 each of which can be further optionally substituted with one to four substituents independently selected from F, Br, Cl, I, OCH₃, CN, OCF₃, CF₃, SCF₃, Me, Et, i-Pr, t-Bu, NMe₂, CONH₂, OCH₂CH₂OH, OCH₂CH₂NMe₂, CHCH₂, OMe, OEt, O(iPr), O(tBu), and OC₅H₁₁.

In some embodiments of Formula II, Z(X¹)Q¹ is H.

In some embodiments of Formula II, the compound represented by Formula IID: 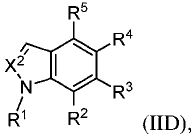 a tautomer thereof, a stereoisomer thereof, a hydrate thereof, or a pharmaceutically acceptable salt thereof, wherein:
X² is N, CCl, CF, CBr, CI, CCN, CCONH₂, CCOOH, CH, or CQ², wherein Q² is optionally substituted C6-C10 aryl, optionally substituted C5-C10 heteroaryl, optionally substituted C6-C10 aryl, optionally substituted C3-C10 cycloalkyl, or optionally substituted C3-C10 hetercyclyl; and
R¹, R², R³, R⁴, and R⁵ are independently as defined for Formula II.

In some embodiments of Formula IID, X² is CQ², wherein Q² is optionally substituted C6-C10 aryl, optionally substituted C5-C10 heteroaryl, optionally substituted C6-C10 aryl, optionally substituted C3-C10 cycloalkyl, or optionally substituted C3-C10 hetercyclyl.

In some embodiments of Formula IID, X² is CQ², wherein Q² is optionally substituted phenyl, optionally substituted naphthyl, optionally substituted quinolinyl, optionally substituted cyclopropyl, or optionally substituted cyclohexyl.

In some embodiments, the compound represented by Formula IIE: 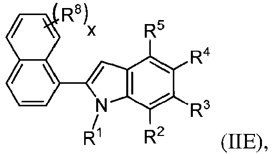 a tautomer thereof, a stereoisomer thereof, a hydrate thereof, or a pharmaceutically acceptable salt thereof, wherein R¹, R², R³, R⁴, and R⁵ are independently as defined for Formula II; R⁸, at each occurrence, is independently H, halogen, CN, optionally substituted C1-C10 alkyl, optionally substituted C3-C8 cycloalkyl, optionally substituted C2-C6 alkenyl, optionally substituted C2-C6 alkynyl, optionally substituted C3-C10 heteroalkyl, optionally substituted C3-C10 heterocyclyl, optionally substituted C6-C10 aryl, optionally substituted C5-C10 heteroaryl, optionally substituted C1-C6 alkoxy, optionally substituted C3-C8 cycloalkyloxy, OCF₃, CF₃, NR'R", SCF₃, or C(O)NR'R"; x is an integer ranging from 1 to 7; and R' and R" are H, optionally substituted C1-C10 alkyl, optionally substituted C3-C8 cycloalkyl, optionally substituted C2-C6 alkenyl, optionally substituted C2-C6 alkynyl, or optionally substituted C3-C10 heteroalkyl; or R' and R", together with the nitrogen atom to which they are attached, form an optionally substituted 5-membered ring or an optionally substituted 6-membered ring.

In some embodiments of Formula IIE, R¹ is H or an optionally substituted C1-C10 alkyl. In some embodiments of Formula IIE, R¹ is H. In some embodiments of Formula IIE, all R⁸ are H.

In some embodiments of Formulae II, IIA, IIB, IIC, or IID, R¹ is H, CH₃, or C(O)R⁹, wherein R⁹ is H, an optionally substituted C1-C10 alkyl, optionally substituted C3-C10 cycloalkyl, optionally substituted C3-C10 heteroalkyl, optionally substituted C3-C10 hetercyclyl, optionally substituted C6-C10 aryl, or optionally substituted C5-C10 heteroaryl.

In some embodiments of Formulae II, IIA, IIB, or IIC, Q¹ is a phenyl optionally substituted with one or two substituents independently selected from F, Cl, OCH₃, CH₃, CN, CF₃, OCF₃, SCF₃, t-Bu, NMe₂, CONH₂, 1-piperazyl, OCH₂CH₂OH, OCH₂CH₂NMe₂, and 1-naphthoyl.

In some embodiments of Formulae II, IIA, IIB, IIC, IID, or IIE, R² is H, F, Cl, Br, or I. In some embodiments of Formulae II, IIA, IIB, IIC, IID, or IIE, R³ is H, F, Cl, Br, or I. In some embodiments of Formulae II, IIA, IIB, IIC, IID, or IIE, R⁴ is H, F, Cl, Br, or I. In some embodiments of Formulae II, IIA, IIB, IIC, IID, or IIE, R⁵ is H, F, Cl, Br, or I.

In some embodiments of Formulae II, IIA, IIB, IIC, IID, or IIE, the compound is a compound of Table 2.

In a third aspect, provided herein is an AhR ligand compound represented by Formula III: 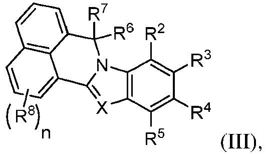 a tautomer thereof, a stereoisomer thereof, a hydrate thereof, or a pharmaceutically acceptable salt thereof, wherein:
R⁶ and R⁷ are independently H, halogen, OH, or optionally substituted C1-C6 alkyl, or R⁶ and R⁷ taken together are =O or =S;
X is N or CR¹;
R¹ is H, halogen, optionally substituted C6-C10 aryl; optionally substituted C5-C10 heteroaryl, or optionally substituted C1-C6 alkyl;
R², R³, R⁴, and R⁵ are independently H, halogen, CN, optionally substituted C1-C10 alkyl, optionally substituted C3-C10 cycloalkyl, optionally substituted C1-C6 alkoxy, SO₂R⁹, CO₂R⁹, or CONR⁹R¹⁰, or any one of R² and R³, R³ and R⁴, and R⁴ and R⁵ pairs, together with the carbon atoms to which they are attached, forms an optionally substituted a five- or six-membered cycloalkenyl, heterocyclenyl, aryl or heteroaryl; and;
R⁸, at each occurrence, is independently CN, optionally substituted C1-C6 alkyl, or halogen;
R⁹ and R¹⁰ are independently H, optionally substituted C1-C10 alkyl, or optionally substituted C3-C10 cycloalkyl, or R⁹ and R¹⁰, together with the nitrogen atom to which they are attached, form an optionally substituted 5-membered ring or an optionally substituted 6-membered ring; and n is 0, 1, 2, 3, 4, 5, or 6.

In some embodiments of Formula III, R⁶ and R⁷ taken together are =O. In other embodiments, R⁶ is H or C1-C10 alkyl and R⁷ is OH. In some embodiments of Formula III, X is CH.

In some embodiments of Formula III, the compound is represented by Formula IIIA: 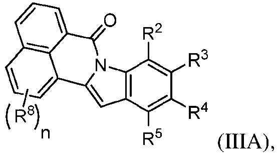 wherein all substituents are as defined for Formula III above.

In some embodiments of Formulae III or IIIA, R⁶ and R⁷ are H. In certain embodiments of Formulae III or IIIA, X is N.

In some embodiments of Formulae III or IIIA, the compound is represented by Formula IIIB: 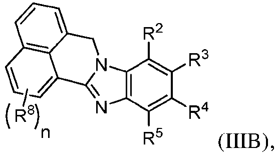 wherein all substituents are as defined for Formula III above. In some embodiments of Formulae III, IIIA, or IIIB, R² is H or halogen. In some embodiments of Formulae III, IIIA, or IIIB, R³ is H or halogen. In certain embodiments of Formulae III, IIIA, or IIIB, R⁴ is H or halogen. In particular embodiments of Formulae III, IIIA, or IIIB, R⁵ is H or halogen.

In some embodiments of Formulae III, IIIA, or IIIB, n is 0.

In a fourth aspect, provided herein is an AhR ligand compound represented by Formula IV: 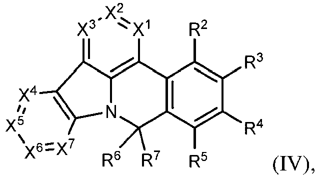 a tautomer thereof, a stereoisomer thereof, a hydrate thereof, or a pharmaceutically acceptable salt thereof, wherein:
R⁶ and R⁷ are independently H, halogen, OH, or optionally substituted C1-C6 alkyl, or R⁶ and R⁷ taken together are =O or =S;
R², R³, R⁴, and R⁵ are independently H, halogen, CN, optionally substituted C1-C10 alkyl, optionally substituted C3-C10 cycloalkyl, optionally substituted C1-C6 alkoxy, SO₂R⁹, CO₂R⁹, or CONR⁹R¹⁰, or any one of R² and R³, R³ and R⁴, and R⁴ and R⁵ pairs, together with the carbon atoms to which they are attached, forms an optionally substituted a five- or six-membered cycloalkenyl, heterocyclenyl, aryl, or heteroaryl; and
X¹, X², X³, X⁴, X⁵, X⁶, and X⁷ are independently N or CR⁸, provided that no more than two of X¹, X², X³, X⁴, X⁵, X⁶, and X⁷ are N;
each of R⁸ is independently H, CN, halogen, optionally substituted C1-C10 alkyl, optionally substituted C3-C10 cycloalkyl, optionally substituted C1-C6 alkoxy, SO₂R⁹, CO₂R⁹, or CONR⁹R¹⁰; and
R⁹ and R¹⁰ are independently H, optionally substituted C1-C10 alkyl, or optionally substituted C3-C10 cycloalkyl, or R⁹ and R¹⁰, together with the nitrogen atom to which they are attached, form an optionally substituted 5-membered ring or an optionally substituted 6-membered ring.

In some embodiments of Formula IV, R⁶ is H and R⁷ is H. In particular embodiments of Formula IV, X¹ is N. In other embodiments of Formula IV, R⁶ and R⁷ together are =O.

In some embodiments of Formula IV, the compound is represented by Formula IVA or (IVB): 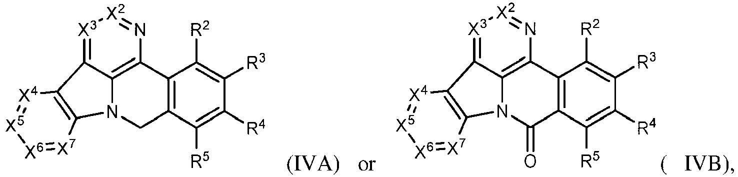 wherein all substituents are as defined above for Formula IV.

In some embodiments of Formulae IV, IVA, or IVB, R² is H or halogen. In some embodiments of Formulae IV, IVA, or IVB, R³ is H or halogen. In particular embodiments of Formulae IV, IVA, or IVB, R⁴ is H or halogen. In some embodiments of Formulae IV, IVA, or IVB, R⁵ is H or halogen.

In certain embodiments of Formulae IV, IVA, or IVB, each of X¹, X², X³, X⁴, X⁵, X⁶, and X⁷ is CR⁸, wherein R⁸ is H, optionally substituted C1-C10 alkyl, or halogen.

In some embodiments of the Formulae above, the AhR ligand is one or more compounds of Tables 1-5.

As used herein, the terms "alkyl," "alkenyl," and "alkynyl" include straight-chain, branched-chain, and cyclic monovalent hydrocarbyl radicals, and combinations of these, which contain only C and H when they are unsubstituted. Examples include methyl, ethyl, isobutyl, cyclohexyl, cyclopentylethyl, 2-propenyl, 3-butynyl, and the like. The total number of carbon atoms in each such group is sometimes described herein, e.g., when the group can contain up to ten carbon atoms it can be represented as 1-10C, as C₁-C₁₀, C-C10, or C1-10.

The terms "heteroalkyl," "heteroalkenyl," and "heteroalkynyl," as used herein, mean the corresponding hydrocarbons wherein one or more chain carbon atoms have been replaced by a heteroatom. Exemplary heteroatoms include N, O, S, and P. When heteroatoms are allowed to replace carbon atoms, for example, in heteroalkyl groups, the numbers describing the group, though still written as e.g. C3-C10, represent the sum of the number of carbon atoms in the cycle or chain and the number of such heteroatoms that are included as replacements for carbon atoms in the cycle or chain being described.

Typically, the alkyl, alkenyl, and alkynyl substituents contain 1-10 carbon atoms (alkyl) or 2-10 carbon atoms (alkenyl or alkynyl). Preferably, they contain 1-8 carbon atoms (alkyl) or 2-8 carbon atoms (alkenyl or alkynyl). Sometimes they refer to as "lower alkyl," meaning that they contain 1-6 carbon atoms (alkyl) or 2-6 carbon atoms (alkenyl or alkynyl). A single group can include more than one type of multiple bond, or more than one multiple bond; such groups are included within the definition of the term "alkenyl" when they contain at least one carbon-carbon double bond, and are included within the term "alkynyl" when they contain at least one carbon-carbon triple bond.

As used herein, the terms "alkylene," "alkenylene," and "alkynylene" include straight-chain, branched-chain, and cyclic divalent hydrocarbyl radicals, and combinations thereof.

Alkyl, alkenyl, and alkynyl groups can be optionally substituted to the extent that such substitution makes sense chemically. Typical substituents include, but are not limited to, halogens (F, Cl, Br, I), =O, =N-CN, =N-OR, =NR, OR, NR₂, SR, SO₂R, SO₂NR₂, NRSO₂R, NRCONR₂, NRC(O)OR, NRC(O)R, CN, C(O)OR, C(O)NR₂, OC(O)R, C(O)R, and NO₂, wherein each R is independently H, C₁-C₈ alkyl, C₂-C₈ heteroalkyl, C₁-C₈ acyl, C₂-C₈ heteroacyl, C₂-C₈ alkenyl, C₂-C₈ heteroalkenyl, C₂-C₈ alkynyl, C₂-C₈ heteroalkynyl, C₆-C₁₀ aryl, or C3-C10 heteroaryl, and each R is optionally substituted with halogens (F, Cl, Br, I), =O, =N-CN, =N-OR', =NR', OR', NR'₂, SR', SO₂R', SO₂NR'₂, NR'SO₂R', NR'CONR'₂, NR'C(O)OR', NR'C(O)R', CN, C(O)OR', C(O)NR'₂, OC(O)R', C(O)R', and NO₂, wherein each R' is independently H, C₁-C₈ alkyl, C₂-C₈ heteroalkyl, C₁-C₈ acyl, C₂-C₈ heteroacyl, C₆-C₁₀ aryl, or C₅-C₁₀ heteroaryl. Alkyl, alkenyl and alkynyl groups can also be substituted by C₁-C₈ acyl, C₂-C₈ heteroacyl, C₆-C₁₀ aryl, or C₅-C₁₀ heteroaryl, each of which can be substituted by the substituents that are appropriate for the particular group.

While "alkyl" as used herein includes cycloalkyl and cycloalkylalkyl groups, the term "cycloalkyl" is used herein to describe a carbocyclic non-aromatic group that is connected via a ring carbon atom, and "cycloalkylalkyl" is used to describe a carbocyclic non-aromatic group that is connected to the molecule through an alkyl linker. Similarly, "heterocyclyl" is used to identify a non-aromatic cyclic group that contains at least one heteroatom as a ring member and that is connected to the molecule via a ring atom, which may be C or N; and "heterocyclylalkyl" may be used to describe such a group that is connected to another molecule through an alkylene linker. As used herein, these terms also include rings that contain a double bond or two, as long as the ring is not aromatic.

"Aromatic" or "aryl" substituent or moiety refers to a monocyclic or fused bicyclic moiety having the well-known characteristics of aromaticity; examples include phenyl and naphthyl. Similarly, the terms "heteroaromatic" and "heteroaryl" refer to such monocyclic or fused bicyclic ring systems which contain as ring members one or more heteroatoms. Suitable heteroatoms include N, O, and S, inclusion of which permits aromaticity in 5-membered rings as well as 6-membered rings. Typical heteroaromatic systems include monocyclic C5-C6 aromatic groups such as pyridyl, pyrimidyl, pyrazinyl, thienyl, furanyl, pyrrolyl, pyrazolyl, thiazolyl, oxazolyl, and imidazolyl, and fused bicyclic moieties formed by fusing one of these monocyclic groups with a phenyl ring or with any of the heteroaromatic monocyclic groups to form a C8-C10 bicyclic group such as indolyl, benzimidazolyl, indazolyl, benzotriazolyl, isoquinolyl, quinolyl, benzothiazolyl, benzofuranyl, pyrazolopyridyl, quinazolinyl, quinoxalinyl, cinnolinyl, and the like. Any monocyclic or fused ring bicyclic system which has the characteristics of aromaticity in terms of electron distribution throughout the ring system is included in this definition. It also includes bicyclic groups where at least the ring which is directly attached to the remainder of the molecule has the characteristics of aromaticity. Typically, the ring systems contain 5-12 ring member atoms. Preferably, the monocyclic heteroaryls contain 5-6 ring members, and the bicyclic heteroaryls contain 8-10 ring members.

Aryl and heteroaryl moieties can be substituted with a variety of substituents including C₁-C₈ alkyl, C₂-C₈ alkenyl, C₂-C₈ alkynyl, C₃-C₁₂ aryl, C₁-C₈ acyl, and heteroforms of these, each of which can itself be further substituted; other substituents for aryl and heteroaryl moieties include halogens (F, Cl, Br, I), OR, NR₂, SR, SO₂R, SO₂NR₂, NRSO₂R, NRCONR₂, NRC(O)OR, NRC(O)R, CN, C(O)OR, C(O)NR₂, OC(O)R, C(O)R, and NO₂, wherein each R is independently H, C₁-C₈ alkyl, C₂-C₈ heteroalkyl, C₂-C₈ alkenyl, C₂-C₈ heteroalkenyl, C₂-C₈ alkynyl, C₂-C₈ heteroalkynyl, C₆-C₁₀ aryl, C₅-C₁₀ heteroaryl, C₇-C₁₂ arylalkyl, or C₆-C₁₂ heteroarylalkyl, and each R is optionally substituted as described above for alkyl groups. The substituent groups on an aryl or heteroaryl group may of course be further substituted with the groups described herein as suitable for each type of such substituents or for each component of the substituent. Thus, for example, an arylalkyl substituent may be substituted on the aryl portion with substituents described herein as typical for aryl groups, and it may be further substituted on the alkyl portion with substituents described herein as typical or suitable for alkyl groups.

"Optionally substituted," as used herein, indicates that the particular group being described may have one or more hydrogen substituents replaced by a non-hydrogen substituent. In some optionally substituted groups or moieties, all hydrogen substituents are replaced by a non-hydrogen substituent, e.g., C₁-C₆ alkyl, C₂-C₆ heteroalkyl, alkynyl, halogens (F, Cl, Br, I), N₃, OR, NR₂, SR, SO₂R, SO₂NR₂, NRSO₂R, NRCONR₂, NRC(O)OR, NRC(O)R, CN, C(O)OR, C(O)NR₂, OC(O)R, C(O)R, oxo, and NO₂, wherein each R is independently H, C₁-C₆ alkyl, or C₂-C₆ heteroalkyl. Where an optional substituent is attached via a double bond, such as a carbonyl oxygen or oxo (=O), the group takes up two available valences, so the total number of substituents that may be included is reduced according to the number of available valences.

Salts, stereoisomers, and tautomers of the compounds disclosed herein, such as compounds disclosed herein, are also within the scope of this disclosure. As used herein, "stereoisomer" or "stereoisomers" refer to compounds that differ in the chirality of one or more stereocenters. Stereoisomers include enantiomers and diastereomers. As used herein, "tautomer" refers to alternate forms of a compound that differ in the position of a proton, such as enol-keto and imine-enamine tautomers. As used herein, "salt" of a compound refers to an ion of the compound ionically association with a counterion. A salt of a compound can be formed by the neutralization reaction of an acid and a base. Salts can be derived from a variety of organic and inorganic counter ions well known in the art and include, by way of example only, sodium, potassium, calcium, magnesium, ammonium, and tetraalkylammonium; and when the molecule contains a basic functionality, salts of organic or inorganic acids, such as hydrochloride, hydrobromide, tartrate, mesylate, acetate, maleate, and oxalate. Although structures of the compounds disclosed herein can be shown in only one resonance form, it is understood that all resonance forms are included.

Synthesis of the compounds disclosed herein, e.g., compounds of Formulae I, IA, IB, IC, ID, IE, II, IIA, IIB, IIC, IID, IIE, III, IIA, IIB, IV, IVA, and IVB can be achieved in any suitable manner using techniques and methods known in the art.

In certain embodiments, the compounds of Formulae I , IA, IB, IC, ID, IE, II, IIA, IIB, IIC, IID, IIE, III, IIA, IIB, IV, IVA, and IVB disclosed herein are AhR activators. In some embodiments, the compounds activate AhR about 5, about 10, about 20, about 30, or about 35-fold in *in vitro* screening assays at about 10 nM, about 100 nM, about 1 uM, about 10uM, or about 100 uM. In some embodiments, the compounds of the disclosure adhere to one or more of the Lipinski rules.

In a fifth aspect, the disclosure provides a method of treating an autoimmune disease treatable through induction of regulatory T-cells comprising administering a therapeutically effective amount of an aryl hydrocarbon receptor (AhR) ligand to a subject in need thereof, wherein the aryl hydrocarbon receptor (AhR) ligand is a compound of any one of compounds of Formulae I, IA, IB, IC, ID, IE, II, IIA, IIB, IIC, IID, IIE, III, IIA, IIB, IV, IVA, and IVB disclosed herein. Autoimmune diseases suitable for treatment by the methods disclosed herein include diabetes mellitus type 1, graft versus host disease, Celiac disease, autoimmune hepatitis, autoimmune pancreatitis, Crohn's disease, interstitial cystitis, microscopic colitis, ulcerative colitis, alopecia areata, atopic dermatitis, cicatricial pemphigoid, dermatomyositis, dermatitis herpetiformis, lichen planus, pemphigus vulgaris, or psoriasis.

As used herein, the term "treat" refers to medical management of a disease, disorder, or condition (e.g., diabetes) of a subject (e.g., a human or non-human mammal, such as another primate, horse, dog, mouse, rat, guinea pig, rabbit, and the like). Treatment can encompass any indicia of success in the treatment or amelioration of a disease or condition (e.g., diabetes), including any parameter such as abatement, remission, diminishing of symptoms or making the disease or condition more tolerable to the subject, slowing in the rate of degeneration or decline, and/or making the degeneration less debilitating. The treatment or amelioration of symptoms can be based on objective or subjective parameters, including the results of an examination by a physician. Accordingly, the term "treating" includes the administration of the compounds and/or compositions of the present disclosure to alleviate, or to arrest or inhibit development of the symptoms or conditions associated with disease or condition (e.g., diabetes). The term "therapeutically effective" refers to an amount of the compound or composition that results in a therapeutic effect and can be readily determined.

The compounds of the disclosure can be administered in any suitable manner. In some embodiments, the compounds can be delivered locally (e.g., topically) or systemically. In some embodiments, the aryl hydrocarbon receptor (AhR) ligands of the disclosure are administered orally. In some embodiments, the compounds are administered topically, intravenously, or subcutaneously. A physiologically or pharmaceutically acceptable carrier or vehicle can be used to formulate the compound for administration and can be selected according to the mode of administration. In some embodiments, the compounds are delivered orally together with a suitable pharmaceutically acceptable carrier, e.g., at a predetermined dose.

Typically, the AhR ligands disclosed herein can be administered with one or more pharmaceutically acceptable carriers. Any suitable pharmaceutically acceptable carriers can be used with the compounds of the disclosure. Non-limiting examples of pharmaceutically acceptable carriers include saline, phosphate buffered saline (PBS), water, aqueous ethanol, emulsions such as oil/water emulsions, triglyceride emulsions, wetting agents, tablets, and capsules. In some embodiments, the compounds are formulated with a nanoparticle, e.g., a micelle or a liposome. Nanoparticles can include lipids, polymers, dendrimers, silicon materials, carbon materials, cyclodextrins, or other suitable components.

Thus, in another aspect provided herein is a pharmaceutical composition comprising a compound of the disclosure, e.g., an aryl hydrocarbon receptor (AhR) ligand of Formulae I, IA, IB, IC, ID, IE, II, IIA, IIB, IIC, IID, IIE, III, IIA, IIB, IV, IVA, and IVB described above.

Publications cited herein and the subject matter for which they are cited are hereby specifically incorporated by reference in their entireties.

While illustrative embodiments have been described, it will be appreciated that various changes can be made therein without departing from the spirit and scope of the invention.

The following examples are provided for the purpose of illustrating, not limiting, the invention.

### EXAMPLES

### SYNTHESIS OF EXEMPLARY COMPOUNDS

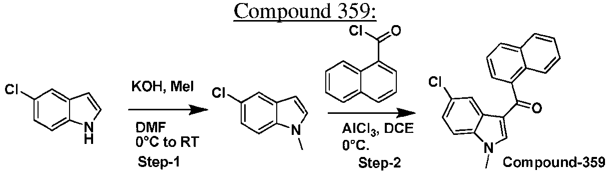

Step 1: 5-chloro-1H-indole (1.0 g, 6.59 mmol) was dissolved in DMF (8.0 ml) and potassium hydroxide (0.56 g, 7.9 mmol) was added to it. The reaction mixture was stirred at room temperature for one hour followed by cooling the reaction mixture to 0°C and addition of iodomethane (0.05 ml, 7.9 mmol). Reaction mixture was then stirred at room temperature for three hours followed by extraction with ethyl acetate and washing with brine solution. The organic layer was dried to get crude which was purified by column chromatography to afford 5-chloro-1-methyl-1H-indole (0.8g) as a brown solid.

Step 2: 5-chloro-1-methyl-1H-indole (0.2 g, 1.2 mmol) was dissolved in dichloroethane (4.0 ml) and cooled to 0°C. Aluminum trichloride (0.19 g, 1.45 mmol) was added to it. After few minutes of stirring, 1-naphthoyl chloride (0.218 ml, 1.44 mmol) was added dropwise. The resulting mixture was stirred at same temperature for one hour. After this, the reaction mixture was quenched with water and extracted with ethyl acetate. The organic layer was dried over anhydrous sodium sulphate and concentrated under vacuum to provide crude. The crude was purified by column chromatography to afford (5-chloro-1-methyl-1H-indol-3-yl)(naphthalen-1-yl)methanone (62mg) as an off white solid.

Compound 359: LCMS-UPLC 320.2 (M)⁺; @ 254 nm = 99.89%, @ 220 nm = 99.83%. ¹H NMR (400 MHz, CDCl₃) *δ* 8.55 (*d, J* = 1.75 Hz, 1H), 8.15 (*d, J* = 8.33 Hz, 1H), 7.98 (*d, J =* 8.33 Hz, 1H), 7.91 (d, *J =* 7.89 Hz, 1H), 7.64 (*d, J* = 5.70 Hz, 1H), 7.46 - 7.56 (*m,* 3H), 7.27 - 7.35 (*m,* 3H), 3.76 (s, 3H).

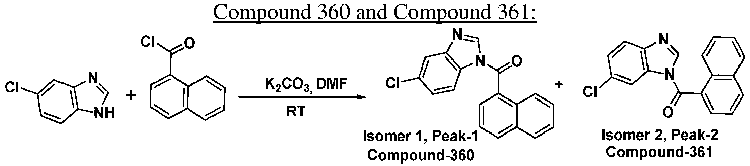

Step 1: To a solution of 5-chloro-1H-benzo[d]imidazole (0.46g, 3.0 mmol) in DMF was added K2CO3 (0.83g, 6.0 mmol) followed by addition of 1-naphthoyl chloride (0.5 mL, 1.1 mmol) and the reaction mixture was stirred at rt for overnight. After completion of reaction, the reaction mixture was diluted with sodium bicarbonate (20mL) and extracted with DCM (50 mL x 2). The combined organic layer was washed with water (50 mL), brine solution (50 mL), dried over anhydrous sodium sulfate and concentrated under reduced pressure to afford crude product, which was purified by flash chromatography to afford the first isomer (Peak 1), (5-chloro-1H-benzo[d]imidazol-1-yl)(naphthalen-1-yl)methanone (0.12g) as an off white solid.

Compound 360: LCMS-UPLC 307.1 (M)⁺; Purity @ 254 nm = 99.18% and @ 220 nm = 97.92%. ¹H NMR (400 MHz, DMSO-*d*₆): 8.40 (s, 1 H), 8.28 (*d, J*=8.33 Hz, 1 H), 8.09 - 8.17 (*m,* 2 H), 7.96 - 8.03 *(m,* 2 H), 7.88 - 7.94 *(m,* 1 H), 7.63 - 7.74 *(m,* 3 H), 7.54 *(d, J*=1.32 Hz, 1 H).

Flash chromatography also afforded the second isomer (Peak 2), (6-chloro-1H-benzo[d]imidazol-1-yl)(naphthalen-1-yl)methanone (0.2g) as an off white solid.

Compound 361: LCMS-UPLC 307.1 (M)⁺ Purity @ 254 nm = 99.62% and @ 220 nm = 98.22%. ¹H NMR (400 MHz, DMSO-*d*₆): 8.34 (s, 1 H), 8.28 (*d, J*=8.33 Hz, 1 H), 8.07 - 8.21 (m, 2 H), 7.92 - 8.03 (*m,* 2 H), 7.85 (s, *J*=8.33 Hz, 1 H), 7.58 - 7.73 (*m,* 3 H), 7.53 (*m,* J=8.33 Hz, 1 H).

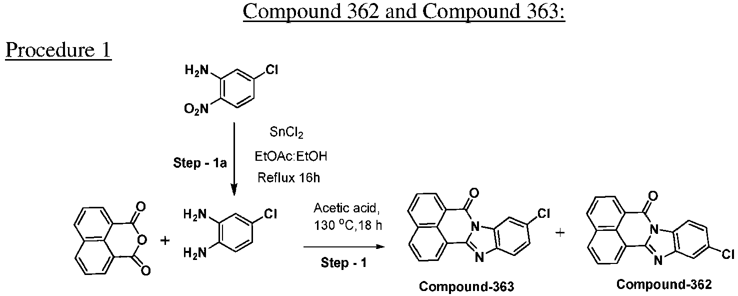

Step 1a: To a solution of 5-chloro-2-nitroaniline (10 g, 0.058 mol) in ethyl acetate (30 ml) and ethanol (15 mL) was added tin chloride (54.6 g. 29 mol). The reaction mixture was then refluxed at 80 °C for 16 h. TLC (30% ethyl acetate in hexane) and NMR showed the formation of desired product. Reaction mixture was concentrated under reduced pressure to remove excess solvent and then neutralized with saturated solution of sodium bicarbonate (1000 mL). The reaction mixture was extracted using ethyl acetate (2000 mL). Organic layer was dried over sodium sulphate and concentrated under reduced pressure to afford crude product which was purified by column chromatography (eluent was 0-30% ethyl acetate in hexane) to obtain 7.0g of 4-chlorobenzene-1,2-diamine as an off-white solid.

Step 1: To a solution of benzo[d]isochromene-1,3-dione (4.10 g, 0.020 mmol) in acetic acid (20 ml) was added 4-chlorobenzene-1,2-diamine (4.01 g. 0.028 mol). The reaction mixture was then heated to 130°C for 18 h. LCMS showed the formation of desired product. The reaction mixture was diluted with diethyl ether (50 mL). The precipitate thus obtained was filtered to get crude solid. This solid mass was further triturated in diethyl ether (100 mL) and filtered to get mixture of two regioisomers.

### Step 2: Purification of Compound 362 and Compound 363

The crude (mixture of isomers, 1.0 g, 3.27 mmol) was purified by column chromatography to yield 50 mg of Compound 362: -6-chloro-3,10-diazapentacyclo[10.7.1.0²,^{1⁰}.0⁴,⁹.0^{1⁶},^{2⁰}]icosa-1(19),2,4(9),5,7,12,14,16(20),17-nonaen-11-on (yellow solid) and 20 mg of Compound 363:-7-chloro-3,10-diazapentacyclo[10.7.1.0²,^{1⁰}.0⁴,⁹.0^{1⁶},^{2⁰}]icosa-1(19),2,4(9),5,7,12,14,16(20),17-nonaen-11-on (yellow solid) along with mixture of isomers.

Compound 362: LCMS: 305.2 (M)+, HPLC 220 nm = 99.88 %, UPLCMS @ 220 nm = 99.20 %, @ 220 nm = 99.03 %. ¹H NMR (400 MHz, DMSO-*d*⁶) δ 8.73 *(dd, J =* 16.0, 7.3 Hz, 2H), 8.56 (*d, J =* 8.1 Hz, 1H), 8.41 (*dd, J =* 8.4, 3.5 Hz, 2H), 8.01-7.88 (*m,* 3H), *7.52 (dd, J =* 8.6, 2.1 Hz, 1H). ¹H NMR (400 MHz, Chloroform-d) δ 8.83 (*dd, J =* 16.1, 7.3 Hz, 2H), 8.49 (*d, J* = 8.5 Hz, 1H), 8.32 (*d, J =* 8.2 Hz, 1H), 8.19 (*d, J =* 8.2 Hz, 1H), 7.90-*7.79* (*m,* 3H), 7.44 (*dd, J =* 8.5, 2.0 Hz, 1H).

Compound 363: LCMS: 305.2 (M)+; HPLC @ 220 nm = 99.12%, UPLCMS @ 220 nm = 98.74 %, 254 nm = 97.84 %. ¹H NMR (400 MHz, Chloroform-d) δ 8.89-8.79 (m, 2H), 8.61 (s, 1H), 8.32 (*d, J =* 8.2 Hz, 1H), 8.19 (*d, J =* 8.2 Hz, 1H), 7.88-7.81 (*m,* 3H), *7.46* (*dd, J =* 8.4, 2.2 Hz, 1H). ¹H NMR (400 MHz, DMSO-*d*₆) δ 8.78 *(d, J =* 7.3 Hz, 1H), *8.74* (*d, J* = 7.3 Hz, 1H), 8.58 (*d, J =* 8.2 Hz, 1H), 8.47-8.40 (*m,* 2H), 8.03-7.88 (*m,* 3H), 7.56 (*dd, J* = 8.5, 2.2 Hz, 1H).

### Procedure 2

### 11-substituted chloro-7H-Benzimidazo[P,I-a]benz[de]-isoquinolin-7-ones (Compound 362)

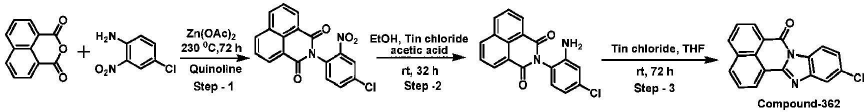

Step 1: The mixture of benzo[d]isochromene-1,3-dione (15 g, 0.075 mol), 4-chloro-2-nitroaniline (15.67 g, 0.090 mole), zinc acetate (13.76 g, 0.075 mol) and quinoline (80.0 ml) was heated at 230 °C using sealed tube for 72 h. The reaction mixture was then allowed to cool resulting in precipitation of solid which was filtered. The filtered solid was then further washed using MTBE (50 ml x 3). The isolated product was subjected to slurry wash using DM water (350 ml) at reflux and filtered to get 19.0 g of pure 2-(4-chloro-2-nitrophenyl)-1H-benzo[de]isoquinoline-1,3(2H)-dione.

Step 2: To the suspension of 2-(4-chloro-2-nitrophenyl)-1H-benzo[d]isoquinoline-1,3(2H)-dione (10.0 g, 0.0283 mole) in ethanol (300 ml), was added acetic acid (10.0 ml) and tin chloride (51.17 g, 0.226 mole). The reaction mixture was then stirred for 72 h. The reaction mixture was then concentrated to get crude product. The crude product was subjected to slurry wash using DM water (150 ml) and filtered to get 10.0 g 2-(2-amino-4-chlorophenyl)-1H-benzo[de]isoquinoline-1,3(2H)-dione.

Step 3: To the suspension of 2-(2-amino-4-chlorophenyl)-1H-benzo[de]isoquinoline-1,3(2H)-dione (10.0g, 0.031mol) in THF (600 ml) was added acetic acid (20.0 ml) followed by tin chloride (69.7g, 0.309 mol) in 5 portion (2.0 equiv. in each portion) over a period of 40 h. The TLC showed the formation of desired product along with starting material. The reaction mixture was then concentrated and partitioned between DM water (600 ml) and ethyl acetate (600 ml). The aqueous layer was extracted using ethyl acetate (400 ml). The organic layer was then dried over sodium sulfate and concentrated to get crude product. The crude product was purified by flash chromatography (eluent: 0-30 % ethyl acetate in hexane) to get 1.21 g of 6-chloro-3,10-diazapentacyclo[10.7.1.0²,^{1⁰}.0⁴,⁹.0^{1⁶},^{2⁰}]icosa-1(19),2,4(9),5,7,12,14,16(20),17-nonaen-11-one as a yellow solid.

Compound 362: LCMS- 305.1 (M)+ HPLC @ 220 nm = 97.56 %, UPLCMS @ 220 nm = 98.75 %, 254 nm = 98.56%. ¹H NMR (400 MHz, DMSO-d6) δ 8.73 (*dd, J =* 16.0, 7.3 Hz, 2H), 8.56 (*d, J =* 8.1 Hz, 1H), 8.41 (*dd, J =* 8.4, 3.5 Hz, 2H), 8.01-7.88 *(m,* 3H), 7.52 (*dd*, *J* = 8.6, 2.1 Hz, 1H). ¹H NMR (400 MHz, Chloroform-d) δ 8.83 (*dd, J =* 16.1, 7.3 Hz, 2H), 8.49 *(d, J =* 8.5 Hz, 1H), 8.32 (*d, J =* 8.2 Hz, 1H), 8.19 (*d, J =* 8.2 Hz, 1H), 7.90-7.79 (*m,* 3H), 7.44 (*dd, J =* 8.5, 2.0 Hz, 1H).

### Compound 363

### 7-chloro-3,10-diazapentacyclo[10.7.1.0²,^{1⁰}.0⁴,⁹.0^{1⁶},^{2⁰}]icosa-1(19),2,4(9),5,7,12,14,16(20),17-nonaen-11-one (Compound 363)

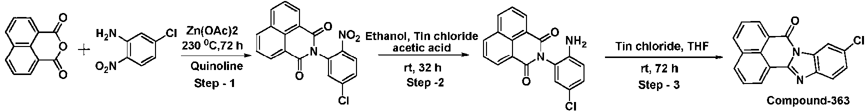

### Steps 1-3: were performed as described above for synthesis of Compound 362 (procedure 2)

Compound 363: LCMS- 305.2 (M)⁺: HPLC @ 220 nm = 99.58 %, UPLCMS @ 220 nm = 99.68 %, @ 220 nm = 99.16%. ¹H NMR (400 MHz, DMSO-*d*₆) δ 8.78 (*d, J =* 7.3 Hz, 1H), 8.74 (*d, J =* 7.3 Hz, 1H), 8.58 (*d, J =* 8.2 Hz, 1H), 8.47-8.40 (*m,* 2H), 8.03-7.88 (*m,* 3H), 7.56 *(dd, J =* 8.5, 2.2 Hz, 1H). ¹H NMR (400 MHz, Chloroform-d) δ 8.89-*8.79* (*m,* 2H), 8.61 (s, 1H), 8.32 (d, *J =* 8.2 Hz, 1H), 8.19 (*d, J =* 8.2 Hz, 1H), 7.88-7.81 *(m,* 3H), 7.46 (*dd, J =* 8.4, 2.2 Hz, 1H).

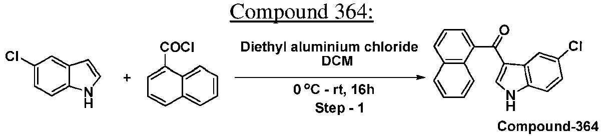

Step 1: To a solution of 5-chloro-1H-indole (2.0 g, 0.013mol) in DCM (40 ml) was added diethylaluminium chloride (21 mL, 0.019 mol) drop wise at 0 °C. The reaction mixture was stirred at 0 °C for 15 min. To this reaction mixture was added naphthalene-1-carbonyl chloride (3.03 g, 0.015mol) and the reaction mixture was allowed to stir for 16 h at room temperature. LCMS and HNMR showed the formation of desired product. The reaction mixture was quenched by DM water (100 ml) and extracted using dichloromethane (500 ml x 2). The organic layer was then dried over sodium sulfate and concentrated to get the crude product. The crude product was purified by column chromatography (eluent: 0-*5* % ethyl acetate in hexane) which yielded 3.0 g of (5-chloro-1H-indol-3-yl)(naphthalen-1-yl)methanone as an off white solid.

Compound 364: LCMS - 306.1(M)⁺: HPLC @ 220 nm = 99.53 %, UPLCMS @ 220 nm = 99.78 %, @ 220 nm = 99.80 %. ¹H NMR (400 MHz, DMSO-*d*6) δ 12.23 (s, 1H), 8.28 (s, 1H), 8.10 (*d*, *J* = 8.2 Hz, 1H), 8.02 (*dd, J =* 12.6, 8.1 Hz, 2H), 7.77 *(d, J =* 3.1 Hz, 1H), 7.71 *(d, J =* 6.9 Hz, 1H), 7.63-7.48 (*m,* 4H), 7.32 (*dd, J =* 8.7, 2.2 Hz, 1H).

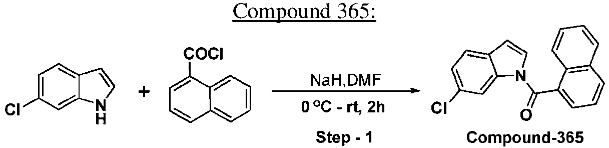

Step 1: To a solution of 6-chloro-1H-indole (0.5 g, 3.31mmol) in DMF (50 ml) was added sodium hydride (198 mg. 4.96 mmol) portion-wise at 0 °C. To this reaction mixture was added naphthalene-1-carbonyl chloride (754 mg, 3.97mmol) at 0 °C and was allowed to stir for 2 h at room temperature. LCMS showed the formation of desired product. The reaction mixture was then quenched by DM water (100 ml) and extracted using ethyl acetate (200 ml x 2). The organic layer was dried over sodium sulfate and concentrated to obtain crude product. The crude product was purified by column chromatography (eluent: 0-10 % ethyl acetate in hexane) which yielded 0.861g of (6-chloro-1H-indol-1-yl)(naphthalen-2-yl)methanone as off white solid.

Compound 365: LCMS- 306.1(M)+: HPLC @ 220 nm = 99.65%, UPLCMS @ 220 nm = 99.80 %, @ 254 nm = 99.69 %. ¹H NMR (400 MHz, Chloroform-d) δ ¹H NMR (400 MHz, DMSO-d 6 ) d 8.40 (s, 1H), 8.23 (*d, J =* 8.3 Hz, 1H), 8.11 (*d, J =* 8.1 Hz, 1H), 7.85 *(d, J =* 7.0 Hz, 1H), 7.79 (*d, J =* 8.3 Hz, 1H), 7.72-7.71 (*m,* 1H), 7.69-7.54 (*m,* 3H), 7.42 (dd, *J* = 8.3, 2.1 Hz, 1H), 7.12 (*d, J =* 3.8 Hz, 1H), 6.72 (*d, J =* 3.8 Hz, 1H).

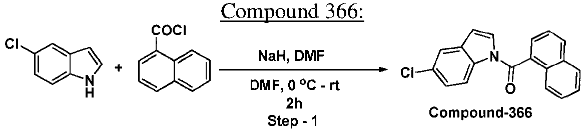

### Step 1: was performed as described above for synthesis of Compound 365

Compound 366: LCMS: 306.1 (M)+; HPLC @ 220 nm = 99.81%, @ 254 nm = 99.81 %. ¹H NMR (400 MHz, Chloroform-d) δ 8.45 *(d, J =* 8.8 Hz, 1H), 8.06 (*d, J =* 8.2 Hz, 1H), 7.99-7.92 (*m,* 1H), 7.87 (*d, J =* 8.2 Hz, 1H), 7.68 - 7.66 (*m,* 1H), 7.65-7.48 *(m,* 4H), 7.38 (*dd, J =* 8.8, 2.2 Hz, 1H), 7.02 *(d, J =* 3.8 Hz, 1H), 6.48 (*d, J =* 3.8 Hz, 1H).

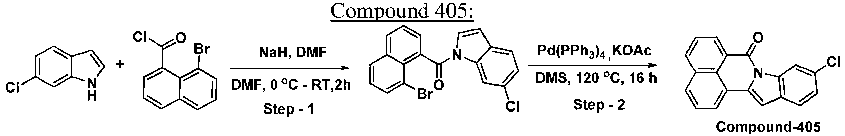

### Step 1 was performed as described above for synthesis of Compound 365

Step 2: The mixture of (8-bromonaphthalen-1-yl)(6-chloro-1H-indol-1-yl)methanone (0.5 g, 1.3mmol), potassium acetate (0.255 g, 2.6 mmol), tetrakistriphenylphosphine (0.150 g, 0.130 mmol) and DMS (8.0 ml) was purged for 10 min using nitrogen. The resultant reaction mixture was then stirred for 16 h at 120 °C. The TLC (10 % ethyl acetate in hexane) showed complete consumption of starting material. The reaction mixture was then quenched by DM water (20.0 ml) and extracted by ethyl acetate (25 ml x 2). The ethyl acetate layer was then concentrated to get crude product. The crude product was then purified by flash chromatography (eluent: 0-5 % ethyl acetate in hexane) to get 4.0 mg of (7-chloro-10-azapentacyclo[10.7.1.0²,^{1⁰}.0⁴,⁹.0^{1⁶},^{2⁰}]icosa-1(19),2,4(9),5,7,12,14,16(20),17-nonaen-11-one) as a white solid.

LCMS: 304.0 (M)+, HPLC @ 220 nm = 92.30 %, @ 254 nm = 91.48 %. ¹H NMR (400 MHz, Chloroform-d) δ 8.85 (s, 1H), 8.73 (*d, J* = 7.1 Hz, 1H), 8.20-8.17 (*m,* 2H), 7.95 (*d, J =* 8.1 Hz, 1H), 7.77 (t, *J* = 7.7 Hz, 1H), 7.68 (*t, J =* 7.8 Hz, 1H), 7.58 (*d, J =* 8.3 Hz, 1H), 7.38-7.30 (*m*, 2H).

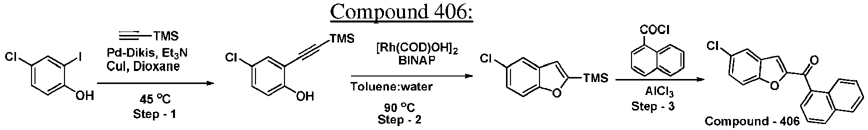

Step 1: To a solution of 4-chloro-2-iodophenol (4.0 g, 15.72 mmol) in dioxane (40.0 ml) under nitrogen, was added ethynyltrimethylsilane (1.852 g, 18.86 mmol), triethylamine (5.46 ml, 39.3 mmol) followed by addition of bis(triphenylphosphine)palladium(II) dichloride (1.10 g, 1.572 mmol) and copper iodide (0.598 g, 3.144 mmol). The reaction mixture was then allowed to stir at 45 °C for 1h. The TLC (10 % ethyl acetate in hexane) showed the formation of desired product. The reaction mixture was quenched by DM water (50.0 ml) and was extracted using ethyl acetate (50.0 ml x 3). The organic layer was then dried over sodium sulfate and concentrated to get 4.8 g of crude product which was then purified by column chromatography (eluent: 0-10 % ethyl acetate in hexane) to yield 3.2g of 4-chloro-2-((trimethylsilyl)ethynyl)phenol as colorless oil.

¹H NMR (400 MHz, Chloroform-d) δ 7.31 (d, J = 2.63 Hz, 1 H) 7.20-7.18 (m, 1 H) 6.88 (d, J = 8.77 Hz, 1 H) 0.28 (s, 9 H).

Step 2: The mixture of [Rh(COD)OH]₂ (0.324 g, 0.71 mmol) and BINAP (0.707 g, 1.13 mmol) in toluene: water (48 ml, 40:8) was stirred at room temperature. To this reaction mixture, was added solution of 4-chloro-2-((trimethylsilyl)ethynyl)phenol (3.2 g, 14.23 mmol) in toluene (20.0 ml). The resultant reaction mixture was stirred at 90 °C for 2h. The TLC (pentane) showed the formation of desired product. The reaction mixture was then filtered through a celite bed and concentrated. The obtained solid was subjected to slurry wash using pentane (150 ml). The resultant suspension was then filtered and concentrated to get 2.0 g of (5-chlorobenzofuran-2-yl)trimethylsilane as colorless oil.

¹H NMR (400 MHz, Chloroform-*d*) δ 7.89 (s, 1 H) 7.78 *(d, J =* 8.33 Hz, 1 H) 7.57 - 7.64 *(m,* 2 H) 0.71 - 0.74 *(m,* 9 H).

Step 3: To a solution of 1-naphthoyl chloride (0.190 g, 1.0 mmol) in dichloroethane (10.0 ml) at 0 °C was added AlCl₃ (0.133 g, 1.0 mmol) portion-wise. The reaction mixture was allowed to stir for 30 min. To this reaction mixture was added (5-chlorobenzofuran-2-yl)(naphthalen-1-yl)methanone (0.224 g, 1.0 mmol) and it was allowed to stir for 2h at room temperature. TLC (10 % ethyl acetate in hexane) showed the formation of desired product. The reaction mixture was quenched by DM water (15.0 ml). The reaction mixture was extracted using ethyl acetate (20.0 ml x 2). The organic layer was then concentrated to get crude product which was then purified by column chromatography (eluent: 0-10 % ethyl acetate in hexane) to yield 0.110 g of (5-chlorobenzofuran-2-yl)(naphthalen-1-yl)methanone as an off-white solid.

LC-MS: 307.1 (M)+, HPLC@ 220 nm = 99.51 %, UPLCMS @ 220 nm = 99.55 %, @ 254 nm = 99.92 %. ¹H NMR (400 MHz, Chloroform-d) δ 8.30-8.23 (*m,* 1H), 8.08 (*d, J* = 8.3 Hz, 1H), 7.97-7.93 (*m,* 1H), 7.87 (d, *J =* 7.1 Hz, 1H), 7.66 - 7.56 *m,* 5H), 7.47 (*dd, J* = 8.9, 2.2 Hz, 1H), 7.29 (s, 1H).

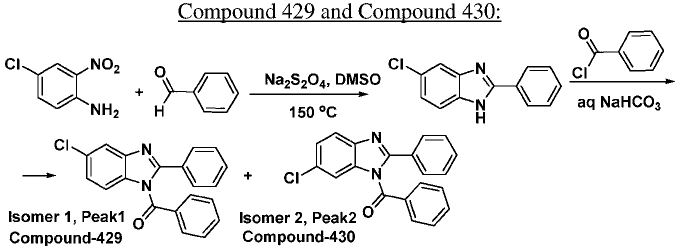

Step 1: To a solution of 4-chloro-2-nitroaniline (0.34g, 2.0 mmol, 1.0 eq) and benzaldehyde (0.21g, 2.0 mmol, 1.0 eq) in (5.0 mL) in DMSO was added Na₂S₂O₄ (0.61g, 1.0 mmol, 2.0 eq) and the reaction mixture was heated at 150 °C for 3h. After completion of reaction, solution was diluted with water and the precipitate thus obtained was filtered, washed with ether and dried to give the desired product as 5-chloro-2-phenyl-1H-benzo[d]imidazole (0.35g) as an off-white solid.
LCMS: 229.1 (M)+

Step 2: To a solution of 5-chloro-2-phenyl-1H-benzo[d]imidazole (0.23g, 1.0 mmol) in (30mL) saturated aq NaHCO₃ was added benzoyl chloride (0.13mL, 1.1 mmol) and the reaction mixture was stirred at room temperature for overnight. After completion of reaction, the reaction mixture was diluted with water and extracted with DCM (50 mL x 2). The combined organic layer was washed with water (50 mL), brine solution (50 mL), dried over anhydrous sodium sulfate and concentrated under reduced pressure to afford crude product, which was purified by flash chromatography [silica gel 100-200 mesh; elution 0-10% EtOAc in hexane] to afford the first isomer (Peak 1)(5-chloro-2-phenyl-1H-benzo[d]imidazol-1-yl)(phenyl)methanone (45 mg) as white solid.

Compound 429: LCMS- 332.2 (M)⁺ UPLC @ 254 nm = 99.04% and @ 220 nm = 99.39%. ¹H NMR (400 MHz, DMSO-*d*₆): δ 7.95 (s, 1 H), 7.74 (*d, J*=7.89 Hz, 2 H), 7.51 - 7.65 (*m,* 3 H), 7.29 - 7.45 (*m,* 7H).

Flash chromatography also afforded the second isomer (Peak 2), (6-chloro-2-phenyl-1H-benzo[d]imidazol-1-yl)(phenyl)methanone (25mg) as white solid.

Compound 430: LCMS- 332.2 (M)+; UPLC @ 254 nm = 97.48% and @ 220 nm = 98.98%. ¹H NMR (400 MHz, DMSO-*d*₆): δ 7.87 (*d, J*=8.77 Hz, 1 H), 7.73 (*d, J*=7.45 Hz, 2 H), 7.54 - 7.61 (*m,* 3 H), 7.37 - 7.50 (*m,* 4 H), 7.28 - 7.34 (*m,* 3 H).

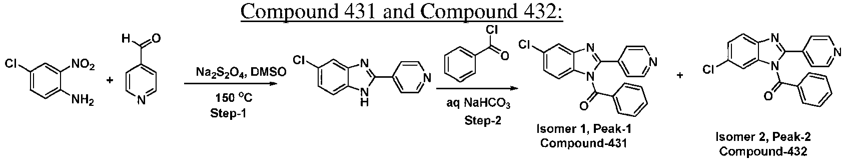

Steps 1 and 2: were performed as described above for synthesis of Compound 429 LCMS: 230.1 (M)+

Compound 431: LCMS- 334.2 (M)+; UPLC @ 254 nm = 99.41% and @ 220 nm = 96.62%. ¹H NMR (400 MHz, DMSO-*d*₆): δ 8.54 (*d, J*=6.14 Hz, 1 H), 8.01 (d*, J*=2.19 Hz, 1 H), 7.79 *(d, J*=7.89 Hz, 2 H), 7.61 - 7.68 *(m,* 2 H), 7.57 (*d, J*=6.14 Hz, 2 H), 7.39 - 7.46 (*m,* 3 H), 7.30 - 7.36 (*m,* 1 H).

Compound 432: LCMS- 334.2 (M)⁺; UPLC @ 254 nm = 99.64% and @ 220 nm = 98.98%. ¹H NMR (400 MHz, DMSO-*d*₆): δ 8.53 (*d,* J=4.82 Hz, 1 H), 7.93 (*d, J*=8.77 Hz, 1 H), 7.78 (*d*, *J*=7.02 Hz, 2 H), 7.59 - 7.66 *(m,* 1 H), 7.51 - 7.58 (*m,* 2 H), 7.40 - 7.51 (*m,* 2 H), 7.30 - 7.40 *(m,* 3 H).

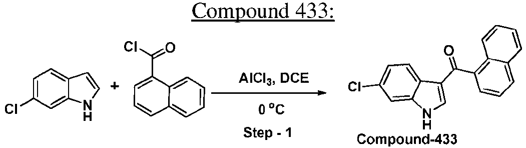

### Step 1 and 2: were performed as described above for synthesis of Compound 359 Compound 433: LCMS- 306.1 (M)+;

UPLC @ 254 nm = 95.87%, @ 220 nm = 99.10%. ¹H NMR (400 MHz, DMSO-*d*₆)*: δ* 12.14 (*br. s.,* 1H), 8.28 (*d, J =* 8.33 Hz, 1H), 8.10 *(d, J* = 8.33 Hz, 1H), 8.00 *(d, J =* 8.33 Hz, 1H), 8.04 (*d, J =* 7.45 Hz, 1H), 7.69 - 7.75 *(m,* 2H), 7.46 - 7.66 (*m,* 4H), 7.31 *(d, J* = 8.33 Hz, 1H).

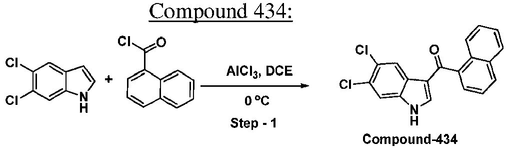

### Step 1 was performed as described above for synthesis of Compound 359

LCMS: 340.2. (M)⁺ ; UPLC @ 254 nm = 98.33% and @220 nm = 98.36%. ¹H NMR (400 MHz, DMSO-*d*₆): δ 12.30 (*br. s.,* 1H), 8.46 (s, 1H), 8.12 (*d, J =* 8.33 Hz, 1H), 7.99 - 8.07 (*m,* 2H), 7.83 (s, 1H), 7.79 (s, 1H), 7.73 *(d, J =* 6.58 Hz, 1H), 7.49 - 7.65 (*m,* 3H).

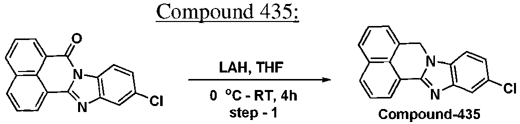

Step-1: To a suspension of 6-chloro-3,10-diazapentacyclo-[10.7.1.0²,^{1⁰}.0⁴,⁹.0^{1⁶},^{2⁰}]icosa-1(19),2,4(9),5,7,12,14,16(20),17-nonaen-11-one (0.140 g, 0.459 mmol) in THF (5.0 ml) at 0 °C was added lithium aluminium hydride (0.104 g, 2.754 mmol) portion-wise. The reaction mixture was then stirred for 4h. The reaction mixture was quenched using ice-cold water, NaOH solution and stirred for 10.0 min. The reaction mixture was then extracted using ethyl acetate and concentrated to get 150 mg of crude product. The crude product was purified using prep chromatography and resulted in 12 mg of Compound 435(6-chloro-3,10-diazapentacyclo[10.7.1.0²,^{1⁰}.0⁴,⁹.0^{1⁶},^{2⁰}]icosa-1(19),2,4(9),5,7,12,14,16(20),17-nonaene) as a white solid.

Compound 435: LCMS-291.1 (M)⁺; HPLC @ 220 nm = 98.09%, UPLCMS @ 220 nm = 97.92 %, @ 220 nm = 97.92 = 97.68 %. ¹H NMR (400 MHz, Chloroform-d) δ 8.40 (*d, J =* 7.2 Hz, 1H), 8.10 (*d, J =* 8.3 Hz, 1H), 8.02-7.94 (*m,* 1H), 7.81 (s, 1H), 7.72-7.67 (*m,* 4H), 7.37 (*dd, J =* 8.5, 2.0 Hz, 1H), 5.92 (s, 2H).

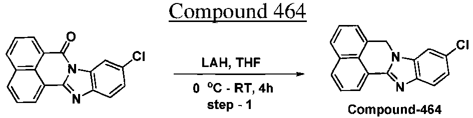

### Step 1 was performed as described above for synthesis of Compound 435

Compound 464: LCMS- 290.9 (M)+, HPLC @ 220 nm = 99.29 %, UPLCMS @ 220 nm = 98.90 %, @ 254 nm = 99.44 %. ¹H NMR (400 MHz, DMSO-*d*₆) δ 8.39 *(d, J =* 7.1 Hz, 1H), 8.09 *(d, J =* 8.3 Hz, 1H), 7.98 *(d, J =* 7.6 Hz, 1H), 7.81 - 7.63 *(m,* 5H), 7.31 (*dd*, *J* = 8.5, 2.1 Hz, 1H), 5.90 (s, 2H).

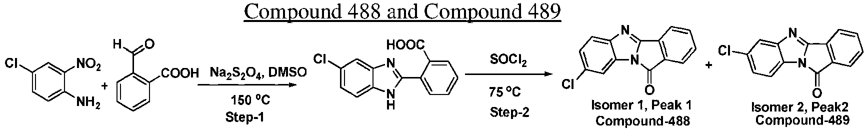

### Step 1 was performed as described above for synthesis of Compound 429

Step 2: A solution of -(5-chloro-1H-benzo[d]imidazol-2-yl)benzoic acid ( 0.27g, 1.0 mmol) in SOCl₂ (2mL) was heated at 70°C for 2 h and the progress of the reaction was monitored by TLC. After completion of reaction, solvent was evaporated, neutralized with sodium bicarbonate and extracted with DCM (50 mL x 2). The combined organic layer was washed with water (50 mL), brine solution (50 mL), dried over anhydrous sodium sulfate and concentrated under reduced pressure to afford crude product, which was purified by flash chromatography (elution" 0-10% EtOAc in hexane) to afford isomer 1 (peak 1), 4-chloro-1,8-diazatetracyclo[7.7.0.0²,⁷.0^{1⁰},^{1⁵}]hexadeca-2,4,6,8,10,12,14-heptaen-16-one (15 mg) as a light yellow solid.

Compound 488: LCMS- 254.9 (M)+ UPLC @ 254 nm = 98.78% and @ 220 nm = 98.87%. ¹H NMR (400 MHz, DMSO-*d*₆): δ 7.90 (*t, J =* 6.80 Hz, 2H), 7.75 - 7.83 (*m,* 2H), 7.62 - 7.75 (*m,* 2H), 7.42 (*dd, J =* 1.75, 8.33 Hz, 1H).

Flash chromatography [silica gel 100-200 mesh; elution 0-10% EtOAc in hexane] also afforded the second isomer (Peak 2), 5-chloro-1,8-diazatetracyclo-[7.7.0.0²,⁷.0^{1⁰},^{1⁵}]hexadeca-2,4,6,8,10,12,14-heptaen-16-one (105mg) as a light yellow solid

Compound 489: LCMS- 254.9 (M)+; UPLC @ 254 nm = 93.74% and @ 220 nm = 92.17%. ¹H NMR (400 MHz, DMSO-*d*₆): δ 7.91 (*dd, J =* 3.29, 7.24 Hz, 2H), 7.73 - 7.83 *(m,* 2H), 7.61 - 7.73 (*m,* 2H), 7.37 *(dd, J =* 1.97, 8.55 Hz, 1H).

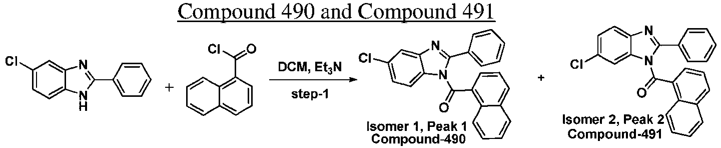

Step 1: To a solution of 5-chloro-2-phenyl-1H-benzo[d]imidazole (0.23g, 1.0 mmol) in (10mL) DCM was added Et₃N (0.57ml, 4.0 mmol) followed by 1-naphthoyl chloride (0.2 mL, 1.1 mmol) and the reaction mixture was stirred at rt for overnight. After completion of reaction, the reaction mixture was diluted with water and extracted with DCM (50 mL x 2). The combined organic layer was washed with water (50 mL), brine solution (50 mL), dried over anhydrous sodium sulfate and concentrated under reduced pressure to afford crude product, which was purified by flash chromatography (elution: 0-10% EtOAc in hexane) to afford the isomer 1 (peak 1) (5-chloro-2-phenyl-1H-benzo[d]imidazol-1-yl)(naphthalen-1-yl)methanone (25mg) as an off white solid.

Compound 490: LCMS- 383.0 (M)+ UPLC @ 254 nm = 99.78% and @ 220 nm = 99.05%. ¹H NMR (400 MHz, DMSO-*d*₆): δ 8.15 (*d, J =* 8.33 Hz, 1H), 8.02 (*d, J =* 8.33 Hz, 1H), 7.89 - 7.99 (*m,* 2H), 7.57 - 7.71 *(m,* 4H), 7.44 *(d, J* = 7.89 Hz, 3H), 7.34 (*t, J =* 7.67 Hz, 1H), 7.12 (*d, J =* 7.45 Hz, 1H), 6.97 - 7.04 (*m,* 2H).

Flash chromatography also afforded the second isomer (Peak 2), 6-chloro-2-phenyl-1H-benzo[d]imidazol-1-yl)(naphthalen-1-yl)methanone (15 mg, 3.9%) as a light brown solid.

Compound 491: LCMS- 383.2 (M)+ UPLC @ 254 nm = 97.61% and @ 220 nm = 98.38%. ¹H NMR (400 MHz, DMSO-*d*₆): δ 8.15 *(d, J =* 8.33 Hz, 1H), 7.86 - 8.03 (*m,* 3H), 7.58 - 7.71 (*m,* 4H), 7.49 - 7.53 (*m,* 1H), 7.41 *(d, J =* 7.45 Hz, 2H), 7.33 (*t, J =* 7.67 Hz, 1H), 7.10 (*t*, *J =* 7.02 Hz, 1H), 6.98 (*t, J =* 7.67 Hz, 2H).

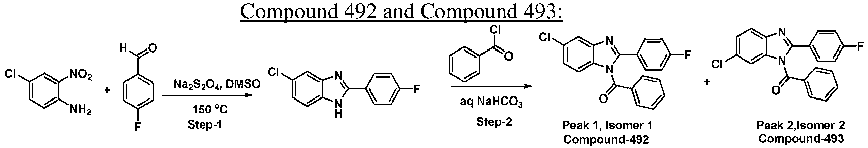

### Steps 1 and 2 were performed as described above for synthesis of Compound 429

Compound 492: LCMS- 351.2 (M)⁺; UPLC @ 254 nm = 98.96% and @ 220 nm = 97.44%. ¹H NMR (400 MHz, DMSO-*d*₆): δ 7.94 (s, 1H), 7.73 (*d, J =* 7.45 Hz, 2H), 7.57 - *7.67* (*m,* 3H), 7.34 - 7.51 (*m,* 4H), 7.17 (*t, J =* 8.77 Hz, 2H).

Compound 493: LCMS- 351.2 (M)⁺; UPLC @ 254 nm = 99.58% and @ 220 nm = 99.31%. ¹H NMR (400 MHz, DMSO-*d*₆): δ 7.87 (*d, J =* 8.33 Hz, 1H), 7.72 (*d, J =* 7.45 Hz, 2H), 7.58 - 7.67 (*m,* 3H), 7.35 - 7.49 (*m,* 4H), 7.15 (*t, J =* 8.77 Hz, 2H).

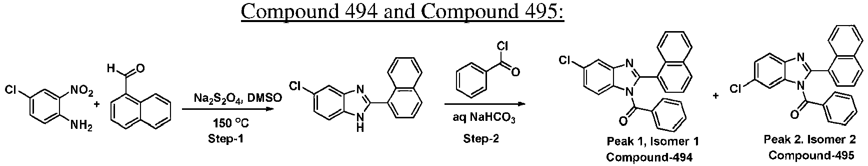

### Steps 1 and 2 were performed as described above for synthesis of Compound 429

Compound 494: LCMS- 383.2 (M)⁺; UPLC @ 254 nm = 98.60% and @ 220 nm = 97.83%.¹H NMR (400 MHz, DMSO-*d*₆): δ 7.99 - 8.07 (*m,* 2 H), 7.82 - 7.91 (*m,* 2 H), 7.43 - 7.63 (*m,* 7 H), 7.33 - 7.43 (*m,* 2 H), 7.10 (*t*, *J*=7.89 Hz, 2 H)

Compound 495: LCMS- 383.2 (M)⁺; UPLC @ 254 nm = 96.78% and @ 220 nm = 94.04%. ¹H NMR (400 MHz, DMSO-*d*₆): δ 8.03 (*d, J*=8*.*77 Hz, 1 H), 7.94 (*d, J*=8.33 Hz, 1 H), 7.88 (*t, J*=8.77 Hz, 2 H), 7.43 - 7.65 (*m,* 7 H), 7.32 - 7.43 (*m*, 2 H), 7.08 *(t, J*=7.45 Hz, 2 H).

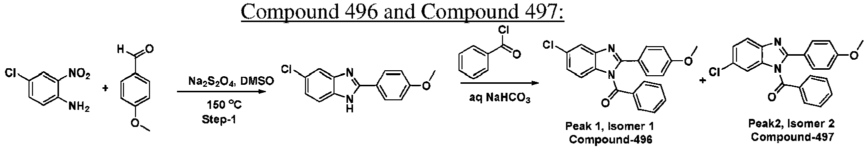

Steps 1 and 2 were performed as described above for synthesis of Compound 429.

Compound 496: LCMS- 363.3 (M)⁺; UPLC @ 254 nm = 99.58% and @ 220 nm = 99.30%. ¹H NMR (400 MHz, DMSO-*d*₆): δ 7.87 *(d, J*=1.75 Hz, 1 H), 7.70 (d, *J*=7.45 Hz, 2 H), 7.59 - 7.63 (*m,* 1 H), 7.50 (*m,* J=8.77 Hz, 2 H), 7.41 (*t, J*=7.67 Hz, 2 H), 7.27 - 7.34 *(m,* 2 H), 3.70 (s, 3 H) 6.85 (*m, J*=8.77 Hz, 2 H).

Compound 497: LCMS- 363.3 (M)⁺; UPLC @ 254 nm = 97.14% and @ 220 nm = 95.46%. ¹H NMR (400 MHz, DMSO-*d*₆): δ 7.80 (*d, J*=8.33 Hz, 1 H), 7.65 - 7.72 (*m,* 2 H), 7.57 - 7.63 (*m,* 1 H), 7.49 (*m, J*=8.77 Hz, 2 H), 7.41 (*t, J*=7.67 Hz, 3 H), 7.32 (*d, J*=1.75 Hz, 1 H), 6.84 (m, *J*=8.77 Hz, 2 H), 3.69 (s, 3 H).

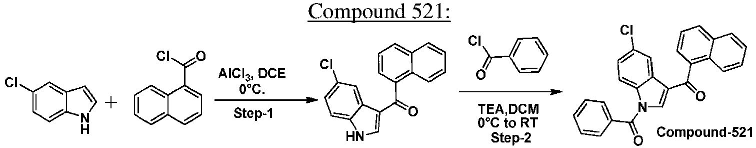

### Step 1 was performed as described above for synthesis of Compound 359

### Step 2 was performed as described above for synthesis of Compound 490

Compound 521: LCMS - 410.3(M)⁺, UPLC @ 254 nm = 99.67%, % and @ 220 nm = 99.58%. ¹H NMR (400 MHz, DMSO-*d*₆): δ 8.40 (d, *J* = 1.96 Hz, 1H), 8.29 (s, 1H), 8.11 - 8.19 (*m,* 2H), 7.98 - 8.04 *(m,* 1H), 7.90 (*d, J* = 6.36 Hz, 1H), 7.80 (*d, J* = 7.34 Hz, 2H), 7.59 - 7.66 (*m,* 6H), 7.47 - 7.52 *(m,* 2H).

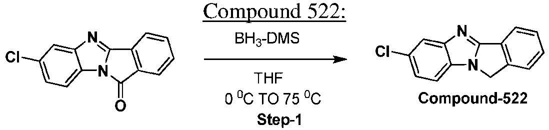

Step 1: 7-chloro-11H-benzo[4,5]imidazo[2,1-a]isoindol-11-one (0.500 g, 1.96 mmol, 1.0 eq) was dissolved in THF (10.0 ml) and cooled to 0°C. BH₃-DMS (0.3 ml, 2.95 mmol, 1.5 eq) was slowly added to it. After few minutes of stirirng the reaction mixture was refluxed at 75°C for 16 hours. After completion, the reaction mixture was cooled to 0°C and was slowly quenched with methanol (100.0 ml). The reaction mixture was dried under vacuum to give a crude Compound . The crude was purified by prep chromatography to afford 7-chloro-11H-benzo[4,5]imidazo[2,1-a]isoindole (40 mg) as an off white solid.

Compound 522: LCMS- 241.2 (M)⁺ UPLC @ 254 nm = 98.69% and @ 220 nm: 98.10%. ¹H NMR (400 MHz, DMSO-*d*₆): δ 7.97 (*d, J =* 4.89 Hz, 1H), 7.79 (*br. s.,* 2H), *7.69* (*d, J* = 8.31 Hz, 1H), 7.50 - 7.64 (*m,* 2H), 7.24 (*d, J =* 9.29 Hz, 1H), 5.27 (s, 2H).

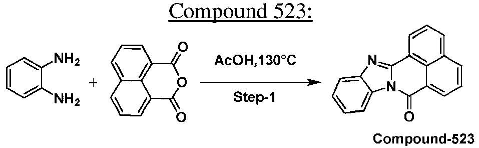

### Step 1 was performed as described above for synthesis of Compound 362 (procedure 1)

Compound 523: LCMS- 271.2 (M+H)⁺ UPLC@ 254 nm= 97.46% and @ 220 nm = 99.46%. ¹H NMR (400 MHz, DMSO-*d*₆): δ 8.78 (*d, J* = 7.34 Hz, 1H), 8.74 (*dd, J =* 0.98, 7.34 Hz, 1H), 8.56 (*d, J =* 7.83 Hz, 1H), 8.47 (*dd, J =* 2.93, 5.87 Hz, 1H), 8.41 (*d, J* = 7.83 Hz, 1H), 7.91 - 7.99 (*m,* 2H), 7.89 (*br. s.,* 1H), 7.44 - 7.54 (*m,* 2H).

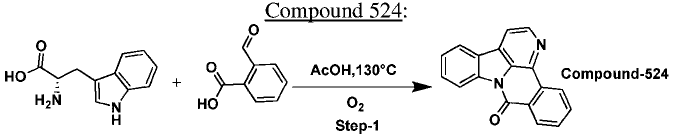

Step 1: L-tryptophan (1.0 g, 73.45 mmol) was dissolved in acetic acid (10.0 ml). This was followed by addition of 2-formylbenzoic acid (0.800 g, 80.80 mmol, 1.1 eq) to it. The resulting mixture was stirred at 130°C for 16 hours. After this, the reaction mixture was stirred under oxygen at same temperature for another 16 hours. The progress of reaction was monitored by LCMS. After completion, the reaction mixture was poured in ice cold water (500.0 mL) which resulted in precipitation of a solid that was filtered, washed with water (500.0mL) and dried under vacuum to afford 7H-benzo[de]benzo[4,5]imidazo[2,1-a]isoquinolin-7-one (1.1 g) as a yellow solid.

Compound 524: LCMS- 271.2 (M+H)⁺ UPLC@ 254 nm = 98.70% and @ 220 nm = 99.09%. ¹H NMR (400 MHz, DMSO-*d*₆): δ 8.89 *(d, J =* 4.89 Hz, 1H), 8.78 (*d, J =* 7.83 Hz, 1H), 8.68 (*d, J =* 7.83 Hz, 1H), 8.55 (*d, J =* 8.31 Hz, 1H), 8.44 (*d, J =* 8.31 Hz, 1H), *8.33* (*d, J =* 4.89 Hz, 1H), 8.04 (*t, J =* 7.09 Hz, 1H), 7.79 - 7.89 (*m,* 2H), 7.64 (*t, J =* 7.58 Hz, 1H).

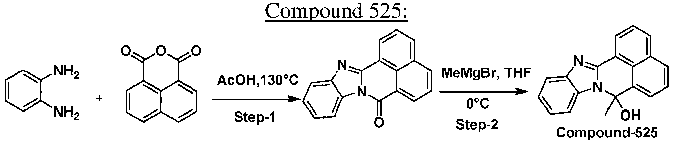

Step 1 was performed as described above for synthesis of Compound 362 (procedure 1).

Step 2: 7H-benzo[de]benzo[4,5]imidazo[2,1-a]isoquinolin-7-one (0.150 g, 0.55 mmol, 1.0 eq) was dissolved in THF (2.0 ml) and cooled to 0°C. This was followed by addition of methyl magnesium bromide (2M in THF, 1.4 ml, 2.78 mmol, 5.0 eq) to it. The resulting mixture was stirred at same temperature for one hour. The progress of reaction was monitored by LCMS. After completion, the reaction mixture was quenched with saturated solution of ammonium chloride and extracted with ethyl acetate. The organic layer was concentrated under vacuum to give crude product which was purified by column chromatography (0-60 % EtOAc:Hexane as effluent), to afford 7-methyl-7H-benzo[de]benzo[4,5]imidazo[2,1-a]isoquinolin-7-ol (120 mg) as an off yellow solid.

Compound 525: LCMS- 287.2 (M+H)⁺ UPLC@ 254 nm= 94.48% and @ 220 nm= 95.05%. ¹H NMR (400 MHz, DMSO-*d*₆): δ 8.50 (*d, J =* 6.85 Hz, 1H), 8.06 - 8.16 *(m,* 2H), 8.01 (s, 2H), 7.71 - 7.81 (*m,* 4H), 7.22 - 7.32 *(m,* 2H), 1.98 (s, 3H).

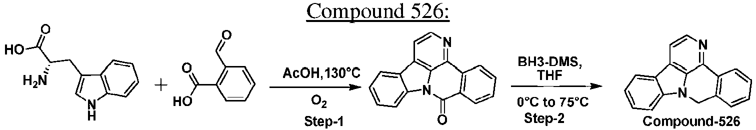

### Step 1 was performed as described above for synthesis of Compound 524

### Step 2 was performed as described above for synthesis of Compound 522

Compound 526: LCMS- 257.2 (M+H)⁺ UPLC:@ 254 nm = 97.35% and @ 220 nm= 98.52%.

¹H NMR (400 MHz, DMSO-*d*₆): *δ* 8.38 *(d, J =* 4.89 Hz, 1H), 8.23 - 8.34 (*m,* 2H), *7.96* (*d, J* = 4.89 Hz, 1H), 7.61 - 7.72 (*m,* 2H), 7.49 (*d, J =* 3.91 Hz, 3H), 7.35 (*t, J =* 7.34 Hz, 1H), 5.77 (s, 2H).

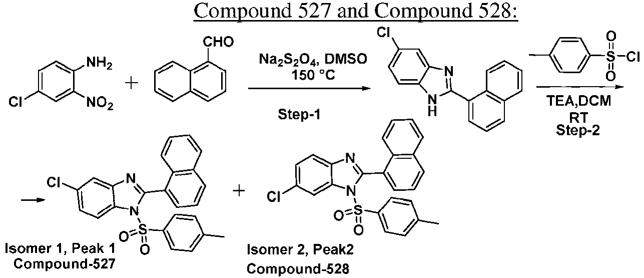

### Step 1 was performed as described above for synthesis of Compound 429

Step 2: To a solution of 5-chloro-2-(naphthalen-1-yl)-1H-benzo[d]imidazole (100 mg, 0.3597 mmol) in DCM (7 mL) was added TEA (0.15 mL, 1.0791 mmol) and the resultant reaction mixture was stirred for 15 minutes followed by addition of 4-methylbenzenesulfonyl chloride (68 mg, 0.3597 mmol). The reaction was stirred overnight at room temperature. Reaction was monitored by TLC and LCMS. After completion of reaction mixture was diluted with DCM (100 mL) and washed with sodium bicarbonate solution (2×100 mL). The organic layer was separted and dried over sodium sulphate, concentrated under reduced pressure to yield crude product which was purified by flash chromatography (elution: 0-10% EtOAc in hexane) to afford first isomer (peak 1) 5-chloro-2-(naphthalen-1-yl)-1-tosyl-1H-benzo[d]imidazole (11.48 mg) as a yellow solid.

Compound 527: LCMS- 433.1 (M)⁺ UPLC @ 254 nm = 96.11%, @ 220 nm = 97.33%. ¹H NMR (Peak 1)- (400 MHz, DMSO-*d*₆) δ ppm 8.18 (d, *J*=8.80 Hz, 2 H) 8.04 (*d, J*=8.31 Hz, 1 H), 7.96 (*d, J*=1.96 Hz, 1 H) 7.52 - 7.67 (m, 4 H) 7.36 (*br. s.,* 1 H) 7.30 (*m, J=8.31* Hz, 2 H) 7.20 (*d, J*=8.31 Hz, 1 H) 7.15 (*m, J*=8.31 Hz, 2 H) 2.25 (s, 3 H).

Flash chromatography also afforded the second isomer (Peak 2), 6-chloro-2-(naphthalen-1-yl)-1-tosyl-1H-benzo[d]imidazole (8.16 mg, 5.26 %) as a yellow solid.

Compound 528: LCMS- 433.2(M)⁺ UPLC @ 254 nm = 96.94%, @ 220 nm = 96.27 %. ¹H NMR (Peak 2) (400 MHz, DMSO-*d*₆) δ ppm 8.15 - 8.21 (*m,* 2 H) 8.02 *(d, J*=8.31 Hz, 1 H) 7.87 (*d, J*=8.31 Hz, 1 H) 7.61 - 7.68 (*m,* 2 H) 7.51 - 7.59 (*m,* 2 H) 7.26 - *7.37* (*m,* 3 H) 7.08 - 7.18 (*m,* 3 H) 2.24 (s, 3 H).

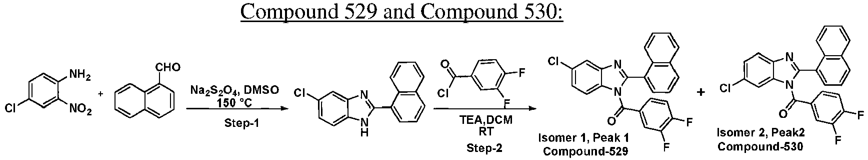

### Steps 1 and 2 were performed as described above for synthesis of Compounds 429 and 490

Compound 529: LCMS- 419.2 (M)⁺ UPLC @ 254 nm = 99.35%, @ 220 nm = 99.03%. ¹H NMR (Peak 1) (400 MHz, DMSO-*d*₆) δ ppm 8.03 (*d, J*=1.96 Hz, 1 H) 7.92 (*d, J*=7.34 Hz, 4 H) 7.81 (*d, J*=8.80 Hz, 1 H) 7.64 (s, 1 H) 7.50 - 7.60 (*m,* 5 H) 7.42 (s, 1 H).

Compound 530: LCMS- 419.1(M)⁺ UPLC @ 254 nm = 98.81%, @ 220 nm = 97.93%. ¹ H NMR (DMSO-*d*₆) δ ppm 1.23 (*br. s.,* 3 H), 7.40 (*br. s.,* 2 H), 7.56 (*d,* 3 H), 7.63 (*d,* 2 H), 7.85 - 7.98 (*m,* 3H).

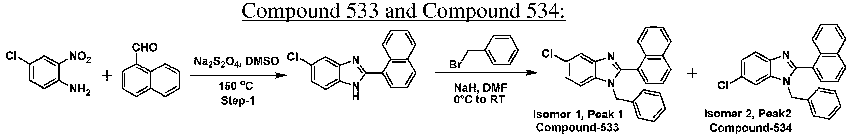

### Step 1 was performed as described above for synthesis of Compound 429

Step 2: To an ice-cold solution of 5-chloro-2-(naphthalen-1-yl)-1H-benzimidazole (0.300 g, 1.07 mmol) in DMF (5 mL), sodium hydride (0.065 g, 1.61 mmol) was added. After five minutes of stirring, (bromomethyl)benzene (0.15 mL, 1.18 mmol) was added and the reaction mixture was stirred at room temperature for two hours. After completion of reaction, the reaction mixture was quenched with ice, extracted with ethyl acetate (50 mL x 2). The combined organic layer was washed with water (50 mL), brine solution (50 mL), dried over anhydrous sodium sulfate and concentrated under reduced pressure to afford crude product, which was purified by flash chromatography (elution: 0-10% EtOAc in hexane) to afford the (Peak1), 1-benzyl-5-chloro-2-(naphthalen-1-yl)-1H-benzimidazole (10 mg) as a white solid.

Compound 533: LCMS- 369.3 (M)⁺ UPLC @ 254 nm = 95.31% and @ 220 nm = 97.23%. Peak 1: ¹H NMR (400 MHz, DMSO-*d*₆) δ ppm 8.05 (s, 1 H) 7.86 (*d,* J=1.96 Hz, 1 H) 7.67 - 7.74 (*m,* 3 H) 7.64 (s, 1 H) 7.58 (*d*, *J*=8.80 Hz, 3 H) 7.10 - 7.20 (*m,* 4 H) 6.80 - 6.88 (*m,* 2 H) 5.34 (s, 2 H).

Flash chromatography also afforded the second isomer (Peak2) 1-benzyl-5-chloro-2-(naphthalen-1-yl)-1H-benzimidazole (15 mg, 3.7 %) as a white solid.

Compound 534: LCMS- 369.3 (M)⁺ UPLC @ 254 nm = 97.12% and @ 220 nm = 96.80%. Peak 2: ¹H NMR (400 MHz, DMSO-*d*₆) δ ppm 8.15 (*d, J*=8.31 Hz, 1 H) 8.06 (*d, J*=7.83 Hz, 1 H) 7.80 (*d, J*=8.31 Hz, 1 H) 7.64 - 7.74 (*m,* 3 H) 7.59 (*d, J*=6.85 Hz, 1 H) 7.63 (*d, J*=7.34 Hz, 1 H) 7.53 (*d, J*=8.31 Hz, 1 H) 7.33 (*dd, J*=8.80, 1.96 Hz, 1 H) 7.11 - 7.20 (*m,* 3 H) 6.81 - 6.88 (*m*, 2 H) 5.34 (s, 2 H).

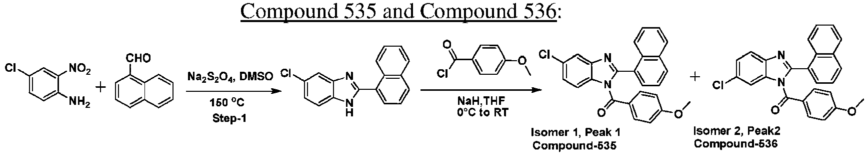

### Step 1 was performed as described above for synthesis of Compound 429

Step 2: To an ice-cold solution of 5-chloro-2-(naphthalen-1-yl)-1H-benzimidazole (0.100 g, 0.35 mmol, 1.0 eq) in THF (5 mL), sodium hydride (0.021 g, 0.53 mmol, 1.5 eq) was added. After five minutes of stirring, 4-methoxybenzoyl chloride (0.05 mL, 0.39 mmol, 1.1 eq) was added and the reaction mixture was stirred at room temperature for two hours. After completion of reaction, the reaction mixture was quenched with ice, diluted with water and extracted with ethyl acetate (50 mL x 2). The combined organic layer was washed with water (50 mL), brine solution (50 mL), dried over anhydrous sodium sulfate and concentrated under reduced pressure to afford crude product, which was purified by flash chromatography (elution: 0-10% EtOAc in hexane) to afford the desired isomer 1 (Peak1), (5-chloro-2-(naphthalen-1-yl)-1H-benzo[d]imidazol-1-yl)(4-methoxyphenyl)methanone (10 mg) as a white solid.

Compound 535: LCMS- 413.2 (M)⁺ UPLC @ 254 nm = 93.31% and @ 220 nm 98.38%. Peak1: ¹H NMR (400 MHz, DMSO-*d*₆) d δ ppm 8.01 (s, 1 H), 7.95 (*d, J*=7.34 Hz, 2 H), 7.61 (*d,* J=8.80 Hz, 2 H), 7.53 - 7.59 (*m,* 3 H), 7.43 (s, 4 H), 6.75 (*d, J*=8.31 Hz, 2 H), 3.72 (s, 3 H).

Flash chromatography also afforded the second isomer (Peak2) (6-chloro-2-(naphthalen-1-yl)-1H-benzo[d]imidazol-1-yl)(4-methoxyphenyl)methanone (10 mg) as a white solid.

Compound 536: LCMS- 413.2 (M)⁺ UPLC @ 254 nm = 98.48% and @ 220 nm = 97.90%. Peak 2: ¹H NMR (400 MHz, DMSO-*d*₆) δ ppm 7.93 (d, *J*=8.80 Hz, 3 H), 7.51 - 7.64 (*m,* 6 H), 7.49 (br.s., 3 H), 6.71 (*d, J*=8.80 Hz, 2 H), 3.71 (s, 3 H).

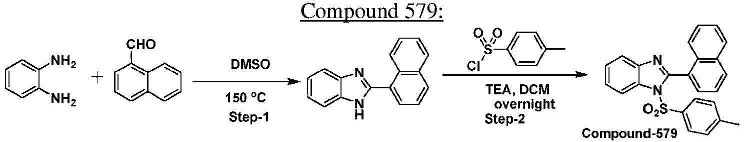

Step 1: To a solution of benzene-1,2-diamine (5 g, 46.236 mmol) in DMSO (20 mL), benzaldehyde (7.94 g, 50.859 mmol) was added and the reaction mixture was heated at 150 ⁰C for 3h. After completion of reaction, solution was diluted with water which resulted in precipitation of a solid which was filtered washed with ether and dried to give the desired product as 2-(naphthalen-1-yl)-1H-benzo[d]imidazole (6g) as a yellow solid. LCMS: 245.2 (M+H)⁺

### Step 2: was performed as described above for synthesis of Compound 527

Compound 579: LCMS- 399.1 (M+H)⁺ UPLC @ 254 nm = 99.43%, @ 220 nm = 99.07%. ¹H NMR (400 MHz, DMSO-d6) δ 8.17 (t, J = 8.1 Hz, 2H), 8.04 (d, J = 8.2 Hz, 1H), 7.83 (d, J = 7.8 Hz,1H), 7.71-7.60 (m, 2H), 7.52 (dt, J = 24.9, 7.3 Hz, 3H), 7.43-7.31 (m, 3H), 7.24 (d, J = 8.5 Hz, 1H),7.17 (d, J = 8.1 Hz, 2H), 2.25 (s, 3H).

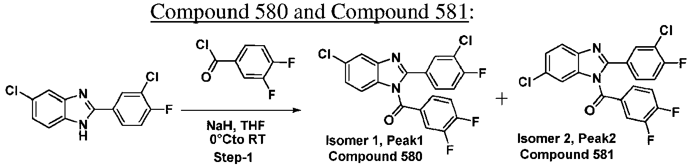

### Step 1 was performed as described above for step 2 of synthesis of Compound 535

Compound 580: LCMS-421.2 (M)⁺ UPLC @ 254 nm = 98.71%, @ 220 nm = 96.63%. ¹H NMR- (400 MHz, DMSO-*d*₆) δ ppm 7.42 (s, 1 H) 7.44 - 7.48 (*m*, 2 H) 7.52 (*br. s.,* 1 H) 7.61 (*d, J*=8.77 Hz, 2 H) 7.83 (*d, J*=6.58 Hz, 1 H) 7.91 (*br. s.,* 1 H) 7.96 (*d, J*=2.19 Hz, 1 H).

Compound 581: LCMS-421.2 (M)⁺ UPLC @ 254 nm = 97.97%, @ 220 nm = 95.09%. Peak 2: ¹H NMR (400 MHz, DMSO-*d*₆) δ ppm 7.47 (s, 1 H) 7.50 (*d, J*=2.19 Hz, 1 H) 7.52 (*d, J*=2.19 Hz, 1 H) 7.57 - 7.62 (*m,* 2 H) 7.72 (*d, J*=2.19 Hz, 1 H) 7.80 (*dd, J*=7.45*,* 2.19 Hz, 1 H) 7.88 (s, 1 H) 7.89 - 7.91 (*m,* 1 H).

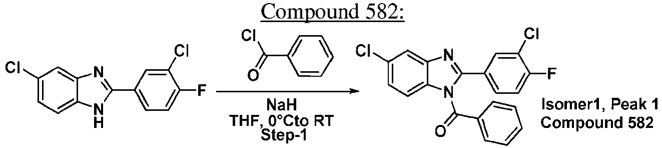

### Step 1 was performed as described above for step 2 of synthesis of Compound 535

Compound 582: LCMS- 385.1 (M)⁺ UPLC @ 254 nm = 97.91%, @ 220 nm = 96.50% ¹H NMR (400 MHz, DMSO-*d*₆) δ ppm 7.41 - 7.46 (*m,* 4 H) 7.49 - 7.53 (*m,* 2 H) 7.60 (*d, J*=7.45 Hz, 2 H) 7.72 (*d, J*=7.02 Hz, 2 H) 7.96 (*d,* J=1.75 Hz, 1 H).

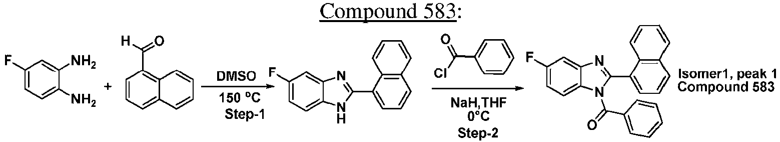

### Step 1 was performed as described above for step 1 of synthesis of Compound 579

### Step 2 was performed as described above for step 2 of synthesis of Compound 535

Compound 583: LCMS- 367.2 (M+H)⁺ UPLC @ 254 nm = 98.18%, @ 220 nm = 96.19%. ¹H NMR (DMSO-*d*₆) δ ppm 7.09 (t, 2 H) 7.31 - 7.38 (m, 2 H) 7.40 (d, 1 H) 7.48 - 7.52 (m, 2 H) 7.53 - 7.58 (m, 2 H) 7.59 - 7.64 (m, 2 H) 7.78 (dd, 1 H) 7.85 - 7.91 (m, 2 H) 8.03 (d, 1 H).

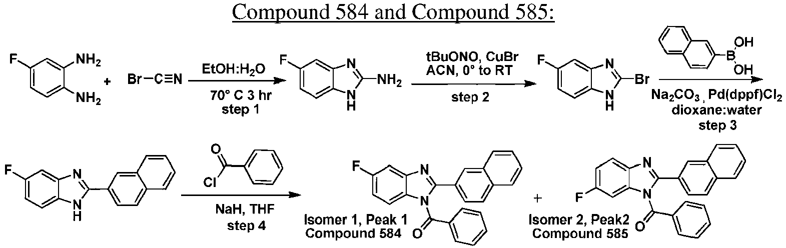

Step 1: A solution 4-fluorobenzene-1,2-diamine (3 g, 23.8 mmol) and cyanogen bromide (3.74g, 35.7 mmol) in EtOH: H₂O (10:10 mL) was heated at 70°C for 3h. After completion of reaction, the reaction mixture was concentrated under reduced pressure to remove ethanol and water and after lypholisation it afforded 5-fluoro-1H-benzo[d]imidazol-2-amine (3.25g) as a brown solid. LCMS: 152.1(M+H)⁺

Step 2: To a solution 5-fluoro-1H-benzo[d]imidazol-2-amine (2 g, 13.2 mmol) in ACN (20 mL)was added CuBr₂(4.43 g , 19.8 mmol) at 0°C portionwise and the reaction mixture was stirred for 15 minutes followed by addition of tertiarybutyl nitrite (2.37mL 19.8mmol) dropwise at 0°C. The reaction mixture was allowed to stir at room temperature for 2 hours. After completion of reaction, the reaction mixture was diluted with water and extracted with ethyl acetate (250 mL x 2). The combined organic layer was washed with water (100 mL), brine solution (100 mL), dried over anhydrous sodium sulfate and concentrated under vacuum to afford the crude product, which was purified by flash chromatography (elution 0-30% EtOAc in hexane) to afford the 2-bromo-5-fluoro-1H-benzo[d]imidazole (0.6 g) as a yellow solid. LCMS- 215.2 (M+H)⁺

Step 3: To a solution of 2-bromo-5-fluoro-1H-benzo[d]imidazole (0.2 g, 0.9 mmol) and naphthalen-2-ylboronic acid (0.241 g, 1.4 mmol) in dioxane (5 mL) was added Na₂CO₃ (0.297 g, 2.8 mmol) which was dissolved in water(1 mL). Then the reaction mixture was purged using nitrogen for 20 minutes followed by addition of Pd(dppf)Cl₂ (0.034 g, 0.0467 mmol). The resulting reaction mixture was heated for 120°C for overnight. Progress of the reaction was monitored by TLC and LCMS. After completion of reaction the reaction mixture was diluted with water and extracted with ethyl acetate (150 mL x 2). The combined organic layer was washed with water (70 mL), brine solution (70 mL), dried over anhydrous sodium sulfate and concentrated under vacuum to afford the crude product, which was purified by flash chromatography (elution: 0-30% EtOAc in hexane) to afford the 5-fluoro-2-(naphthalen-2-yl)-1H-benzo[d]imidazole (0.150 g) as a white solid. LCMS-263.2 (M+H)⁺

Step 4 was performed as described above for step 2 of synthesis of Compound Compound 535.

Compound 584: LCMS-367.3 (M+H)⁺ UPLC @ 254 nm = 93.73%, @ 220 nm = 97.13%. ¹H NMR (400 MHz, DMSO-*d*₆) δ ppm 7.23 - 7.30 (m, 1 H), 7.33 (t, J=7.67 Hz, 2 H), 7.47 (dd, J=8.77, 4.82 Hz, 2 H), 7.51 - 7.58 (m, 2 H), 7.67 - 7.77 (m, 4 H), 7.83 - 7.90 (m, 2 H), 7.94 (d, J=7.89 Hz, 1 H), 8.18 (s, 1 H).

Compound 585: LCMS- 367.1 (M+H)⁺ UPLC @ 254 nm = 98.78%, @ 220 nm = 98.36%. ¹H NMR (400 MHz, DMSO-*d*₆) δ ppm 7.32 (t, *J*=7.89 Hz, 3 H) 7.53 (s, 2 H) 7.66 - 7.76 *(m,* 3 H) 7.83 (s, 2 H) 7.92 (s, 4 H) 8.16 (s, 1 H).

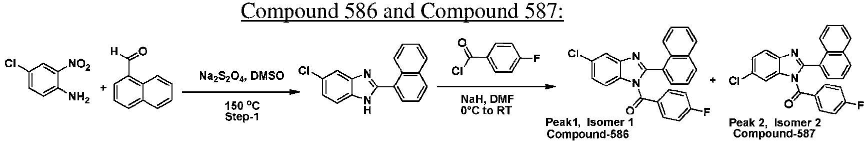

### Step 1 was performed as described above for step 1 of synthesis of Compound 429

Compound 586: LCMS- 401.2 (M)⁺ UPLC @ 254 nm = 99.12% and @ 220 nm 97.31%. ¹H NMR (400 MHz, DMSO-*d*₆) δ ppm 8.02 (*d, J*=2.19 Hz, 1 H) 7.89 - 7.99 (*m,* 3 H) 7.62 - 7.68 (*m,* 2 H) 7.50 - 7.62 (*m,* 4 H) 7.49 (*d, J*=1.75 Hz, 1 H) 7.43 (*d, J*=7.89 Hz, 1 H) 6.90 (t, *J*=8.99 Hz, 2 H)

Compound 587: LCMS- 401.2 (M)⁺ UPLC @ 254 nm = 98.21% and @ 220 nm = 97.84%. ¹H NMR (400 MHz, DMSO-*d*₆) δ ppm 7.86 - 7.98 (*m*, 4 H) 7.72 (*d, J*=1.75 Hz, 1 H) 7.63 (*d, J*=6.58 Hz, 1 H) 7.51 - 7.59 (*m,* 5 H) 7.41 (*t, J*=7.67 Hz, 1 H) 6.86 (*t, J*=8.77 Hz, 2 H).

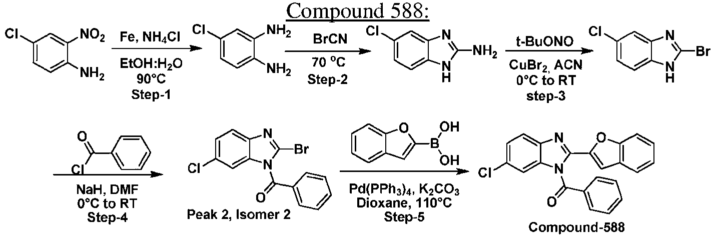

Step 1: To a solution of 4-chloro-2-nitro aniline (5.0 g, 28.9 mmol) and ammonium chloride (15.4 g, 289.9 mmol) in ethanol (50.0 mL) and water (50.0 mL) iron powder (12.9 g, 231.8 mmol) was added and reaction mixture was stirred at 90°C for one hour. After completion of reaction, the reaction mixture was dried under vacuum to get crude product. The crude Compound was washed with ether and organic layer was concentrated to yield 4-chlorobenzene-1,2-diamine (4.0 g) as a brown solid. LCMS: 143.0 (M)⁺

### Steps 2-5 were performed as described for steps 1-4 of synthesis of Compound 584

Compound 588: LCMS- 373.1 (M)⁺ UPLC @ 254 nm = 97.27% and @ 220 nm 94.14%. ¹H NMR (400 MHz, DMSO-d6) δ 7.92 (d, J = 8.4 Hz, 1H), 7.82 (d, J = 7.5 Hz, 2H), 7.67 (d, J = 7.7 Hz, 1H), 7.60 (s, 1H), 7.56 (t, J = 7.5 Hz, 1H), 7.53-7.47 (m, 2H), 7.43 (t, J = 7.6 Hz, 2H), 7.38 (d, J = 8.2 Hz, 1H), 7.33 (t, J = 7.6 Hz, 1H), 7.26 (t, J = 7.4 Hz, 1H).

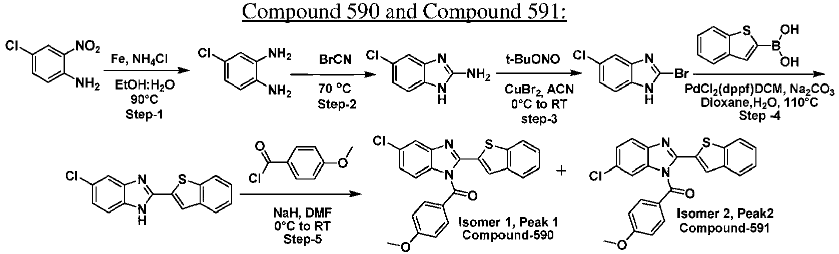

### Steps 1-4 were performed as described for steps 1-4 of synthesis of Compound 588

Compound 590: LCMS- 419.2 (M)⁺ UPLC @ 254 nm = 99.01% and @ 220 nm 99.96%. ¹H NMR (400 MHz, DMSO-*d*₆) δ ppm 8.03 (*d, J*=8.33 Hz, 2 H) 7.96 (*d, J*=1.75 Hz, 1 H) 7.85 - 7.91 (*m,* 2 H) 7.69 (s, 1 H) 7.34 (s, 3 H) 7.15 (s, 1 H) 7.09 (s, 2 H) 3.84 (s, 3 H)

Compound 591: LCMS- 419.3 (M)⁺ UPLC @ 254 nm = 96.73% and @ 220 nm = 96.26%. ¹H NMR (400 MHz, DMSO-d6) δ 8.02 (d, J = 7.9 Hz, 1H), 7.87 (td, J = 9.0, 5.5 Hz, 4H), 7.65 (s, 1H), 7.52-7.34 (m, 3H), 7.19 (d, J = 2.0 Hz, 1H), 7.16-7.03 (m, 2H), 3.84 (s, 3H).

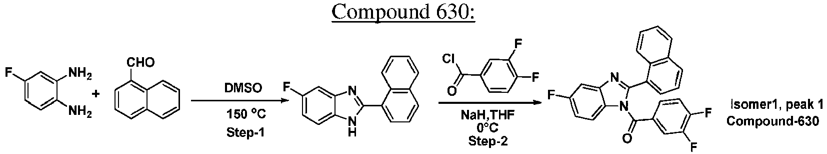

### Step 1 was performed as described for synthesis of Compound 535

Compound 630: LCMS 403.2 (M+H)⁺ UPLC @ 254 nm = 98.30%, @ 220 nm = 96.71 %. ¹H NMR (400 MHz, DMSO-*d*₆) δ ppm 7.02 - 7.11 (*m,* 1 H) 7.29 - 7.39 (*m,* 2 H) 7.39 - 7.45 (*m,* 1 H) 7.53 - 7.61 (*m,* 3 H) 7.64 *(d,* 1 H) 7.79 (*dd,* 1 H) 7.84 (*dd,* 1 H) 7.88 - 7.95 (*m,* 3 H).

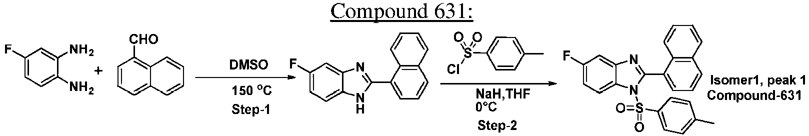

### Step 1 was performed as described for synthesis of Compound 579

Step 2:To a solution of 5-fluoro-2-(naphthalen-1-yl)-1H-benzo[d]imidazole (0.2 g, 0.7633 mmol) in THF (5 mL) was added NaH (0.045 g , 1.1449 mmol) at 0°C and the reaction mixture was stirred for 15 minute followed by addition of 4-methylbenzenesulfonyl chloride (0.145 g,0.7633mmol). The reaction mixture was stirred for 3 hours at room temperature. After completion of reaction, the reaction mixture was diluted with water and extracted with ethyl acetate (50 mL x 2). The combined organic layer was washed with water (50 mL), brine solution (50 mL), dried over anhydrous sodium sulfate and concentrated under vacuum to afford the crude product, which was purified by flash chromatography (elution 0-10% EtOAc in hexane) to afford the peak 1 as isomer 5-fluoro-2-(naphthalen-1-yl)-1-tosyl-1H-benzo[d]imidazole (0.016 g) as a brown solid.

Compound 631: LCMS- 417.1 (M+H)⁺ UPLC @ 254 nm = 94.24%, @ 220 nm = 90.24 %. ¹H NMR (400 MHz, DMSO-*d*₆) δ 8.18 (dd, J = 8.8, 5.0 Hz, 2H), 8.04 (d, J = 8.3 Hz, 1H), 7.75-7.61 (m, 3H), 7.55 (t, J = 7.6 Hz, 1H), 7.43 (td, J = 9.3, 2.6 Hz, 1H), 7.37 (t, J = 7.6 Hz, 1H), 7.31 (d, J = 8.0 Hz, 2H), 7.22 (d, J = 8.5 Hz, 1H), 7.15 (d, J = 8.1 Hz, 2H), 2.25 (s, 3H).

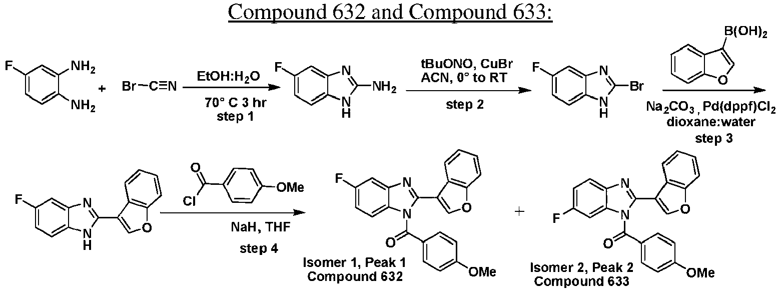

### Steps 1-4 were performed as described for steps 1-4 of synthesis of Compound 584

Compound 632: LCMS- 387.3(M+H)⁺ UPLC @ 254 nm =90.07 %, @ 220 nm =94.62 %.¹H NMR (DMSO-*d*₆) δ ppm 3.79 (s, 3 H) 7.03 (*m,* 2 H) 7.29 - 7.32 (*m,* 2 H) 7.33 - 7.39 (*m,* 2 H) 7.45 (*d,* 1 H) 7.61 (s, 1 H) 7.70 - 7.75 (*m,* 2 H) 7.84 (*m,* 2 H)

Compound 633: LCMS- 387.3 (M+H)⁺ UPLC @ 254 nm =88.75%, @ 220 nm =96.99 %.¹H NMR (DMSO-*d*₆) δ ppm 3.78 (s, 3 H) 7.02 (*d,* 2 H) 7.13 (s, 1 H) 7.24 - 7.40 (*m,* 3 H) 7.56 (s, 2 H) 7.71 (*d,* 2 H) 7.82 *(d,* 2 H).

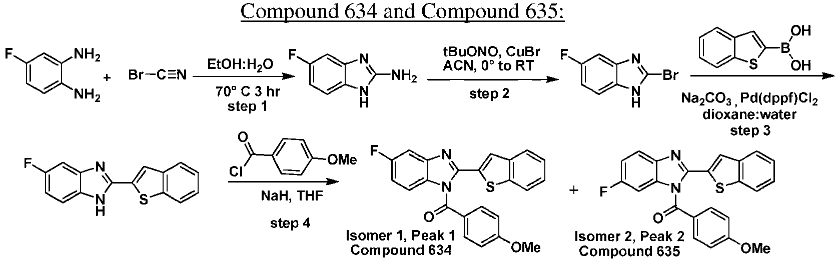

### Steps 1-4 were performed as described for steps 1-4 of synthesis of Compound 584

Compound 634: LCMS-403.2 (M+H)⁺ UPLC @ 254 nm = 98.36%, @ 220 nm =99.04 %. ¹H NMR (400 MHz, DMSO-*d*₆) δ ppm 8.02 *(d, J*=7.89 Hz, 1 H) 7.84 - 7.91 (*m,* 3 H) 7.66 - 7.74 (*m,* 2 H) 7.36 - 7.47 (*m,* 2 H) 7.07 - 7.21 (*m,* 4 H) 3.84 (s, 3 H)

Compound 635: LCMS- 403.2 (M+H)⁺ UPLC @ 254 nm = 98.65%, @ 220 nm = 98.48 %. ¹H NMR (400 MHz, DMSO-*d*₆) δ ppm 8.01 (*d, J*=7.89 Hz, 1 H) 7.81 - 7.93 (*m,* 4 H) 7.63 (s, 1 H) 7.34 - 7.49 (*m,* 2 H) 7.27 (td, *J*=9.32*,* 2.41 Hz, 1 H) 7.09 (*d, J*=8.77 Hz, 2 H) 6.96 (*dd, J*=9.21*,* 2.63 Hz, 1 H) 3.84 (s, 3 H).

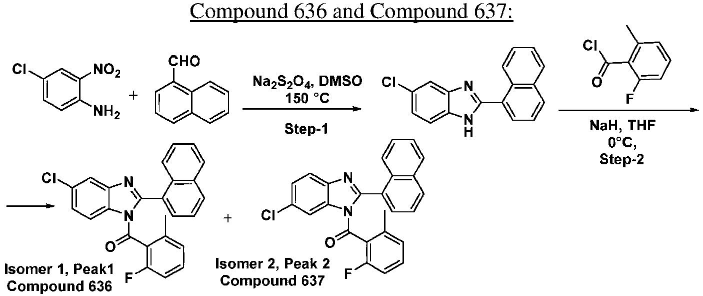

### Steps 1 and 2 were performed as described for synthesis of Compound 429

Compound 636: LCMS- 415.2(M)⁺ UPLC @ 254 nm =99.88 %, @ 220 nm = 99.93%. ¹H NMR (400 MHz, DMSO-*d*₆) δ ppm 8.11 (*d, J*=8.77 Hz, 1 H) 8.03 (*d, J*=1.75 Hz, 1 H) 7.81 - 7.89 *(m,* 2 H) 7.73 *(br. s.,* 1 H) 7.65 (*d, J*=7.02 Hz, 1 H) 7.61 (*dd, J=*8.77*,* 2.19 Hz, 1 H) 7.50 - 7.56 *(m,* 2 H) 7.34 - 7.40 (*m,* 1 H) 6.90 (*br. s.,* 1 H) 6.61 (*br. s.,* 1 H), 6.39 (s, 1H), 2.04 (*br. s.,* 3 H)

Compound 637: LCMS- 415.2(M)⁺ UPLC @ 254 nm = 99.87%, @ 220 nm = 99.93%. ¹H NMR (400 MHz, DMSO-d6) δ 8.12 *(d, J =* 2.1 Hz, 1H), 7.95 *(d, J =* 8.5 Hz, 1H), 7.90-7.80 (*m,* 2H),7.74 (s, 1H), 7.68-7.56 (*m,* 2H), 7.56-7.47 (m, 2H), 7.36 (*t, J* = 7.7 Hz, 1H), 6.91 (q, *J* = 7.5 Hz, 1H), 6.61 (s, 1H), 6.40 (s, 1H), 2.14-1.91 (*m,* 3H).

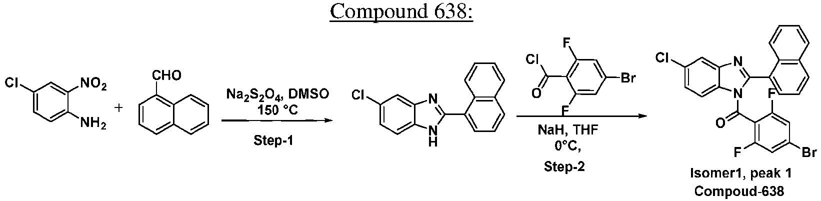

### Steps 1 and 2 were performed as described for synthesis of Compound 535

LCMS- 497.2 (M)⁺ UPLC @ 254 nm = 98.37%, @ 220 nm = 98.25 %. ¹H NMR (400 MHz, DMSO-d6) δ 8.16 (*d,* J = 8.7 Hz, 1H), 8.04 (*d,* J = 2.1 Hz, 1H), 7.90 (*dd,* J = 12.1,7.2 Hz, 2H), 7.72 (q, J = 4.8, 3.9 Hz, 1H), 7.67 (*d*, J = 7.1 Hz, 1H), 7.61 (*dd,* J = 8.7, 2.1 Hz, 1H), 7.56(dt, J = 6.3, 3.8 Hz, 2H), 7.44 (*t,* J = 7.7 Hz, 1H), 7.37 (*t,* J = 7.1 Hz, 1H), 7.16 (*dd,* J = 8.8, 5.3 Hz, 1H).

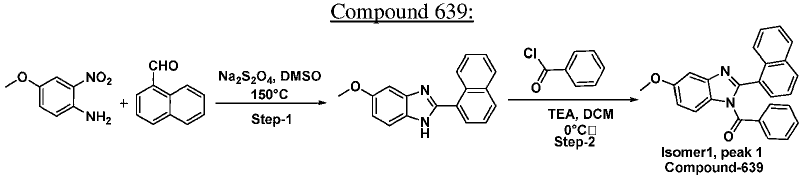

### Steps 1 and 2 were performed as described for synthesis of Compound 535

Compound 639: LCMS- 379.3 (M+H)⁺ UPLC @ 254 nm = 97.63%, @ 220 nm = 94.51 % ¹H NMR (400 MHz, DMSO-d6) δ 8.08-8.00 (*m,* 1H), 7.91-7.77 (*m,* 3H), 7.60-*7.49* (*m,* 3H), 7.47 (*d,J* = 7.5 Hz, 2H), 7.38 (*t, J* = 7.7 Hz, 1H), 7.32 (*t, J =* 7.5 Hz, 1H), 7.15-7.04 (*m*, 4H), 3.78 (s, 3H).

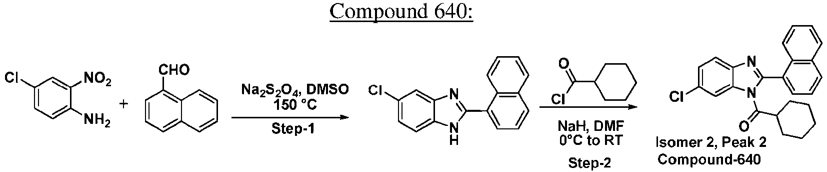

### Steps 1 and 2 were performed as described for synthesis of Compound 535

Compound 640: LCMS- 389.3 ((M)⁺ UPLC @ 254 nm = 98.16% and @ 220 nm 97.23%. ¹H NMR (400 MHz, DMSO-*d*₆) δ ppm 8.24 *(d, J*=8.33 Hz, 1 H) 8.10 - 8.18 (*m,* 2 H) 7.85 - 7.92 (*m,* 2 H) 7.72 (*t, J*=7.67 Hz, 1 H) 7.61 - 7.68 (*m,* 2 H) 7.51 - 7.60 (*m,* 2 H) 1.95 (*br. s.,* 1 H) 1.28 - 1.39 (*m,* 5 H) 1.21 - 1.28 (*m,* 3 H) 1.09 - 1.19 (*m,* 2 H). 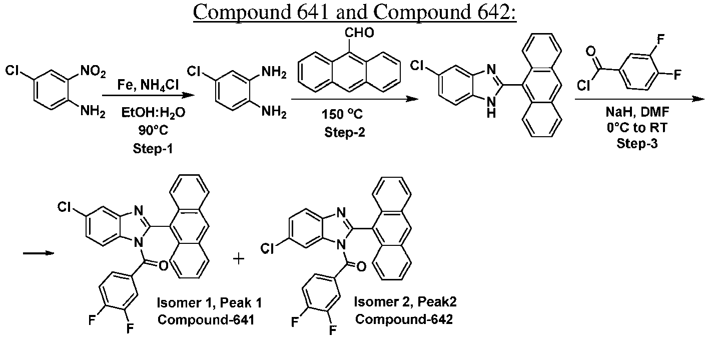
Step 1 was performed as described for step 1 of synthesis of Compound 588
Step 2 was performed as described for step 1 of synthesis of Compound 429
Step 3 was performed as described for step 2 of synthesis of Compound 535

Compound 641: LCMS- 469.2 (M)⁺ UPLC @ 254 nm = 98.90% and @ 220 nm 99.87% ¹H NMR (400 MHz, DMSO-*d*₆) δ ppm 8.63 (s, 1 H) 8.06 - 8.12 (*m,* 3 H) 7.91 (*d, J*=8.77 Hz, 1 H) 7.70 (*d*, *J*=7.89 Hz, 2 H) 7.61 (d*, J*=2.19 Hz, 1 H) 7.52 - 7.60 (*m,* 4 H) 7.19 - 7.25 *(m,* 1 H) 6.87 (*br. s.,* 1 H) 6.74 (*dd, J*=10.30, 8.11 Hz, 1 H

Compound 642: LCMS- 469.2 (M)⁺ UPLC @ 254 nm = 93.75% and @ 220 nm = 93.35%. ¹H NMR (400 MHz, DMSO-*d*₆) δ ppm 8.62 (s, 1 H) 8.08 (*d, J*=7.02 Hz, 2 H) 7.97 *-* 8.05 (*m,* 2 H) 7.69 (*d, J*=8.77 Hz, 2 H) 7.62 (*d, J*=8.33 Hz, 1 H) 7.52 - 7.60 (*m,* 3 H) 7.14 (*br. s.,* 1 H) 6.79 (*br. s.,* 1 H) 6.65 (s, 1 H) 6.68 (s, 1 H). 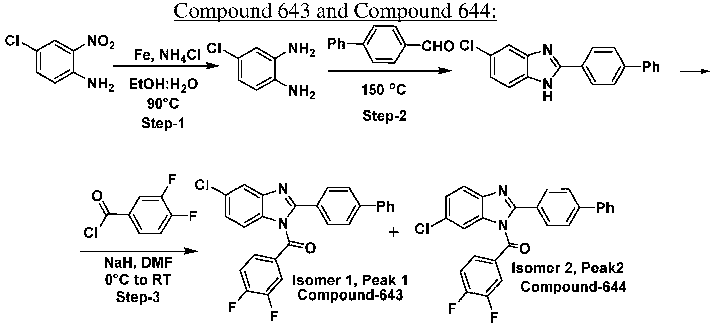
Step 1 was performed as described for step 1 of synthesis of Compound 588;
Step 2 was performed as described for step 1 of synthesis of Compound 429
Step 3 was performed as described for step 2 of synthesis of Compound 535

Compound 643: LCMS- 445.2 (M)⁺ UPLC @ 254 nm = 97.02% and @ 220 nm 97.20% ¹H NMR (400 MHz, DMSO-*d*₆) δ ppm 7.97 (*d, J*=2.19 Hz, 1 H) 7.70 (*br. s.,* 2 H) 7.68 (*br. s.,* 1 H) 7.61 - 7.67 (*m,* 4 H) 7.53 (s, 1 H) 7.50 (*d, J*=8.33 Hz, 3 H) 7.47 (s, 1 H) 7.40 - 7.45 *(m,* 2 H)

Compound 644: LCMS:- 445.2 (M)⁺ UPLC @ 254 nm = 96.82% and @ 220 nm = 93.46%. ¹H NMR (400 MHz, DMSO-*d*₆) δ 7.90 (t, J = 8.9 Hz, 2H), 7.70-7.58 (m, 7H), 7.54-7.44 (m, 4H), 7.40 (t, J = 7.4 Hz, 2H).

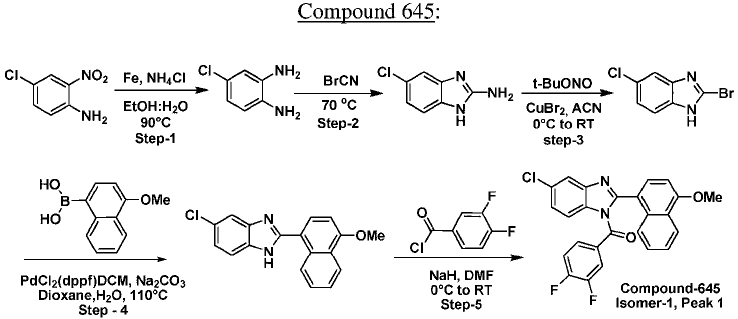

### Steps 1-5 were performed as described for synthesis of Compound 588

Compound 645: LCMS- 449.3 (M)⁺ UPLC @ 254 nm = 98.85% and @ 220 nm 97.92%. ¹H NMR (400 MHz, DMSO-*d*₆) δ ppm 8.11 (*d, J*=8.33 Hz, 1 H), 8.00 (*d, J=*1.75 Hz, 1 H), 7.91 (*d,* J=7.89 Hz, 1 H), 7.79 (d, *J*=8.77 Hz, 1 H), 7.47 - 7.61 (*m,* 5 H), 7.28 (*br. s.,* 1 H), 7.09 (*d, J*=10.09 Hz, 1 H), 6.89 (*d, J*=8.33 Hz, 1 H), 3.95 (s, 3 H).

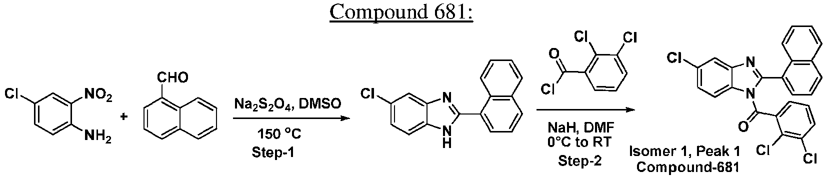

### Steps 1 and 2 were performed as described for synthesis of Compound 535

Compound 681: LCMS- 450.1 (M)⁺ UPLC @ 254 nm = 95.09% and @ 220 nm 95.92%. ¹H NMR (400 MHz, DMSO-*d*₆) δ ppm 8.18 (*d, J*=8.33 Hz, 1 H) 8.04 (*d, J=*1.75 Hz, 1 H) 7.82 - 7.88 (*m,* 2 H) 7.73 (*br. s.,* 1 H) 7.61 - 7.67 (*m,* 2 H) 7.50 - 7.56 (*m,* 2 H) 7.34 - 7.40 (*m,* 2 H) 7.19 (*d, J*=7.89 Hz, 2 H).

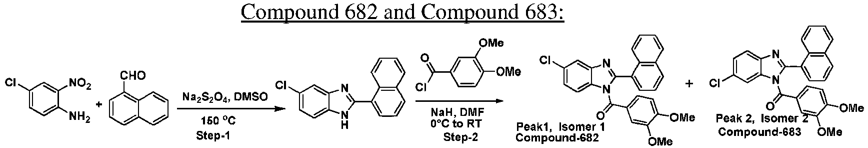

### Steps 1 and 2 were performed as described for synthesis of Compound 535

Compound 682: LCMS- 443.2 (M)⁺ UPLC @ 254 nm = 97.75% and @ 220 nm 98.77% ¹H NMR (400 MHz, DMSO-*d*₆) δ ppm 8.15 (*d, J*=3.07 Hz, 1 H) 8.01 (*d, J=*1.75 Hz, 1 H) 7.92 - 7.97 (*m,* 2 H) 7.61 (*d, J*=6.14 Hz, 1 H) 7.54 - 7.59 (*m,* 2 H) 7.50 - 7.54 (*m,* 1 H) 7.41 - 7.47 (*m,* 2 H) 7.31 (*dd,* J=8.33, 1.75 Hz, 1 H) 7.06 (*d,* J=2.19 Hz, 1 H) 6.77 (*d, J*=8.33 Hz, 1 H) 3.7 (s, 3H) 3.5 (s, 3H)

Compound 683: LCMS- 443.2 (M)⁺ UPLC @ 254 nm = 99.14% and @ 220 nm = 99.91%. ¹H NMR (400 MHz, DMSO-*d*₆) δ ppm 8.16 (*d, J*=9.65 Hz, 1 H) 7.88 - 7.96 (*m, 3* H) 7.54 - 7.61 (*m,* 3 H) 7.50 (*dd, J*=8.77, 2.19 Hz, 1 H) 7.39 - 7.45 (*m,* 2 H) 7.28 (*dd, J*=8.55, 1.97 Hz, 1 H) 7.02 (*d, J*=1.75 Hz, 1 H) 6.74 (*d, J*=8.33 Hz, 1 H) 3.7 (s, 3H) 3.45 (s, 3H). 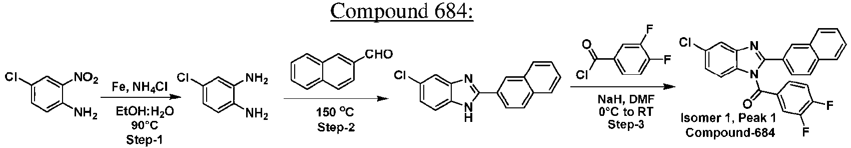
Step 1 was performed as described for synthesis of Compound 588
Step 2 was performed as described for synthesis of Compound 579

Compound 684: LCMS- 419.2 (M)⁺ UPLC @ 254 nm = 98.39% and @ 220 nm 97.40%. ¹H NMR (400 MHz, DMSO-*d*₆) δ ppm 8.17 (s, 1 H) 7.85 - 8.01 (*m,* 4H) 7.68 (*dd, J*=8.55, 1.53 Hz, 2 H) 7.51 - 7.62 (*m,* 3 H) 7.46 (*dd, J*=8.77, 2.19 Hz, 1 H) 7.35 (s, 1 H) 7.37 (s, 1 H).

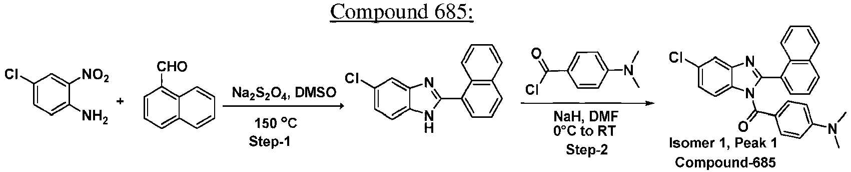

### Steps 1 and 2 were performed as described for synthesis of Compound 535

Compound 685: LCMS- 426.2 (M)⁺ UPLC @ 254 nm = 94.85% and @ 220 nm 91.06%. ¹H NMR (400 MHz, DMSO-*d*₆) δ ppm 8.19 (*d*, *J*=7.02 Hz, 1 H) 7.94 - 8.02 (*m,* 2 H) 7.92 (*d, J*=8.77 Hz, 1 H) 7.42 - 7.68 (*m,* 7 H) 7.29 (*d, J*=1.75 Hz, 1 H) 6.57 (*d, J=*9.21 Hz, 2 H) 2.98 (s, 6H).

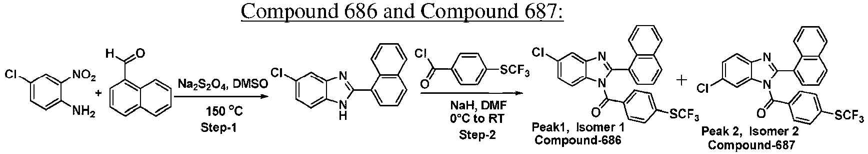

Steps 1 and 2 were performed as described for synthesis of Compound 535

Compound 686: LCMS- 483.2 (M)⁺ UPLC @ 254 nm = 99.24% and @ 220 nm 99.0%. ¹H NMR (400 MHz, DMSO-*d*₆) δ ppm 8.02 - 8.05 (*m,* 1 H) 7.90 (*d, J*=8.77 Hz, 2 H) 7.77 - 7.84 (*m,* 2 H) 7.63 (*d, J*=6.58 Hz, 1 H) 7.50 - 7.59 (*m,* 3 H) 7.48 (*m, J*=7.89 Hz, 2 H) 7.34 (*t, J*=7.67 Hz, 1 H) 7.21 (*m, J*=7.89 Hz, 2 H).

Compound 687: LCMS- 483.2 (M)⁺ UPLC @ 254 nm = 98.55% and @ 220 nm = 97.73%. ¹H NMR (400 MHz, DMSO-*d*₆) δ ppm 7.95 (*d, J*=8.33 Hz, 1 H) 7.87 - 7.93 (*m, 2* H) 7.79 (*d, J*=8.33 Hz, 1 H) 7.82 (*d, J*=7.89 Hz, 1 H) 7.62 (*d, J*=7.02 Hz, 1 H) 7.50 - 7.60 (*m,* 3 H) 7.47 (*m, J*=7.45 Hz, 2 H) 7.33 (*t, J*=7.67 Hz, 1 H) 7.20 (*m, J*=7.45 Hz, 2 H).

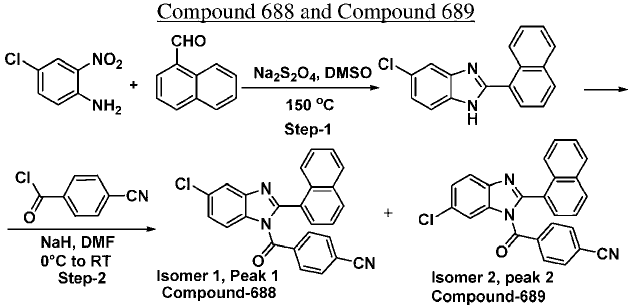

### Steps 1 and 2 were performed as described for synthesis of Compound 535

Compound 688: LCMS- 408.1 (M)⁺ UPLC @ 254 nm = 96.49% and @ 220 nm 92.76%. ¹H NMR (400 MHz, DMSO-*d*₆) δ ppm 8.04 (*d, J*=1.75 Hz, 1 H) 7.87 (*d, J*=8.77 Hz, 4 H) 7.65 (*d, J*=7.02 Hz, 1 H) 7.50 - 7.59 (*m,* 5 H) 7.39 (*d, J*=7.02 Hz, 3 H)

Compound 689: LCMS- 408.1 (M)⁺ UPLC @ 254 nm = 98.97% and @ 220 nm = 97.20%. ¹H NMR (400 MHz, DMSO-*d*₆) δ ppm 7.92 - 7.98 (*m,* 2 H) 7.86 (*d, J*=8.33 Hz, 3 H) 7.63 (*d, J*=6.58 Hz, 1 H) 7.53 - 7.59 (*m,* 3 H) 7.50 (*d, J*=7.89 Hz, 2 H) 7.32 - 7.40 (*m, 3* H). 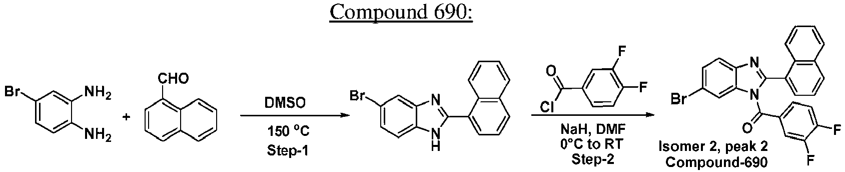
Step 1 was performed as described above for step 1 of synthesis of Compound 579
Step 2 was performed as described above for step 2 of synthesis of Compound 535

Compound 690: LCMS- 463.2 (M)⁺ UPLC @ 254 nm = 98.22% and @ 220 nm 96.19%. ¹H NMR (400 MHz, DMSO-*d*₆) δ ppm 8.04 (*d, J*=1.75 Hz, 1 H) 7.87 - 7.94 (*m,* 4 H) 7.68 (*dd, J*=8.55, 1.97 Hz, 1 H) 7.63 *(d, J*=6.58 Hz, 1 H) 7.52 - 7.58 (*m,* 3 H) 7.38 - 7.43 (*m,* 1 H) 7.28 (*br. s.,* 1 H) 7.04 (*d, J*=10.09 Hz, 1 H). 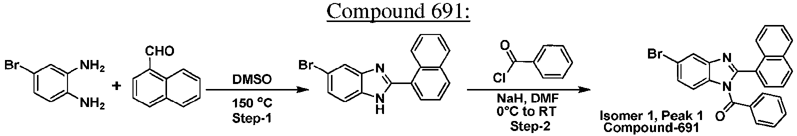
Step 1 was performed as described above for step 1 of synthesis of Compound 579
Step 2 was performed as described above for step 2 of synthesis of Compound 535

Compound 691: LCMS- 427.1 (M)⁺ UPLC @ 254 nm = 99.38% and @ 220 nm 98.53%. ¹H NMR (400 MHz, DMSO-*d*₆) δ ppm 8.16 (*d, J*=1.32 Hz, 1 H) 8.03 (*d, J=*9.21 Hz, 1 H) 7.85 - 7.92 (*m,* 2 H) 7.49 - 7.64 (*m,* 7 H) 7.33 - 7.43 (*m,* 2 H) 7.10 (*t, J*=7.89 Hz, 2H). 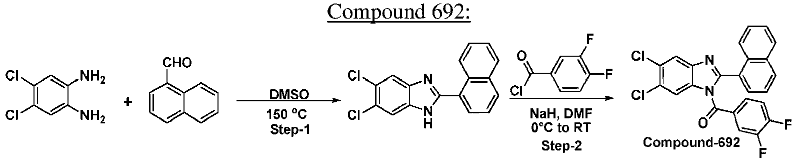
Step 1 was performed as described above for step 1 of synthesis of Compound 579
Step 2 was performed as described above for step 2 of synthesis of Compound 535

Compound 692: LCMS- 453.2 (M)⁺ UPLC @ 254 nm = 95.86% and @ 220 nm 88.79%. ¹H NMR (400 MHz, DMSO-*d*₆) δ ppm 8.29 (s, 1 H) 8.08 (s, 1 H) 7.91 (*d, J=*7.89 Hz, 3 H) 7.64 (*d, J*=7.45 Hz, 1 H) 7.51 - 7.59 (*m,* 3 H) 7.41 (*t, J*=7.67 Hz, 1 H) 7.29 (*br. s.,* 1 H) 7.05 (*d, J*=10.09 Hz, 1 H).\ 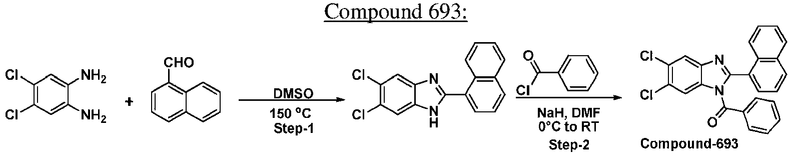
Step 1 was performed as described above for step 1 of synthesis of Compound 579
Step 2 was performed as described above for step 2 of synthesis of Compound 535

Compound 693: LCMS- 417.1 (M)⁺ UPLC @ 254 nm = 97.48% and @ 220 nm 69.25%. ¹H NMR (400 MHz, DMSO-*d*₆) δ ppm 8.28 (s, 1 H) 8.03 (*d, J*=8.33 Hz, 1 H) 7.86 - 7.91 (*m,* 2 H) 7.84 (s, 1 H) 7.62 (*d, J*=7.45 Hz, 1 H) 7.56 (*t, J*=5.92 Hz, 2 H) 7.48 - 7.53 (*m,* 2 H) 7.30 - 7.41 (*m,* 2 H) 7.07 (*t, J*=7.67 Hz, 2 H).

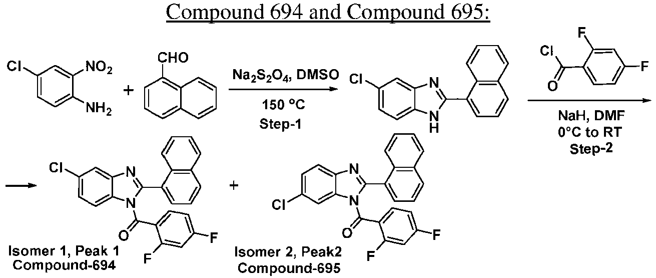

Steps 1 and 2 were performed as described for synthesis of Compound 535

Compound 694: LCMS- 419.1 (M)⁺ UPLC @ 254 nm = 99.70% and @ 220 nm 98.99% ¹H NMR (400 MHz, DMSO-*d*₆) δ ppm 8.00 - 8.05 (*m,* 2 H) 7.90 (*d, J*=8.33 Hz, 2 H) 7.78 - 7.82 (*m,* 1 H) 7.66 (*d, J*=7.02 Hz, 1 H) 7.53 - 7.61 (*m,* 3 H) 7.38 - 7.45 (*m,* 2 H) *6.79* (*t, J*=9.65 Hz, 1 H) 6.59 (*t, J*=7.67 Hz, 1 H)

Compound 695: LCMS- 419.1 (M)⁺ UPLC @ 254 nm = 99.29% and @ 220 nm = 98.48%. ¹H NMR (400 MHz, DMSO-*d*₆) δ ppm 8.07 (s, 1 H) 7.95 (*d, J*=8.33 Hz, 1 H) 7.90 (*d, J*=8.33 Hz, 2 H) 7.79 (*br. s.,* 1 H) 7.65 (*d, J*=6.58 Hz, 1 H) 7.54 - 7.61 (*m,* 3 H) 7.36 - 7.44 (*m,* 2 H) 6.78 (*d, J*=10.09 Hz, 1 H) 6.56 (*br. s.,* 1 H).

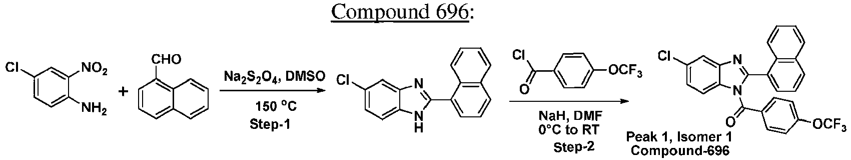

### Steps 1 and 2 were performed as described for synthesis of Compound 535

Compound 696: LCMS- 467.2 (M)⁺ UPLC @ 254 nm =99.22 %, @ 220 nm = 98.29 %. ¹H NMR (400 MHz, DMSO-*d*6) δ 8.03 (*d, J =* 2.1 Hz, 1H), 7.86 (*dd, J =* 15.8, 8.9, 2.3 Hz, 4H), 7.63(*d*, *J* = 7.1 Hz, 1H), 7.54 (*dd, J =* 8.5, 6.3, 3.7 Hz, 5H), 7.37 (*t, J =* 7.7 Hz, 1H), 6.89 (*d, J =* 8.3 Hz, 2H).

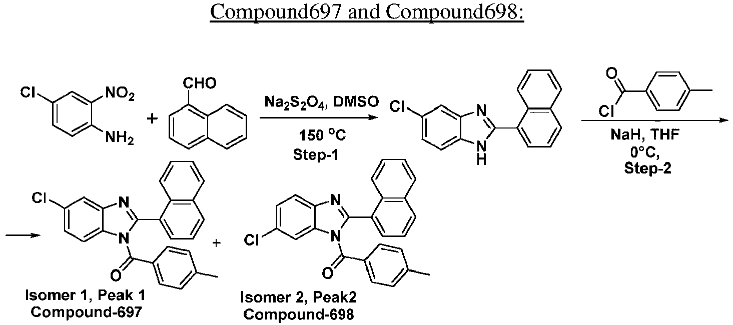

### Steps 1 and 2 were performed as described for synthesis of Compound 535

Compound 697: LCMS-397.2 (M)⁺ UPLC @ 254 nm =98.40%, @ 220 nm = 98.56 %. ¹H NMR- 1H NMR (400 MHz, DMSO-*d*6) δ 8.09-8.03 (*m*, 1H), 8.02 (s, 1H), 7.97-7.90 (*m,* 2H), 7.62 *(d, J =* 7.1Hz, 1H), 7.56 (*dt, J =* 6.4, 3.5 Hz, 2H), 7.50 (*d, J =* 7.9 Hz, 2H), 7.45 (*d, J =* 1.9 Hz, 3H), 7.01 (*d, J* = 7.9Hz, 2H), 2.21 (s, 3H).

Compound 698: LCMS- 397.2 (M)⁺ UPLC @ 254 nm =98.05 %, @ 220 nm = 98.76 %. ¹H NMR- 1H NMR (400 MHz, DMSO-*d*6) δ 8.08-8.01 (*m,* 1H), 7.97-7.88 (*m,* 3H), 7.61 (*d, J* = 6.9 Hz, 1H),7.58-7.48 (*m,* 4H), 7.45 (*dd, J =* 12.0, 7.8 Hz, 3H), 6.98 (*d, J* = 7.9 Hz, 2H), 2.20 (s, 3H).

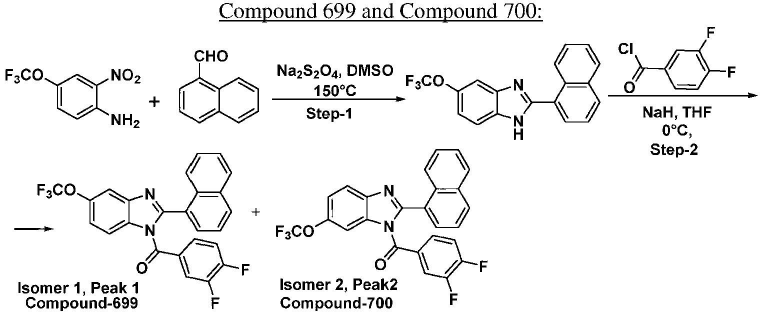

### Steps 1 and 2 were performed as described for synthesis of Compound 535

Compound 699: LCMS- 469.1 (M+H)⁺ UPLC @ 254 nm = 97.91%, @ 220 nm = 94.54%. ¹H NMR (400 MHz, DMSO-d6) δ 8.01-7.87 (*m,* 5H), 7.75-7.53 (*m,* 4H), 7.53-7.46 (*m,* 1H), 7.42 (*t, J* = 7.7 Hz, 1H), 7.34 (*d, J* = 4.8 Hz, 1H), 7.09 (q, *J* = 8.7 Hz, 1H).

Compound 700: LCMS- 469.2 (M+H)⁺ UPLC @ 254 nm = 98.64%, @ 220 nm = 97.28%. ¹H NMR (400 MHz, DMSO-d6) δ 8.04 (*d,* J = 8.7 Hz, 1H), 7.91 (dt, J = 8.6, 4.9 Hz, 3H), 7.83 (s, 1H),7.64 (*d,* J = 7.0 Hz, 1H), 7.60-7.45 (*m,* 4H), 7.42 (q, J = 7.5 Hz, 1H), 7.30 (*t,* J = 5.8 Hz, 1H), 7.05 (p, J= 10.4, 9.0 Hz, 1H).

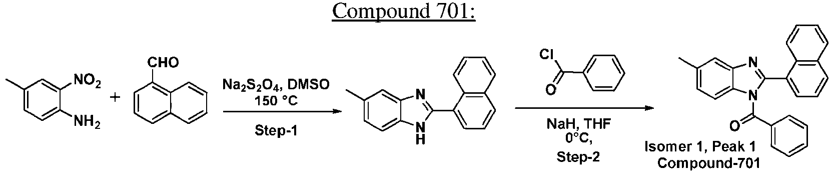

### Steps 1 and 2 were performed as described for synthesis of Compound 535

Compound 701: LCMS- 363.2 (M+H)⁺ UPLC @ 254 = 94.72 %, @ 220 = 85.92 % ¹H NMR (400 MHz, DMSO-*d*₆) δ 8.07-7.98 (*m,* 1H), 7.94 (*d, J =* 7.6 Hz, 1H), 7.92-7.68 (*m,* 3H), 7.66-7.21 (*m,* 9H), 7.16-7.04 (*m,* 1H), 2.45 (s, 3H).

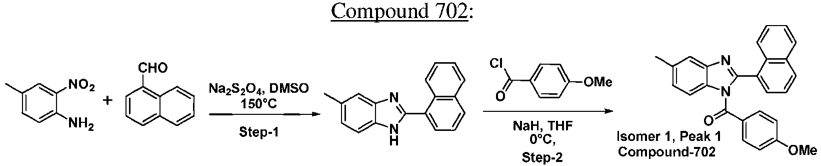

### Steps 1 and 2 were performed as described for synthesis of Compound 535

Compound 702: LCMS-393.2 (M+H)⁺ UPLC @ 254 nm =98.33 % @220 nm = 98.36 % ¹H NMR (400 MHz, DMSO-*d*₆) δ 8.12-8.02 (m, 1H), 7.93 (t, J = 8.7 Hz, 2H), *7.57* (*ddd,* J = 18.0, 7.1, 4.0 Hz, 5H), 7.45 (q, J = 8.0 Hz, 2H), 7.24 (*dd,* J = 25.8, 9.5 Hz, 2H), 6.74 (*dd,* J = 16.3, 8.7 Hz, 2H), 3.71 (*d,* J = 8.1 Hz, 3H), 2.43 (s, 3H).

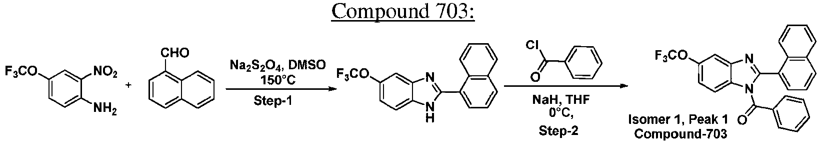

### Steps 1 and 2 were performed as described for synthesis of Compound 535

Compound 703: LCMS- 433.2 (M+H)⁺ UPLC @ 254 nm =97.55 %, @ 220 nm = 92.97%. ¹H NMR (400 MHz, DMSO-*d*₆) δ ppm 8.03 - 8.07 (*m,* 1 H) 7.97 (s, 1 H) 7.86 - 7.91 (*m,* 2 H) 7.69 (*d, J*=8.77 Hz, 1 H) 7.62 (*d, J*=6.14 Hz, 1 H), 7.51 - 7.58 (*m,* 4 H) 7.47 (*d, J*=8.77 Hz, 1 H) 7.41 (*d, J*=7.89 Hz, 1 H) 7.33 - 7.36 (*m,* 1 H) 7.10 (*t, J*=7.67 Hz, 2 H).

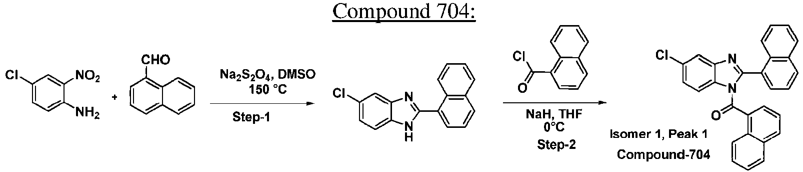

### Steps 1 and 2 were performed as described for synthesis of Compound 535

Compound 704: LCMS - 432.1 (M)⁺ UPLC @ 254 nm = 99.68%, @ 220 nm = 99.96% ¹H NMR (400 MHz, DMSO-*d*6) δ 8.04 (s, 1H), 7.92 (q, *J* = 8.9 Hz, 3H), 7.79-7.62 (*m,* 3H), 7.50 (tt, *J =* 22.1, 6.9 Hz, 8H), 6.91 (*t, J* = 7.6 Hz, 1H), 6.84 (*t, J* = 7.9 Hz, 1H).

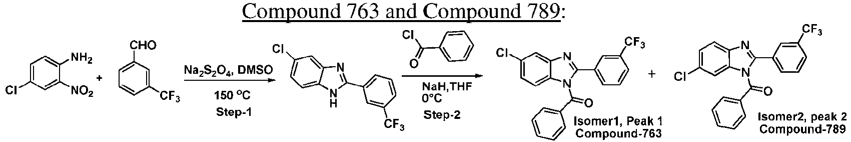

### Steps 1 and 2 were performed as described for synthesis of Compound 535

Compound 763: LCMS- 401.1(M)⁺ UPLC @ 254 nm = 98.75%, @ 220 nm = 99.54%. ¹H NMR (400 MHz, DMSO-d6) δ 8.02-7.96 (*m,* 1H), 7.89 (*d, J =* 11.1 Hz, 2H), 7.72 (*t, J =* 6.7 Hz, 3H), 7.62-7.42 (*m,* 4H), 7.37 (*t, J* = 7.7 Hz, 2H).

Compound 789: LCMS- 401.3 UPLC (M)⁺ @ 254 nm = 93.56%, @ 220 nm = 97.28%. ¹H NMR (400 MHz, DMSO-*d*₆) δ ppm 7.94-7.84 (*m,* 3H), 7.70 (*t, J* = 7.3 Hz, 3H), 7.53 (d*dd, J =* 21.8, 7.2, 2.9 Hz, 4H), 7.36 (t*, J =* 7.7 Hz, 2H).

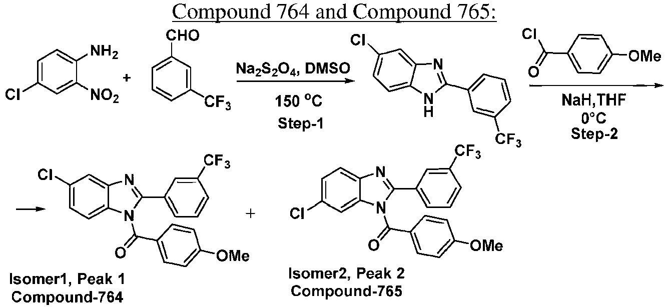

### Steps 1 and 2 were performed as described for synthesis of Compound 535

Compound 764: LCMS-431.2 (M)⁺ UPLC @ 254 nm = 99.06%, @ 220 nm = 98.75%. ¹H NMR (400 MHz, DMSO-*d*₆) δ ppm 8.00-7.91 *(m,* 2H), 7.89 (*d, J* = 7.8 Hz, 1H), 7.78 (*d, J* = 8.3 Hz, 3H), 7.62 (*t,* J= 7.8 Hz, 1H), 7.41 (*dd, J =* 8.8, 2.1 Hz, 1H), 7.36 (*d, J =* 8.8 Hz, 1H), 6.98 (*d, J =* 8.4 Hz, 2H), 3.81 (s, 3H).

Compound 765: LCMS- 431.4 UPLC (M)⁺ @ 254 nm = 93.56%, @ 220 nm = 97.56%. ¹H NMR (400 MHz, DMSO-*d*₆) δ ppm 7.94-7.84 (*m,* 3H), 7.80-7.72 (*m,* 3H), 7.60 (*t, J* = 7.8 Hz, 1H), 7.47(*dd, J* = 8.4, 2.2 Hz, 1H), 7.42 (*d, J* = 2.1 Hz, 1H), 6.96 (*d, J =* 8.5 Hz, 2H), 3.81 (s, 3H).

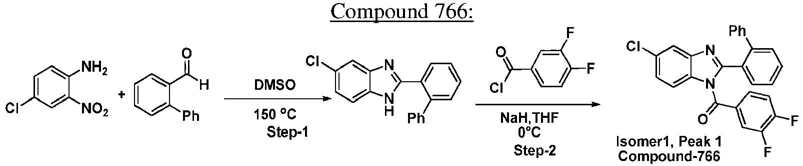

### Steps 1 and 2 were performed as described for synthesis of Compound 535

Compound 766: LCMS- 445.2 (M)⁺ UPLC @ 254 nm = 95.7%, @ 220 nm = 93.29%. ¹H NMR (400 MHz, DMSO-*d*₆) δ ppm δ 7.97 (s, 1H), 7.82-7.73 (*m,* 1H), 7.59-*7.47* (*m,* 2H), 7.47-7.08 (*m,* 9H), 6.92 (*d, J* = 7.0 Hz, 2H).

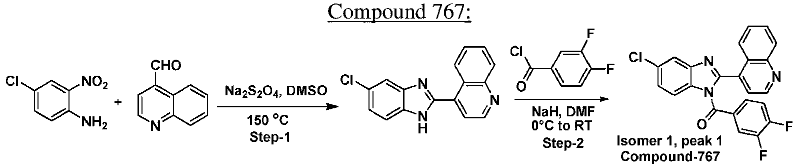

### Steps 1 and 2 were performed as described for synthesis of Compound 535

Compound 767: LCMS: 420.2 (M)⁺ UPLC @ 254 nm = 97.49% and @ 220 nm 95.27%. ¹H NMR (400 MHz, DMSO-*d*₆) δ ppm 8.84 (*d, J*=3.95 Hz, 1 H) 8.02 - 8.10 (*m, 3* H) 7.77 - 7.85 (*m,* 2 H) 7.64 - 7.75 (*m,* 3 H) 7.55 (*dd, J*=8.99, 1.97 Hz, 1 H) 7.50 (*br. s.,* 1 H) 7.19 - 7.28 (*m*, 1 H). 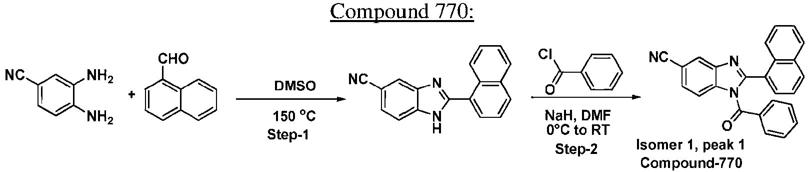
Step 1 was performed as described for step 1 of synthesis of Compound 579
Step 2 was performed as described for step 2 of synthesis of Compound 535

Compound 770: LCMS: 374.3 (M+H)⁺ UPLC @ 254 nm = 99.53% and @ 220 nm 98.26%. ¹H NMR (400 MHz, DMSO-*d*₆) δ ppm 8.53 (*d, J*=0.88 Hz, 1 H) 8.06 (*d, J=*9.21 Hz, 1 H) 7.90 (*d, J*=7.89 Hz, 2 H) 7.83 - 7.88 (*m,* 1 H) 7.72 (*d, J*=8.33 Hz, 1 H) 7.65 (*d, J*=6.14 Hz, 1 H) 7.54 - 7.60 (*m,* 4 H) 7.34 - 7.45 *(m,* 2 H) 7.12 (*t, J=*7.89 Hz, 2 H).

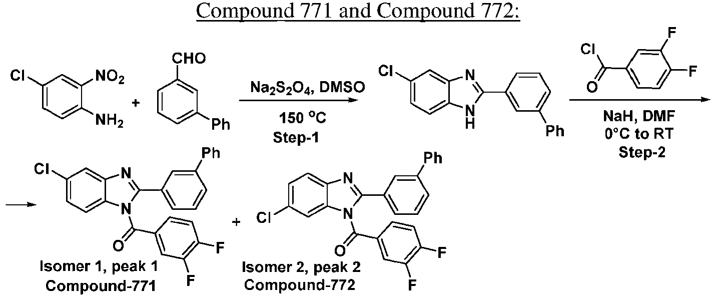

Steps 1 and 2 were performed as described for synthesis of Compound 535

Compound 771: LCMS- 445.3 (M)⁺ UPLC @ 254 nm = 98.17% and @ 220 nm 99.25% ¹H NMR (400 MHz, DMSO-*d*₆) δ ppm 7.98 (*d*, *J*=2.19 Hz, 1 H) 7.89 (*br. s.,* 1 H) 7.79 (s, 1 H) 7.62 - 7.70 (*m,* 3 H) 7.53 - 7.62 (*m,* 3 H) 7.36 - 7.52 (*m,* 6 H)

Compound 772: LCMS- 445.3 (M)⁺ UPLC @ 254 nm = 99.15% and @ 220 nm = 99.56%. ¹H NMR (400 MHz, DMSO-*d*₆) δ ppm 7.90 (*d, J*=8.33 Hz, 1 H) 7.84 (*d, J=*7.89 Hz, 1 H) 7.77 (s, 2 H) 7.56 - 7.68 (m, 4 H) 7.44 - 7.55 (*m,* 4 H) 7.31 - 7.44 (*m,* 3 H)

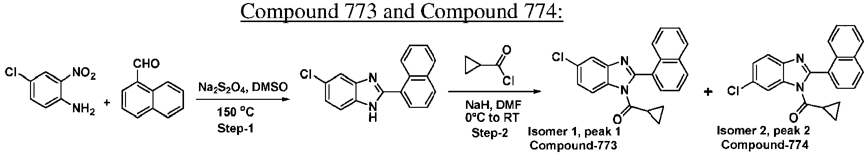

Steps 1 and 2 were performed as described for synthesis of Compound 535

Compound 773: LCMS- 347.1 (M)⁺UPLC @ 220 nm 94.37%. ¹H NMR (400 MHz, DMSO- *d*₆) δ 8.17 (d, J = 8.3 Hz, 1H), 8.10 (d, J = 3.8 Hz, 1H), 8.07 (d, J = 3.2 Hz, 1H), 7.95 (s, 1H), 7.87 (d, J = 7.1 Hz, 1H), 7.82 (d, J = 8.2 Hz, 1H), 7.74-7.64 (m, 1H), 7.65-7.54 (m, 2H), 7.52 (dd, J = 8.7, 2.1 Hz, 1H), 1.47 (dtt, J = 12.3, 8.2, 4.8 Hz, 1H), 1.23 (d, J = 4.0 Hz, 1H), 0.88 (h, J = 5.0, 4.5 Hz, 1H), 0.84-0.72 (m, 1H), 0.51 (dq, J = 8.0, 4.2 Hz, 1H).

Compound 774: LCMS- 347.2 (M)⁺ UPLC @ 220 nm = 94.92%. ¹H NMR (400 MHz, DMSO- *d*₆) δ 8.17 (d, J = 8.3 Hz, 1H), 8.10 (t, J = 2.2 Hz, 1H), 8.07 (s, 1H), 7.87 (dd, J = 8.0, 3.4 Hz, 1H), 7.83 (d, J = 8.3 Hz, 1H), 7.69 (t, J = 7.7 Hz, 1H), 7.63 (t, J = 7.4 Hz, 1H), 7.60-7.54 (m, 2H), 7.52 (dd, J = 8.5, 2.2 Hz, 1H), 1.43 (tt, J = 8.0, 4.5 Hz, 1H), 1.23 (d, J = 4.0 Hz, 1H), 0.88 (h, J = 5.5, 4.9 Hz, 2H), 0.49 (dq, J = 7.8, 4.1 Hz, 1H).

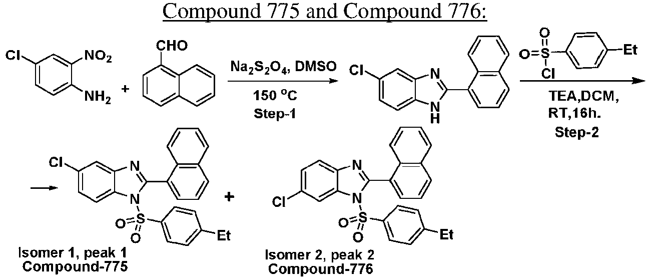

Steps 1 and 2 were performed as described for synthesis of Compound 535

Compound 775: LCMS-447.3 (M)⁺ UPLC @ 254 nm = 96.10%, @ 220 nm = 99.05%. ¹H NMR (400 MHz, DMSO-*d*₆) δ ppm 8.19 (*d, J*=8.77 Hz, 2 H), 8.03 (*d, J*=8.33 Hz, 1 H), 7.96 (s, 1 H), 7.58 - 7.71 (*m,* 3 H), 7.50 - 7.55 (*m,* 1 H), 7.25 - 7.36 (*m,* 3 H), 7.17 (*d, J*=7.89 Hz, 3 H), 2.52 - 2.59 (*m,* 2 H), 1.07 (*t, J*=7.67 Hz, 3 H).

Compound 776: LCMS- 447.3 (M)⁺ UPLC @ 254 nm = 93.64%, @ 220 nm = 98.20%. ¹H NMR (400 MHz, DMSO-*d*₆) δ ppm 8.12 - 8.23 (*m,* 2 H), 8.02 (*d, J*=7.89 Hz, 1 H), 7.88 (*d, J*=8.77 Hz, 1 H), 7.49 - 7.69 *(m,* 4 H), 7.30 (*d, J*=8.33 Hz, 3 H), 7.07 - 7.19 (*m,* 3 H), 2.53 - 2.59 (*m,* 2 H), 1.07 (*t, J*=7.45 Hz, 3 H).

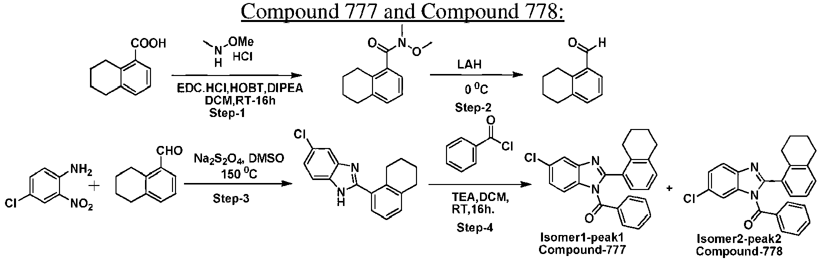

Step 1: To a stirred solution of 5,6,7,8-tetrahydronaphthalene-1-carboxylic acid (1 g , 5.675mmol) in DCM (15mL) was added N,O-dimethylhydroxylamine hydrochloride (608 mg, 6.242 mmol,) followed by the addition of EDC.HCl (2.2g ,11.35mmol), HOBT(1.53g, 11.35mmol) followed by addition of DIPEA (5mL, 28.37mmol, 5.0eq) under nitrogen atmosphere and the reaction mixture was stirred at rt for 16h. After 16h reaction was monitored by TLC and LCMS. After completion of reaction, the reaction mixture was diluted with DCM (200 mL) and washed with water (2×100 mL). The organic layer was washed with brine solution, separated and dried over sodium sulphate, concentrated under vacuo to yield crude product which was purified by flash chromatography (elution 0-20% EtOAc in hexane) to afford N-methoxy-N-methyl-5, 6, 7, 8- tetrahydronaphthalene-1-carboxamide (800mg) as a yellow solid.

Step 2: N-methoxy-N-methyl-5, 6, 7, 8-tetrahydronaphthalene-1-carboxamide (800 mg, 3.6 mmol) was dissolved in dry THF (12mL) under nitrogen atmosphere. To the mixture was added (1.67mL, 4.017mmol) a solution of LAH in THF(2.5M) dropwise. The reaction was quenched after half an hour after by addition of 0.5M potassium hydrogen sulfate aqueous solution. The mixture was extracted with ethyl acetate and the ethyl acetate layer was washed three times with brine and dried over anhydrous sodium sulfate, concentrate under reduced pressure to yield crude product which was purified by flash chromatography (elution 0-20% EtOAc in hexane) to afford 5, 6, 7, 8-tetrahydronaphthalene-1-carbaldehyde (438 mg) as a yellow oil.

### Steps 3 and 4 were performed as described for Compound 490

Compound 777: LCMS - 387.1 (M)⁺ UPLC @ 254 nm = 99.61%, @ 220 nm = 99.86%. ¹H NMR (400 MHz, DMSO-*d*₆) δ ppm 7.92 (d, J=1.75 Hz, 1 H), 7.63 (d, J=7.02 Hz, 2 H), 7.45 - 7.58 (m, 2 H), 7.37 - 7.45 (m, 1 H), 7.25 - 7.37 (m, 2 H), 7.08 (d, J=7.02 Hz, 1 H), 6.90 - 7.01 (m, 2 H), 2.66 (d, J=15.35 Hz, 4 H), 1.67 (br. s., 4 H).

Compound 778: LCMS- 387.1 (M)⁺ UPLC @ 254 nm = 99.88%, @ 220 nm = 99.81%. ¹H NMR (400 MHz, DMSO-*d*₆) δ ppm 7.85 (*d, J*=8.77 Hz, 1 H), 7.62 (*d, J=*7.45 Hz, 2 H), 7.54 (*br. s.,* 1 H), 7.43 - 7.54 (*m,* 2 H), 7.26 - 7.37 (*m,* 2 H), 7.01 - 7.12 (*m,* 1 H), 6.87 - 7.01 (*m,* 2 H), 2.66 (*d, J*=18.86 Hz, 4 H), 1.66 (*br. s.,* 4 H).

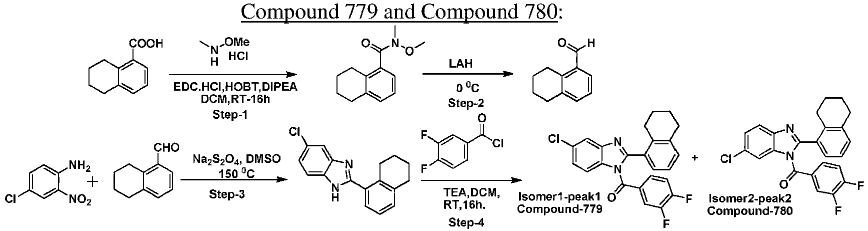

### Steps 1-4 were performed as described for Compound 777

Compound 779: LCMS-423.3 (M)⁺ UPLC @ 254 nm = 99.33%, @ 220 nm = 98.98%. ¹H NMR (400 MHz, DMSO-*d*₆) δ ppm 7.85 - 7.99 (m, 1 H), 7.73 - 7.84 (m, 2 H), 7.48 - 7.61 (m, 2 H), 7.41 - 7.48 (m, 1 H), 7.32 - 7.41 (m, 1 H), 7.08 (d, J=7.02 Hz, 1 H), 6.96 (t, J=7.45 Hz, 1 H), 2.65 (br. s., 4 H), 1.67 (br. s., 4 H).

Compound 780: LCMS- 423.3 (M)⁺ UPLC @ 254 nm = 98.57%, @ 220 nm = 98.17%. ¹H NMR (400 MHz, DMSO-*d*₆) δ 7.76 - 7.92 (m, 2 H), 7.66 - 7.76 (m, 1 H), 7.45 - 7.59 (m, 2 H), 7.32 - 7.43 (m, 1 H), 7.06 (d, ppm J=7.45 Hz, 1 H), 7.00 - 7.03 (m, 1 H), 6.87 - 6.98 (m, 1 H), 2.64 (br. s., 4 H), 1.66 (br. s., 4 H).

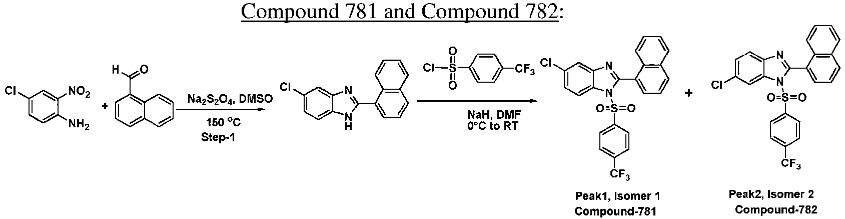

### Steps 1 and 2 were performed as described for Compound 631

Compound 781: LCMS-487.1 (M)⁺ UPLC @ 254 nm = 99.88%, @ 220 nm = 99.79%. ¹H NMR (400 MHz, DMSO-*d*₆) δ ppm: 8.19 (*d, J*=7.89 Hz, 1 H), 8.22 (*d, J=*8.77 Hz, 1 H), 7.92 - 8.06 (*m,* 2 H), 7.59 - 7.81 (*m,* 5 H), 7.43 - 7.59 (*m,* 3 H), 7.25 (*t*, *J*=7.67 Hz, 1 H), 7.08 (d, J=8.77 Hz, 1 H).

Compound 782: LCMS- 487.0 (M)⁺ UPLC @ 254 nm = 99.30%, @ 220 nm = 99.12%. ¹H NMR (400 MHz, DMSO-*d*₆) δ ppm : 8.19 - 8.29 *(m,* 1 H), 8.18 (s, 1 H), 7.99 (*d, J*=7.89 Hz, 1 H), 7.92 *(d,* J=8.33 Hz, 1 H), 7.66 - 7.72 *(m,* 2 H), 7.59 - 7.66 *(m,* 3 H), 7.56 *(d,* 2 H), 7.48 - 7.55 (s, 1 H), 7.39 - 7.48 (*m,* 1 H), 7.02 (*d, J*=8.33 Hz, 1 H).

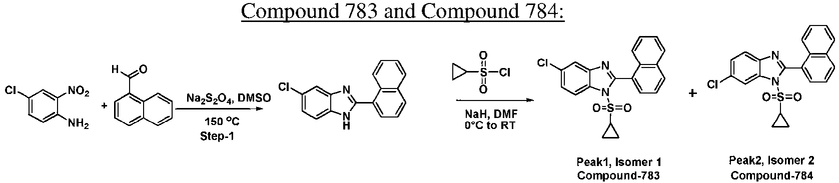

### Steps 1 and 2 were performed as described for Compound 631

Compound 783: LCMS- 383.1 (M)⁺ @ 254 nm = 98.83% ¹H NMR (400 MHz, DMSO-*d*₆) δ 8.16 (d, J = 8.3 Hz, 1H), 8.09-7.96 (m, 3H), 7.82 (d, J = 7.0 Hz, 1H), 7.69-7.48 (m, 5H), 3.07 (td, J = 7.6, 3.8 Hz, 1H), 0.99 (s, 4H)

Compound 784: LCMS- 383.2 (M)⁺ @254nm =98.40 ¹H NMR (400 MHz, DMSO-*d*₆) δ 8.16 (d, J = 8.2 Hz, 1H), 8.09-7.99 (m, 2H), 7.92 (d, J = 8.6 Hz, 1H),7.82 (d, J = 7.0 Hz, 1H), 7.65 (t, J = 7.7 Hz, 1H), 7.62-7.47 (m, 4H), 3.17 (td, J = 7.5, 3.8 Hz, 1H), 1.2-0.80 (m, 4H).

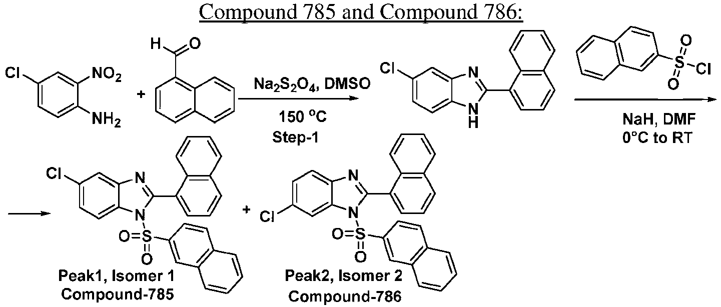

### Steps 1 and 2 were performed as described for Compound 631

Compound 785: LCMS- 469.1 (M)⁺ @254 nm = 97.97%. ¹H NMR (400 MHz, DMSO-d6) δ 8.27 (*d, J* = 8.8 Hz, 1H), 8.17 (*dd, J* = 7.8, 1.9 Hz, 1H), 8.01 (*d, J* =2.2 Hz, 1H), 7.93 (d*dd, J* = 11.0, 6.7, 2.2 Hz, 4H), 7.82 (*d, J* = 8.2 Hz, 1H), 7.78-7.55 *(m,* 5H), 7.41 (*dd,J =* 8.8, 2.1 Hz, 1H), 7.32 (dq, *J* = 9.0, 5.0, 4.5 Hz, 1H), 7.10 (*d, J* = 4.0 Hz, 2H)

Compound 786: LCMS- 469.1 ((MH)⁺ ; @ 254 nm = 99.89% ¹H NMR (400 MHz, DMSO-d6) δ 8.25 (s, 1H), 8.17-8.10 (m, 1H), 7.89 (q, J = 17.9, 9.8 Hz, 5H), 7.78(s, 1H), 7.75-7.51 (m, 5H), 7.41 (d, J = 8.6 Hz, 1H), 7.29-7.22 (m, 1H), 7.02 (s, 2H).

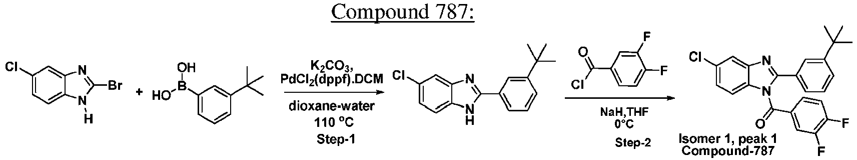

Step 1: To a solution of 2-bromo-5-chloro-1H-benzimidazole (1g, 4.36 mmol), (3-tert-butylphenyl)boronic acid (932 mg, 5.24 mmol) in dioxane:water (20:4 mL) was added K₂CO₃ (924 mg, 8.72 mmol). The reaction mixture was purged with nitrogen for five minutes. PdCl₂(dppf).dcm (354 mg, 0.436mmol, 0.1 eq) was added and it was purged again for additional five minutes followed by heating at 110°C for 16 hours. After completion of reaction, the reaction mixture was diluted with water and extracted with ethyl acetate (50 mL x 2). The combined organic layer was washed with water (50 mL), brine solution (50 mL), dried over anhydrous sodium sulfate and concentrated under reduced pressure to afford crude product, which was purified by flash chromatography (elution 0-20% EtOAc in hexane) to afford the desired Compound : (650 mg) as a yellow solid.

### Step 2: was performed as described above for step 2 of synthesis of Compound 535

Compound 787: LCMS- 425 (M)⁺ UPLC @ 254 nm = 99.18 %, @ 220 nm = 97.18 %. ¹H NMR (400 MHz, DMSO-*d*₆) δ ppm 7.96 (*d, J =* 2.1 Hz, 1H), 7.86-7.73 (*m,* 2H), 7.58 (*dd, J =* 9.1, 4.5Hz, 1H), 7.50-7.43 (*m,* 2H), 7.43-7.32 (*m,* 3H), 7.25 (*t, J* = 7.7 Hz, 1H), 1.19 (s, 9 H).

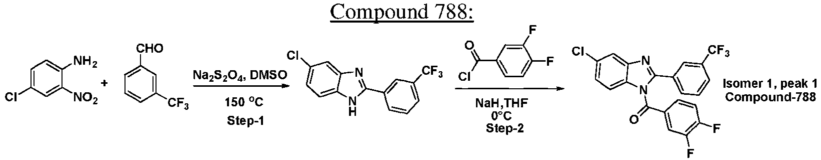

### Steps 1 and 2 were performed as described for Compound 535

Compound 788: LCMS-437.2 (M)⁺ UPLC @ 254 nm = 99.98%, @ 220 nm = 98.81%. ¹H NMR (400 MHz, DMSO-*d*₆) δ ppm 7.99 (*d, J* = 2.1 Hz, 1H), 7.92-7.82 (*m,* 3H), 7.75 (*d, J* = 8.0 Hz, 1H),7.71 (*d, J* = 8.7 Hz, 1H), 7.66-7.54 (*m,* 2H), 7.49 (*dd, J* = 8.7, 2.1 Hz, 1H), 7.42 (*dt, J* = 10.3, 8.2 Hz,1H).

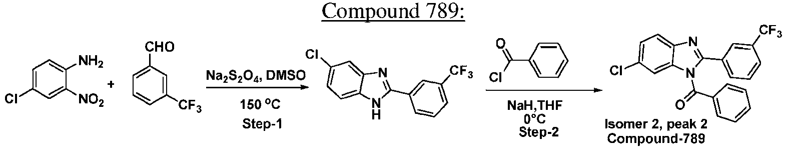

### Steps 1 and 2 were performed as described for Compound 535

Compound 789: LCMS-401.3 (M)⁺ UPLC @ 254 nm = 97.28%, @ 220 nm = 93.56%. ¹H NMR (400 MHz, DMSO-*d*₆) δ ppm 7.94-7.83 (m, 3H), 7.70 (t, J = 7.3 Hz, 3H), 7.53 (ddd, J = 21.8, 7.2, 2.9 Hz, 4H), 7.36 (t, J = 7.7 Hz, 2H).

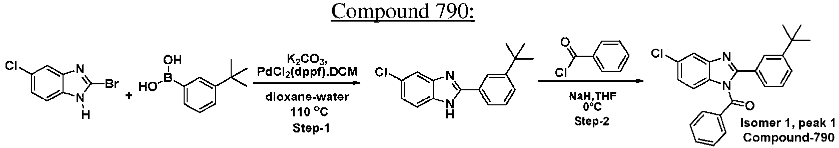

### Steps 1 and 2 were performed as described for Compound 787

Compound 790: LCMS- 389.4 (M)⁺ UPLC @ 254 nm = 97.92 %, @ 220 nm = 96.37 %. ¹H NMR (400 MHz, DMSO-*d*₆) δ ppm 7.96 (*d, J* = 2.2 Hz, 1H), 7.68 (*d, J* = 7.6 Hz, 2H), 7.60-7.50 (*m,* 2H), 7.49-7.39 (*m,* 3H), 7.36 (*t, J* = 7.7 Hz, 3H), 7.26 (*t, J* = 7.7 Hz, 1H), 1.17 (s, 9 H).

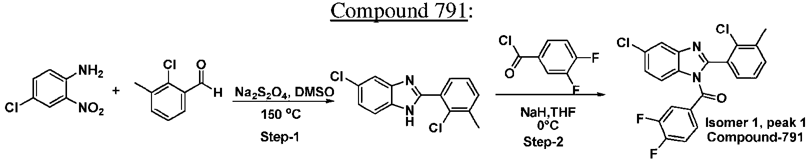

### Steps 1 and 2 were performed as described for Compound 535

Compound 791: LCMS-417.1 (M)⁺ UPLC @ 220 nm = 95.86% ¹H NMR (400 MHz, DMSO-*d*₆) δ ppm 7.98 (*d, J* = 2.1 Hz, 1H), 7.81 (d*dd, J =* 10.4, 7.7, 2.2 Hz, 1H), 7.76(*d, J* = 8.7 Hz, 1H), 7.59 (*d, J =* 10.0 Hz, 1H), 7.55-7.47 (*m,* 2H), 7.46-7.32 (*m,* 2H), 7.29 (*t*, *J* = 7.5Hz, 1H), 2.18 (s, 3H).

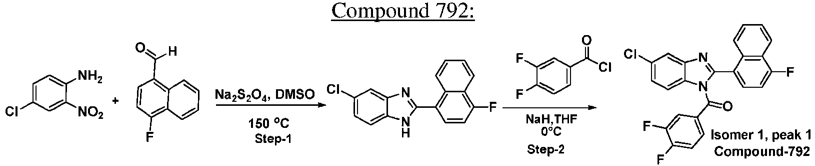

### Steps 1 and 2 were performed as described for Compound 535

Compound 792: LCMS-437.1 (M)⁺ LCMS @ 220 nm = 98.17%. ¹H NMR (400 MHz, DMSO-*d*₆) δ ppm 8.07-7.97 *(m,* 2H), 7.95 (*d, J =* 8.5 Hz, 1H), 7.87 (*d, J =* 2.1 Hz, 1H),7.68 (dq, *J =* 8.6, 5.3, 4.8 Hz, 3H), 7.57 (*td, J =* 8.4, 2.3 Hz, 2H), 7.32 (*d, J =* 8.8 Hz, 1H), 7.26 (*dd, J* = 10.4, 8.0 Hz, 1H), 7.16-7.05 (*m,* 1H).

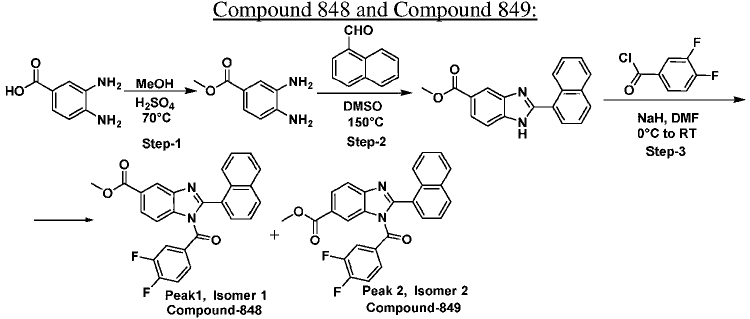

Step 1: To a stirred solution of 3,4-diaminobenzoic acid (1.0 g, 6.57 mmol) in methanol (20 mL) at 0°C was added concentrated sulphuric acid (20.0 mL) and the reaction mixture was refluxed at 70°C for 4 hours. After completion, the reaction mixture was cooled and basified using saturated solution of sodium bicarbonate. The aqueous layer was extracted with DCM. The organic layer was dried under vacuum to give crude Compound . The crude Compound was washed with ether to give desired product methyl-3,4-diaminobenzoate (0.9g) as an off-white solid.

Step 2 was performed as described above for step 1 of synthesis of Compound 579

Step 3 was performed as described above for step 2 of synthesis of Compound 535

Compound 848: LCMS- 443.3(M+H)⁺ UPLC @ 254 nm = 97.79% and @ 220 nm 95.94% ¹H NMR (400 MHz, DMSO-*d*₆) δ ppm 7.90 (*d, J*=8.33 Hz, 1 H) 7.84 (*d, J=*7.89 Hz, 1 H) 7.77 (s, 2 H) 7.56 - 7.68 (*m,* 3H) 7.44 - 7.55 (*m,* 3H) 7.31 - 7.44 (*m,* 3 H), 3.93 (s, 3H)

Compound 849: LCMS- 443.3 (M+H)⁺ UPLC @ 254 nm = 97.51% and @ 220 nm = 95.80%. ¹H NMR (400 MHz, DMSO-*d*₆) δ ppm 8.46 (*d, J*=1.75 Hz, 1 H) 8.10 (*dd, J*=8.77, 1.75 Hz, 1 H) 7.89 - 7.98 (*m,* 4 H) 7.53 - 7.70 *(m,* 4 H) 7.41 - 7.47 (*m,* 1 H) 7.35 (*br. s.,* 1 H) 7.10 (*d, J*=10.09 Hz, 1 H) 3.94 (s, 3H).

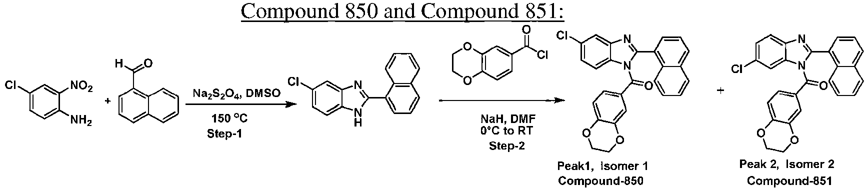

### Steps 1 and 2 were performed as described for Compound 535

Compound 850: LCMS- 441.3 (M)⁺ UPLC @ 254 nm = 91.40% and @ 220 nm 91.30%. ¹H NMR (400 MHz, DMSO-*d*₆) δ ppm 7.93 - 8.06 (*m,* 4 H) 7.60 (*d, J*=6.14 Hz, 1 H) 7.53 - 7.57 (*m,* 2 H) 7.43 - 7.52 (*m,* 3 H) 7.03 - 7.09 (*m,* 2 H) 6.64 (*d, J*=8.33 Hz, 1 H) 4.20 (*br. s., 2* H) 4.08 (*br. s., 2* H)

Compound 851: LCMS- 441.3 (M)⁺ UPLC @ 254 nm = 99.83% and @ 220 nm = 99.66%. ¹H NMR (400 MHz, DMSO-*d*₆) δ ppm 8.00 - 8.04 (*m,* 1 H) 7.90 - 7.98 (*m,* 4 H) 7.52 - 7.60 *(m,* 3 H) 7.50 (*dd,* J=8.55, 1.97 Hz, 1 H) 7.41 - 7.47 (*m,* 1 H) 7.01 - 7.08 (*m, 2* H) 6.61 (s, 1 H) 4.18 (*br. s., 2* H) 4.06 (*br. s.,* 2 H).

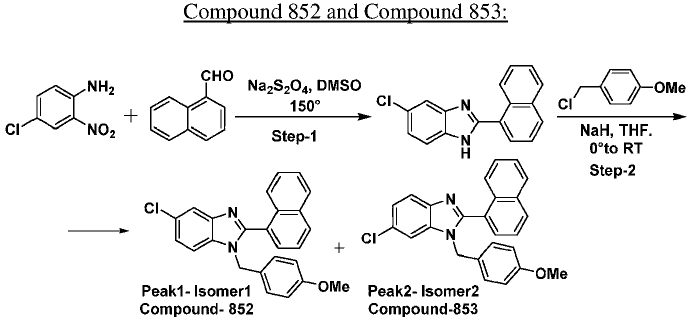

Steps 1 and 2 were performed as described for Compound 533

Compound 852: LCMS-399.3 (M)⁺ UPLC @ 254 nm = 99.34%, @ 220 nm = 99.86%. ¹H NMR (400 MHz, DMSO-*d*₆) δ ppm 8.18 (*d, J*=7.89 Hz, 1 H), 8.08 (*d, J=7.89* Hz, 1 H), 7.85 (s, 1 H), 7.75 (*d, J*=7.02 Hz, 1 H), 7.69 (*d,* 2 H), 7.58 - 7.67 (*m*, 2 H), 7.54 (*d, J*=7.02 Hz, 1 H), 7.35 (*d, J*=9.21 Hz, 1 H), 6.79 (*m, J*=8.33 Hz, 2 H), 6.70 (*m, J*=8.77 Hz, 2 H), 5.26 (s, 2 H), 3.62 (s, 3 H).

Compound 853: LCMS- 399.3 (M)⁺ UPLC @ 254 nm = 99.08%, @ 220 nm = 99.07%. ¹H NMR (400 MHz, DMSO-*d*₆) δ ppm 8.16 (*d, J*=7.89 Hz, 1 H), 8.07 *(d, J=7.89* Hz, 1 H), 7.76 - 7.89 (*m,* 1 H), 7.72 (*d, J*=6.14 Hz, 2 H), 7.62 - 7.69 (*m,* 2 H), 7.60 (*d, J*=7.89 Hz, 1 H), 7.44 - 7.55 (*m,* 1 H), 7.32 (*d, J*=7.02 Hz, 1 H), 6.78 (*m, J*=8.33 Hz, 2 H), 6.70 (*m, J*=8.33 Hz, 2 H), 5.25 (s, 2 H), 3.62 (s, 3 H).

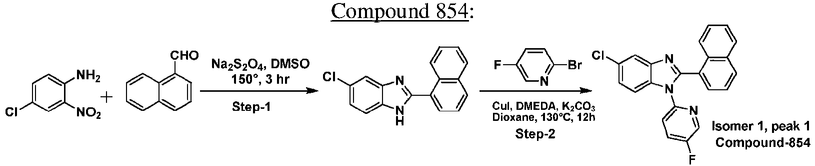

### Step 1 was performed as described above for synthesis of Compound 494

Step 2: To a stirred solution of 5-chloro-2-(naphthalen-1-yl)-1H-benzo[d]imidazole (200 mg, 0.719 mmol) and 2-bromo-5-fluoropyridine (150 mg, 0.863 mmol) in (3 mL) of dioxane were added potassium carbonate (200 mg, 1.438 mmol) and the resulting mixture was purged with nitrogen for 10 min. Copper iodide (27.4 mg, 0.143 mmol), and N,N'-dimethylethylenediamine (DMEDA) (0.03mL, 0.287 mmol) were added to the reaction mixture and it was again purged with nitrogen for 10 min followed by stirring at 130° C overnight. After completion of reaction, the reaction mixture was diluted with water and extracted with EtOAc (250 mL x 2). The combined organic layers were washed with water (250 mL) brine solution (250 mL), dried over anhydrous sodium sulphate and concentrated under reduced pressure to afford crude product, which was purified by flash chromatography (Peak 1) 5-chloro-1-(5-fluoropyridin-2-yl)-2-(naphthalen-1-yl)-1H-benzo[d]imidazole (4 mg) as a yellow solid.

Compound 854: LCMS-374.2 (M)⁺ UPLC @ 254 nm = 93.25%, @ 220 nm = 98.79%. ¹H NMR (400 MHz, DMSO-*d*₆) δ ppm 8.53 (s, *J*=3.07 Hz, 1 H), 8.06 (*dd, J*=6.36, 2.41 Hz, 2 H), 7.87 - 8.02 (*m,* 2 H), 7.76 (*dd, J*=8.33, 3.07 Hz, 2 H), 7.57 - 7.65 (*m,* 2 H), 7.43 - 7.57 (*m,* 2 H), 7.32 (*dd, J*=8.99, 4.17 Hz, 2 H).

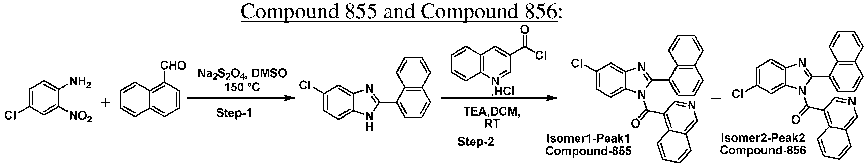

### Steps 1 and 2 were performed as described for Compound 529

Compound 855: LCMS-434.2 (M)⁺ UPLC @ 254 nm = 97.93%, @ 220 nm = 95.59%. ¹H NMR (400 MHz, DMSO-*d*₆) δ ppm 8.75 (*d, J*=2.19 Hz, 1 H), 8.52 (*d, J=*1.75 Hz, 1 H), 8.07 (*d, J*=1.75 Hz, 1 H), 8.01 (*d, J*=8.33 Hz, 1 H), 7.94 (*d, J*=8.77 Hz, 1 H), 7.80 - 7.84 (m, 1 H), 7.74 - 7.78 (*m,* 1 H), 7.65 - 7.72 (*m,* 2 H), 7.47 - 7.62 *(m,* 6 H), 7.18 - 7.23 *(m,* 1 H).

Compound 856: LCMS- 434.3 (M)⁺ UPLC @ 254 nm = 98.91%, @ 220 nm = 97.74%. ¹H NMR (400 MHz, DMSO-*d*₆) δ ppm 8.71 (s, 1 H), 8.47 (s, 1 H), 7.97 - 8.04 (*m,* 3 H), 7.78 - 7.83 (*m,* 1 H), 7.75 (*d, J*=6.14 Hz, 1 H), 7.57 - 7.69 (*m,* 4 H), 7.43 - 7.55 (*m,* 4 H), 7.18 (*t, J*=7.89 Hz, 1 H).

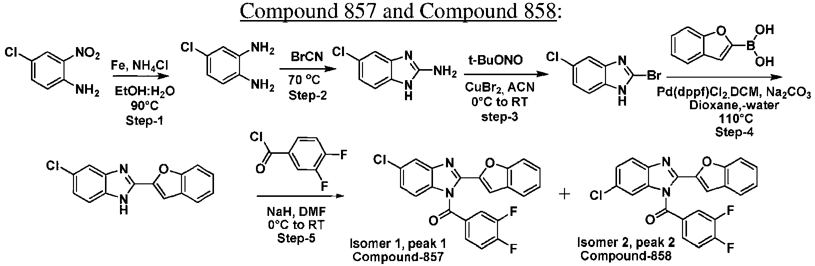

### Steps 1-5 were performed as described for Compounds 588 and 584

Compound 857: LCMS- 409.3 (M)⁺ UPLC @ 254 nm = 99.01 %, @ 220 nm = 99.30 %. ¹H NMR (400 MHz, DMSO-*d*₆) δ ppm 8.04 - 8.11 (*m,* 1 H) 8.01 (*d, J*=1.75 Hz, 1 H) 7.71 (*d, J*=7.45 Hz, 2 H) 7.63 (s, 1 H) 7.53 - 7.57 (*m,* 1 H) 7.44 - 7.50 (*m,* 2 H) 7.43 (s, 1 H) 7.38 (t, *J*=7.24 Hz, 1 H) 7.27 - 7.33 (*m,* 1 H).

Compound 858: LCMS- 409.3 (M)⁺ UPLC @ 254 nm = 96.56%, @ 220 nm = 97.53% ¹H NMR (400 MHz, DMSO-*d*₆) δ ppm 8.01 - 8.08 (m, 1 H) 7.93 (*d, J*=8.33 Hz, 1 H) 7.70 (*d, J*=7.45 Hz, 2 H) 7.65 (*d, J*=2.19 Hz, 1 H) 7.61 (s, 1 H) 7.53 (*dd, J*=8.33, 2.19 Hz, 1 H) 7.39 - 7.49 (*m,* 2 H) 7.34 - 7.39 (*m,* 1 H) 7.26 - 7.32 (*m,* 1 H).

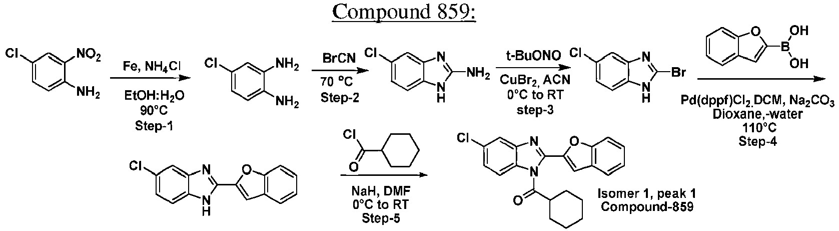

### Steps 1-5 were performed as described for Compounds 588 and 584

Compound 859: LCMS- 379.3 (M)⁺ UPLC @ 254 nm = 97.72 %, @ 220 nm = 97.66 %. ¹H NMR (400 MHz, DMSO-*d*₆) δ ppm 7.95 (*d,* J=1.75 Hz, 1 H) 7.86 (*dd, J=*8.11*,* 3.73 Hz, 2 H) 7.69 - 7.78 (*m,* 2 H) 7.47 - 7.55 (*m,* 2 H) 7.36 - 7.44 (*m,* 1 H), 2.67 (m, 1H) 1.90 (*d, J*=10.96 Hz, 2 H) 1.64 (*d, J*=13.59 Hz, 2 H) 1.50 (*d, J*=9.21 Hz, 2 H) 1.21 (*d, J*=15.79 Hz, 2 H) 0.87 - 0.99 (*m,* 2 H).

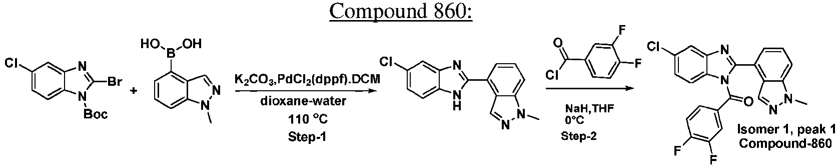

### Steps 1-2 were performed as described for synthesis of Compound 787

LCMS-423.3 (M)⁺ UPLC @ 254 nm = 95.39 %, @ 220 nm = 94.01 %.

¹H NMR (400 MHz, DMSO-*d*₆) δ ppm 8.14 (s, 1H), 8.02 (s, 1H), 7.89-7.79 *(m,* 1H), 7.70 (*d, J* = 8.4 Hz, 1H), 7.58 (*t, J =* 8.4 Hz, 1H), 7.46 (*d, J =* 8.4 Hz, 1H), 7.34 (q, J = 8.8, 8.0 Hz, 3H), 7.26 (*d, J =* 7.1 Hz, 1H), 4.06 (s, 3H).

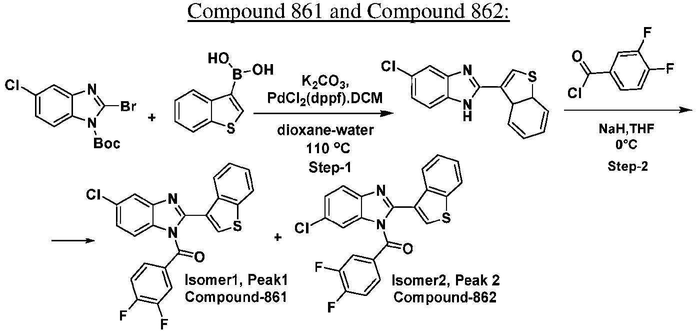

### Steps 1 and 2 were performed as described for synthesis of Compound 787

Compound 861: LCMS-425.1 (M)⁺ UPLC @ 254 nm = 99.44 %, @ 220 nm = 99.88 %. ¹H NMR (400 MHz, DMSO-*d*₆) δ 8.11-7.96 (*m,* 4H), 7.78 (*d, J* = 8.7 Hz, 1H), *7.72* (*ddd, J =* 10.1*,* 7.6, 2.2 Hz, 1H), 7.54-7.39 (*m,* 4H), 7.25 (*dt, J* = 10.5, 8.1 Hz, 1H).

Compound 862: LCMS- 425.1 (M)⁺ UPLC @ 254 nm = 99.59 %, @ 220 nm = 99.38%. ¹H NMR (400 MHz, DMSO-*d*₆) δ 8.09-8.01 (*m,* 2H), 7.98 (*d, J* = 7.9 Hz, 1H), *7.94* (*d, J* = 8.5 Hz, 1H),7.85 (*d, J* = 2.1 Hz, 1H), 7.67 *(ddd, J* = 10.2, 7.7, 2.1 Hz, 1H), 7.54 (*dd, J* = 8.5, 2.1 Hz, 1H), 7.45 (dq, *J*= 14.6, 7.4 Hz, 3H), 7.22 (*dt, J* = 10.4, 8.1 Hz, 1H).

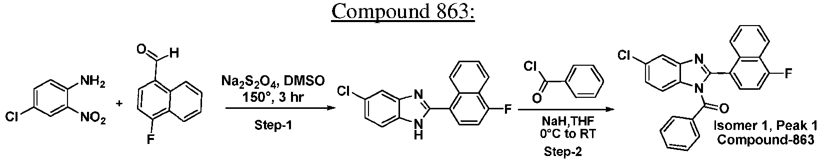

### Steps 1 and 2 were performed as described for synthesis of Compound 535

Compound 863: LCMS -401.2(M)⁺ UPLC @ 254 nm = 99.95%, @ 220 nm = 99.82%. ¹H NMR (401 MHz, DMSO-*d*₆) δ ppm 8.08 (d, J = 7.9 Hz, 1H), 8.05-7.97 (m, 2H), 7.72-7.60 (m, 4H), 7.53-7.46 (m, 3H), 7.36 (t, J = 7.5 Hz, 1H), 7.24 (dd, J = 10.5, 8.0 Hz, 1H), 7.09 (t, J = 7.6 Hz, 2H).

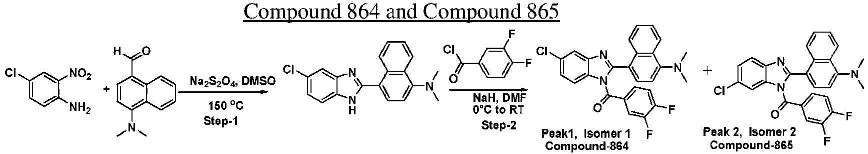

### Steps 1 and 2 were performed as described for synthesis of Compound 535

Compound 864: LCMS- 322.3 (fragment; benzimidazole core) UPLC @ 254 nm = 99.70% and @ 220 nm 97.70% ¹H NMR (400 MHz, DMSO-*d*₆) δ ppm 8.02 - 8.06 (*m*, 1 H) 8.00 (*d, J*=1.75 Hz, 1 H) 7.92 (*d, J*=8.77 Hz, 1 H) 7.77 - 7.81 (*m,* 1 H) 7.49 - 7.57 (*m,* 4 H) 7.41 (*br. s.,* 1 H) 7.15 (*br. s.,* 1 H) 6.99 (*d, J*=10.96 Hz, 1 H) 6.94 (*d, J*=8.33 Hz, 1 H) 2.78 (s, 6 H)

Compound 865: LCMS- 322.3 (fragment; benzimidazole core) UPLC @ 254 nm = 91.49% and @ 220 nm = 92.38%. ¹H NMR (400 MHz, DMSO-*d*₆) δ ppm 8.05 (*br. s.,* 1 H) 7.98 (s, 1 H) 7.93 (*d, J*=8.77 Hz, 1 H) 7.76 (*br. s.,* 1 H) 7.48 - 7.58 (*m,* 3 H) 7.37 (*br. s.,* 1 H) 7.14 (*br. s., 2* H) 6.89 - 7.01 (*m,* 2 H) 2.77 (s, 6 H).

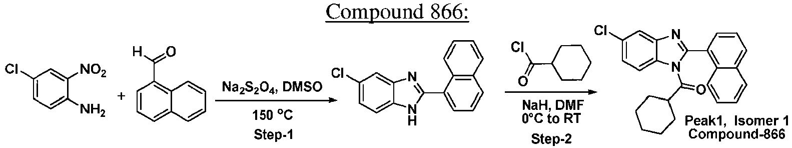

### Steps 1-2 were performed as described for synthesis of Compound 535

Compound 866: LCMS- 389.3 (M)⁺ UPLC @ 254 nm = 99.26% and @ 220 nm 98.82%. ¹H NMR (400 MHz, DMSO-*d*₆) δ ppm 8.24 (*d, J*=8.33 Hz, 1 H) 8.13 (*dd, J=*8.33, 5.26 Hz, 2 H) 7.93 - 7.96 (*m,* 1 H) 7.88 (*d, J*=7.02 Hz, 1 H) 7.71 (*t, J*=7.45 Hz, 1 H) 7.64 (*d, J*=4.38 Hz, 2 H) 7.58 (*d, J*=7.45 Hz, 1 H) 7.51 - 7.56 (*m,* 1 H) 1.95 - 2.04 (*m,* 1 H) 1.35 (*d, J*=12.28 Hz, 4 H) 1.21 - 1.30 (*m,* 2 H) 1.09 - 1.21 (*m,* 3 H) 0.90 (*d, J*=13.15 Hz, 1 H).

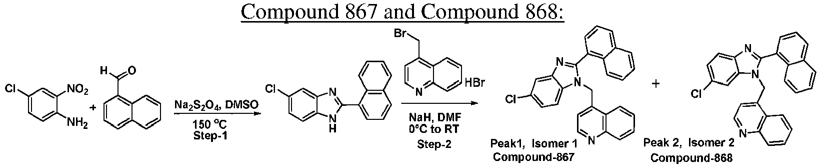

### Steps 1-2 were performed as described for synthesis of Compound 535

Compound 867: LCMS- 420.3 (M)⁺ UPLC @ 254 nm = 98.40% and @ 220 nm 99.35%. ¹H NMR (400 MHz, DMSO-*d*₆) δ ppm 8.63 (*d, J*=4.39 Hz, 1 H) 7.78 - 8.04 (*m,* 7 H) 7.74 (*br. s.,* 1 H) 7.63 (*d, J*=6.58 Hz, 1 H) 7.55 (*d, J*=7.45 Hz, 2 H) 7.45 - 7.52 (*m,* 2 H) 7.38 (*d*, *J*=9.21 Hz, 1 H) 6.54 (*br. s.,* 1 H) 5.96 (s, 2 H)

Compound 868: LCMS- 420.3 (M)⁺ UPLC @ 254 nm = 99.92% and @ 220 nm = 99.66%. ¹H NMR (400 MHz, DMSO-*d*₆) δ ppm 8.62 (*d, J*=4.38 Hz, 1 H) 8.04 (*d, J*=8.33 Hz, 1 H) 7.91 - 8.00 *(m,* 4 H) 7.84 (*d, J*=8.33 Hz, 1 H) 7.73 (*d, J*=8.33 Hz, 1 H) 7.47 - 7.66 (*m,* 6 H) 7.35 (*d, J*=10.52 Hz, 1 H) 6.57 (*d, J*=4.82 Hz, 1 H) 5.96 (s, 2 H).

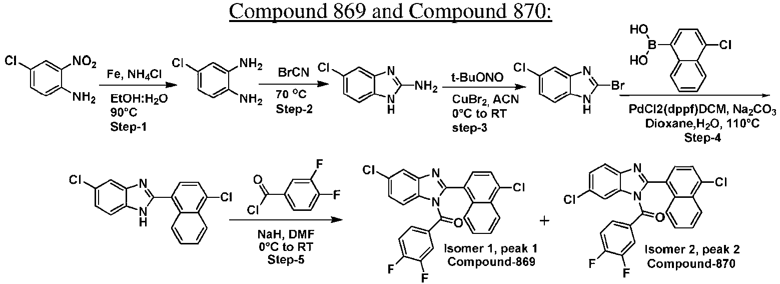

### Steps 1-5 were performed as described for synthesis of Compound 590

Compound 869: LCMS- 453.2(M)⁺ UPLC @ 254 nm = 99.32% and @ 220 nm 98.61% ¹H NMR (400 MHz, DMSO-*d*₆) δ ppm 8.19 (*d, J*=8.33 Hz, 1 H) 7.98 - 8.05 (*m,* 2 H) 7.71 - 7.79 (*m,* 2 H) 7.58 - 7.71 (*m,* 4 H) 7.53 (*dd, J*=8.77, 2.19 Hz, 1 H) 7.35 (*br. s.,* 1 H) 7.10 - 7.19 (*m,* 1 H)

Compound 870: LCMS- 453.2(M)⁺ UPLC @ 254 nm = 99.66% and @ 220 nm = 99.21%. ¹H NMR (400 MHz, DMSO-*d*₆) δ ppm 8.18 (*d, J*=8.33 Hz, 1 H) 8.00 (*d*, *J=*7.89 Hz, 1 H) 7.96 (*d, J*=8.77 Hz, 1 H) 7.86 (*d, J*=1.75 Hz, 1 H) 7.66 - 7.77 *(m,* 2 H) 7.61 - 7.66 *(m,* 2 H) 7.57 (*dd, J*=8.55, 1.97 Hz, 2 H) 7.31 (*br. s.,* 1 H) 7.06 - 7.15 *(m,* 1 H).

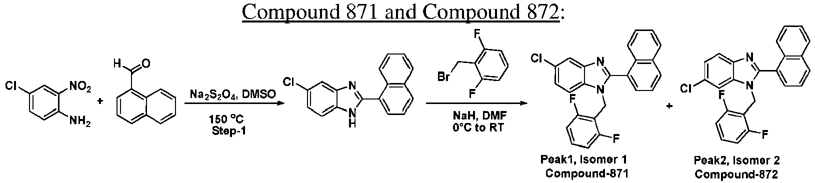

### Steps 1 and 2 were performed as described for synthesis of Compound 533

Compound 871: LCMS- 405.3 (M)⁺ UPLC @ 254 nm = 99.74% and @ 220 nm 99.71%. ¹H NMR (400 MHz, DMSO-d6) δ 8.11 (d, J = 8.1 Hz, 1H), 8.00 (d, J = 8.2 Hz, 1H), 7.82 (d, J = 2.1 Hz, 1H), 7.73-7.58 (m, 3H), 7.58-7.46 (m, 2H), 7.46-7.35 (m, 2H), 7.14 (ddd, J = 14.9, 8.4, 6.5 Hz, 1H), 6.74 (t, J = 8.2 Hz, 2H), 5.46 (s, 2H).

Compound 872: LCMS- 401.2 (M)⁺ UPLC @ 254 nm = 99.91% and @ 220 nm = 99.71%. ¹H NMR (400 MHz, DMSO-d6) δ 8.10 (d, J = 8.1 Hz, 1H), 7.99 (d, J = 8.2 Hz, 1H), 7.81 (d, J = 2.3 Hz,1H), 7.76 (d, J = 8.5 Hz, 1H), 7.66 (d, J = 7.0 Hz, 1H), 7.60 (t, J = 7.6 Hz, 1H), 7.52 (ddd, J = 8.3, 6.5,1.5 Hz, 1H), 7.47-7.29 (m, 3H), 7.12 (ddd, J = 15.0, 8.5, 6.7 Hz, 1H), 6.72 (t, J = 8.2 Hz, 2H), 5.46 (s,2H).

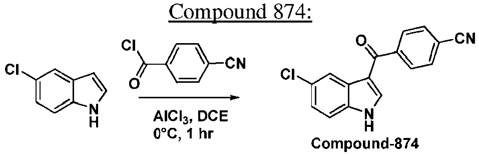

### Step 1 was performed as described for synthesis of Compound 359

Compound 874: LCMS: 281.2 (M)⁺ UPLC @ 254 = 99.92 %, @ 220 = 99.82 % ¹H NMR (400 MHz, DMSO-*d*₆) δ ppm 12.38 (*br. s.,* 1 H) 8.24 (s, 1 H) 8.06 (s, 1 H) 8.02 (*d, J*=7.89 Hz, 2 H) 7.93 (*d,* J=8.33 Hz, 2 H) 7.56 (*d, J*=8.77 Hz, 1 H) 7.32 (*dd, J=*8.77*,* 2.19 Hz, 1 H).

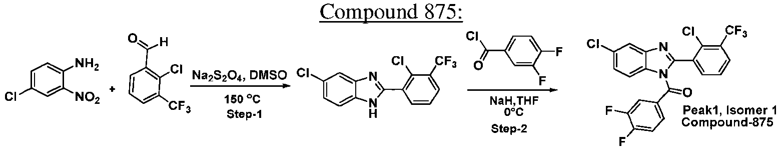

### Steps 1 and 2 were performed as described for synthesis of Compound 535

Compound 875: LCMS-471.2 (M)⁺ UPLC @ 254 nm = 97.03%, @ 220 nm = 83.96%. ¹H NMR (400 MHz, DMSO-*d*₆) δ ppm δ 8.06-7.99 (*m,* 2H), 7.94-7.83 *(m,* 2H), 7.74 (*d, J* = 8.7 Hz, 1H), 7.63 (q, *J* = 9.9, 8.9 Hz, 2H), 7.54 (*dd, J* = 8.9, 2.1 Hz, 1H), 7.41 (q, *J* = 8.9 Hz, 1H).

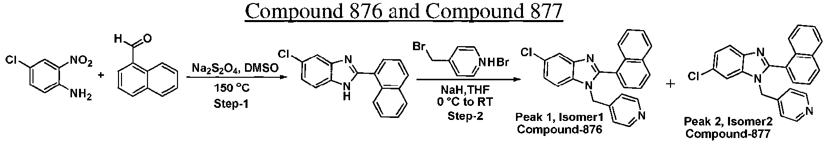

### Steps 1and 2 were performed as described for synthesis of Compound 533

Compound 876: LCMS-370.2 (M)⁺ UPLC @ 254 nm = 96.14%, @ 220 nm = 95.83% ¹H NMR (400 MHz, DMSO-*d*6) δ 8.37-8.30 *(m,* 2H), 8.13 (*d, J* = 8.2 Hz, 1H), 8.04 (*d, J* = 8.2 Hz, 1H),7.83 (*d, J* = 8.6 Hz, 1H), 7.75 *(d, J =* 2.1 Hz, 1H), 7.73-7.64 (*m*, 2H), 7.60 (q, *J* = 7.9 Hz, 2H), 7.51 (*t, J* = 7.6 Hz, 1H), 7.36 *(dd, J =* 8.6, 2.1 Hz, 1H), 6.81 (*d, J* = 5.5 Hz, 2H), 5.39 (s, 2H).

Compound 877: LCMS- 370.1 UPLC (M)⁺ @ 254 nm = 97.97%, @ 220 nm = 98.11%. ¹H NMR (400 MHz, DMSO-*d*6) δ 8.37-8.30 *(m,* 2H), 8.14 (*d, J =* 8.0 Hz, 1H), 8.04 (*d, J* = 8.1 Hz, 1H), 7.90 (*d, J =* 2.0 Hz, 1H), 7.74-7.65 *(m,* 2H), 7.65-7.47 (*m,* 4H)*,* 7.36 (*dd, J* = 8.6, 2.1 Hz, 1H), 6.83 (*d,J =* 5.5 Hz, 2H), 5.39 (s, 2H).

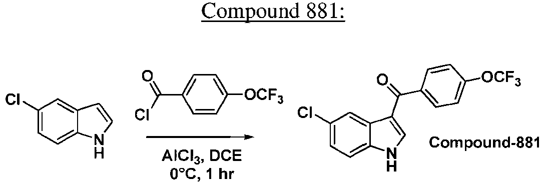

### Step 1 was performed as described for step 2 of synthesis of Compound 359

Compound 881: LCMS- 340.2 (M)⁺ UPLC @ 254 = 99.64 %, @ 220 = 99.89 % ¹H NMR (400 MHz, DMSO-*d*₆) δ ppm 12.32 (br. s., 1 H) 8.24 (d, *J*=2.19 Hz, 1 H) 8.10 (s, 1H) 7.91 - 7.99 (m, 2H) 7.54 (t, *J*=8.99 Hz, 3 H) 7.30 (dd, *J*=8.33, 2.19 Hz, 1 H).

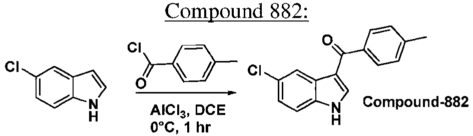

### Step 1 was performed as described for step 2 of synthesis of Compound 359

Compound 882: LCMS- 270.1 (M)⁺ UPLC @220 = 96.0 % ¹H NMR (400 MHz, DMSO-*d*₆) δ ppm 12.19 (br. s., 1 H) 8.21 (d, *J=*1.75 Hz, 1 H) 8.02 (s, 1 H) 7.70 (m, *J*=7.89 Hz, 2 H) 7.52 (d, *J*=8.77 Hz, 1 H) 7.34 (m, *J*=7.89 Hz, 2 H) 7.26 (dd, *J*=8.33, 2.19 Hz, 1 H) 2.39 (s, 3 H).

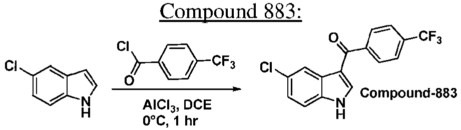

### Step 1 was performed as described for step 2 of synthesis of Compound 359

Compound 883: LCMS- 324.1 (M)+ UPLC @220 = 94.83 % ¹H NMR (400 MHz, DMSO-*d*₆) δ ppm 12.38 (br. s., 1 H) 8.25 (d, *J*=2.19 Hz, 1 H) 8.10 (s, 1 H) 7.98 (d, *J*=7.89 Hz, 2 H) 7.91 (d, *J*=8.33 Hz, 2 H) 7.56 (d, *J*=8.77 Hz, 1 H) 7.31 (dd, *J*=8.77, 2.19 Hz, 1 H).

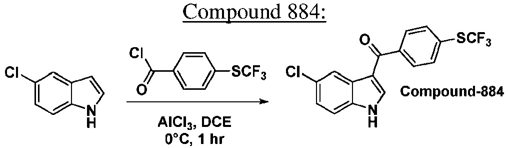

### Step 1 was performed as described for step 2 of synthesis of Compound 359

Compound 884: LCMS- 356.1 (M)⁺ UPLC @220 = 99.38 % ¹H NMR (400 MHz, DMSO-*d*₆) δ ppm 12.32 (br. s., 1 H) 8.24 (d, *J*=2.19 Hz, 1 H) 8.09 (s, 1 H) 7.85 - 7.93 (m, 3H) 7.56 (d, *J*=8.33 Hz, 2 H) 7.31 (dd, *J*=8.77, 2.19 Hz, 1 H).

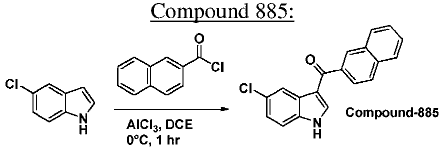

### Step 1 was performed as described for step 2 of synthesis of Compound 359

Compound 885: LCMS- 306.1 (M)+ UPLC @220 = 99.18 % ¹H NMR (400 MHz, DMSO-*d*₆) δ ppm 12.32 (br. s., 1 H) 8.43 (s, 1 H) 8.28 (d, *J*=2.19 Hz, 1 H) 8.11 - 8.17 (m, 2 H) 8.07 (d, *J*=8.77 Hz, 1 H) 8.03 (d, *J*=7.45 Hz, 1 H) 7.88 (dd, *J*=8.33, 1.75 Hz, 1 H) 7.60 - 7.69 (m, 2 H) 7.57 (d, *J*=8.77 Hz, 1 H) 7.29 (dd, *J*=8.55, 1.97 Hz, 1 H).

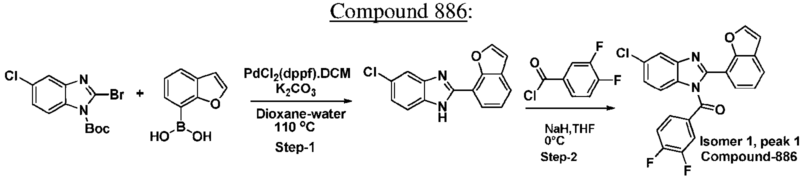

### Steps 1 and 2 were performed as described for synthesis of Compound 787

Compound 886: LCMS-409.3 (M)⁺ UPLC @ 254 nm = 97.18 %, @ 220 nm = 99.18 %. ¹H NMR (400 MHz, DMSO-*d*₆) δ ppm 8.02 (s, 1 H) 7.96 (s, 1 H) 7.83 (*d, J=*8.77 Hz, 1 H) 7.64 - 7.74 (*m,* 3 H) 7.52 (*d, J*=8.77 Hz, 1 H) 7.40 (*br. s.,* 1 H) 7.33 (*t, J*=7.67 Hz, 1 H) 7.14 - 7.24 (*m,* 1 H) 6.92 *(d, J*=1.75 Hz, 1 H).

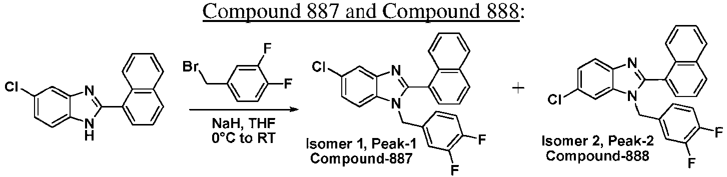

### Step 1 was performed as described for synthesis of Compound 533

Compound 887: LCMS-405.3 (M)+ UPLC @ 254 nm = 98.93 %, @ 220 nm = 99.36 % ¹H NMR (400 MHz, Methanol-*d4*) δ 8.13 (dd, J = 7.5, 2.0 Hz, 1H), 8.01 (d, J = 8.2 Hz, 1H), 7.76 (d, J = 8.6 Hz, 1H), 7.69-7.61 (m, 2H), 7.61-7.53 (m, 1H), 7.49 (dd, J = 5.8, 1.5 Hz, 2H), 7.44-7.32 (m, 1H), 7.00 (dt, J = 10.5, 8.3 Hz, 2H), 6.72-6.56 (m, 2H), 5.26 (s, 2H).

Compound 888: LCMS-405.3 (M)⁺ UPLC @ 254 nm = 98.78 %, @ 220 nm = 98.94 %. ¹H NMR (400 MHz, Methanol-*d4*) δ 8.13 (dd, J = 7.7, 1.9 Hz, 1H), 8.01 (d, J = 8.2 Hz, 1H), 7.79 (d, J = 1.9 Hz, 1H), 7.68-7.60 (m, 2H), 7.60-7.54 (m, 2H), 7.54-7.44 (m, 2H), 7.39 (dd, J = 8.7, 2.0 Hz, 1H), 7.00 (dt, J = 10.4, 8.4 Hz, 1H), 6.70 (ddd, J = 10.8, 7.6, 2.2 Hz, 1H), 6.64-6.55 (m, 1H), 5.28 (s, 2H).

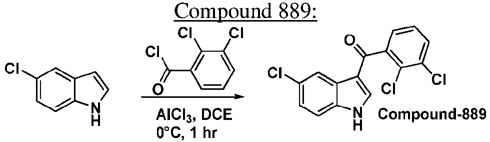

### Step 1 was performed as described for step 2 of synthesis of Compound 359

Compound 889: LCMS: 324.1 (M)⁺ UPLC @ 254 = 99.95%, @ 220 = 99.84% ¹H NMR (400 MHz, DMSO-*d*₆) δ ppm 12.37 (*br. s.,* 1 H) 8.16 (s, 1 H) 7.80 - 7.86 (*m,* 1 H) 7.76 - 7.80 (*m,* 1 H) 7.46 - 7.57 (*m,* 3 H) 7.32 (*dd, J*=8.77, 2.19 Hz, 1 H)

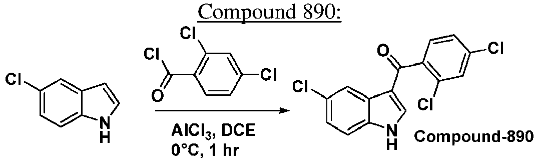

### Step 1 was performed as described for step 2 of synthesis of Compound 359

Compound 890: LCMS: 324.2 (M)⁺ UPLC @ 254 = 99.44%, @ 220 = 99.58% ¹H NMR (400 MHz, DMSO-*d*₆) d ppm 12.37 (*br. s.,* 1 H) 8.16 (*d, J*=1.75 Hz, 1 H) 7.84 (s, 1 H) 7.78 (s, 1 H) 7.48 - 7.60 (*m,* 3 H) 7.31 (*dd, J*=8.55, 1.97 Hz, 1 H).

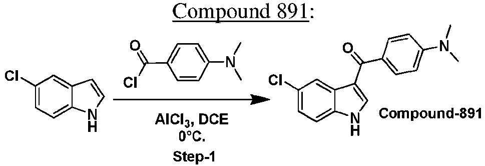

### Step 1 was performed as described for step 2 of synthesis of Compound 359

Compound 891: LCMS- 299.1 (M)⁺ UPLC:- At 254 nm: 96.76%, At 220 nm: 96.34%. ¹H NMR (400 MHz, DMSO-*d*₆) δ ppm 12.07 (*br. s.,* 1 H) 8.17 (*d, J*=1.75 Hz, 1 H) 8.02 (*d, J*=2.63 Hz, 1 H) 7.75 (*m, J*=9.21 Hz, 2 H) 7.52 (*d,* J=8.33 Hz, 1 H) 7.21 - 7.28 (*m,* 1 H) 6.79 (*m, J*=9.21 Hz, 2 H) 3.03 (s, 6 H).

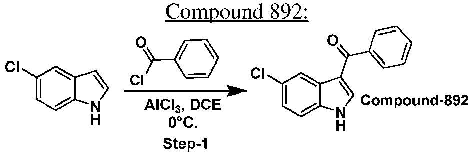

### Step 1 was performed as described for step 2 of synthesis of Compound 359

Compound 892: LCMS- 256.2 (M)⁺ UPLC:- At 255 nm 99.84%, At 220 nm: 99.85%. ¹H NMR (400 MHz, DMSO-*d*₆) δ ppm 12.26 *(̅br. s.,* 1 H) 8.24 (*d, J*=2.19 Hz, 1 H) 8.04 (s, 1 H) 7.80 (*d, J*=7.02 Hz, 2 H) 7.44 - 7.69 (*m,* 4 H) 7.29 (*dd, J*=8.99, 1.97 Hz, 1 H).

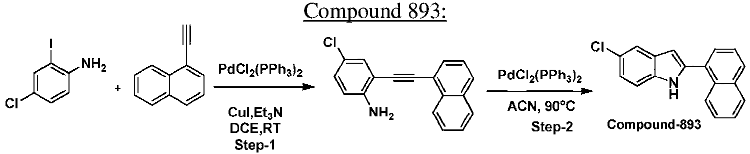

Step 1: To a solution of 4-chloro-2-iodoaniline (0.200 g, 0.78 mmol) and 1-ethynylnaphthalene (0.179 g, 1.18 mmol) in dichloroethane (10.0 ml), triethylamine (0.4 mL, 3.15 mmol) was added. The reaction mixture was purged with nitrogen. After few minutes of purging, copper(I) iodide (0.004 g, 0.023 mmol) and *bis*(triphenylphosphine)palladium(II) dichloride (0.017 g, 0.023 mmol) was added. The reaction mixture was purged again for few more minutes and then the reaction mixture was stirred at RT for 4 hours. After completion of reaction, the reaction mixture was diluted with water and extracted with ethyl acetate (50 mL x 2). The combined organic layer was washed with water (50 mL), brine solution (50 mL), dried over anhydrous sodium sulfate and concentrated under reduced pressure to afford crude product, which was purified by flash chromatography (elution 0-30% EtOAc in hexane) to afford the desired Compound : 4-chloro-2-(naphthalen-1-ylethynyl)aniline (0.200 g) as a off white solid.

Step 2: A solution of 4-chloro-2-(naphthalen-1-ylethynyl)aniline (0.200 g, 0.72 mmol) in ACN (8.0 mL) was purged with nitrogen for five minutes. *Bis*(triphenylphosphine)palladium(II) dichloride (0.019 g, 0.027 mmol) was added to the reaction mixture and it was purged with N₂ for additional five minutes. The reaction mixture was stirred at 90°C for 4 hours. After completion of reaction, the reaction mixture was diluted with water and extracted with ethyl acetate (50 mL x 2). The combined organic layer was washed with water (50 mL), brine solution (50 mL), dried over anhydrous sodium sulfate and concentrated under reduced pressure to afford crude product, which was purified by flash (0-30% EtOAc in hexane) to afford the desired Compound : 5-chloro-2-(naphthalen-1-yl)-1H-indole (0.115 g) as an off-white solid.

Compound 893: LCMS- 278.2 (M)⁺ UPLC:- At 254 nm: 99.93%, At 220 nm: 99.38%. ¹H NMR (400 MHz, DMSO-*d*₆) δ ppm 11.79 (*br. s.,* 1 H) 8.27 (*dd, J*=6.14, 3.51 Hz, 1 H) 7.97 - 8.08 (*m,* 2 H) 7.68 - 7.74 (*m,* 1 H) 7.54 - 7.68 *(m,* 4 H) 7.45 (*d, J*=8.77 Hz, 1 H) 7.14 (*dd, J*=8.77, 2.19 Hz, 1 H), 6.74 (*d, J*=1.75 Hz, 1 H).

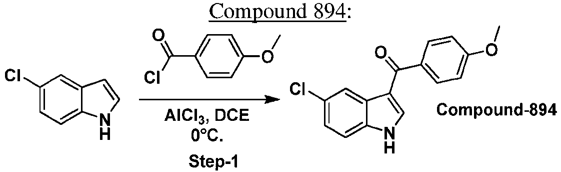

### Step 1 was performed as described for step 2 of synthesis of Compound 359

Compound 894: LCMS- 286.2 (M)⁺ UPLC: @ 254 nm: 99.42%, @ 220 nm: 99.55%. ¹H NMR (400 MHz, DMSO-*d*₆) δ ppm 12.19 (*br. s.,* 1 H) 8.21 (*d, J*=1.75 Hz, 1 H) 8.05 (s, 1 H) 7.82 (*m, J*=8.77 Hz, 2 H) 7.54 (*d, J*=8.33 Hz, 1 H) 7.27 (*dd, J*=8.77, 1.75 Hz, 1 H) 7.08 (m, *J*=8.77 Hz, 2 H) 3.86 (s, 3 H).

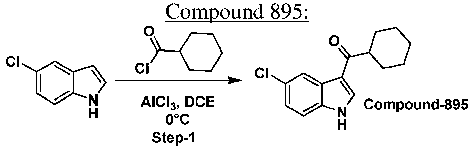

### Step 1 was performed as described for step 2 of synthesis of Compound 359

Compound 895: LCMS- 262.2 (M)⁺ UPLC:- @ 254 nm: 99.85%, @ 220 nm: 99.91% ¹H NMR (400 MHz, DMSO-d6) δ 12.10 (s, 1H), 8.44 (s, 1H), 8.18 (d, J = 2.2 Hz, 1H), 7.47 (d, J *=* 8.6 Hz, 1H), 7.22 (dd, J = 8.6, 2.2 Hz, 1H), 3.17 (qt, J = 6.6, 2.9 Hz, 1H), 1.91-1.58 (m, 5H), 1.41 (tt, J = 8.8, 3.3 Hz, 4H), 1.27-1.07 (m, 1H).

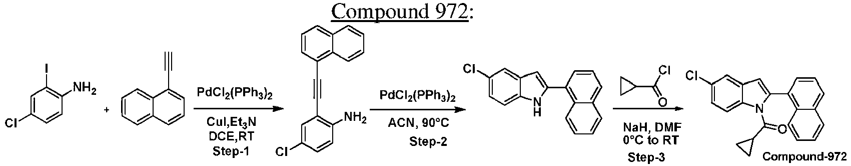

### Steps 1 and 2 were performed as described for synthesis of Compound 893

### Step 3 was performed as described for synthesis of Compound 535

Compound 972: LCMS- 346.3 (M)⁺ UPLC @ 254 nm = 96.78% and @ 220 nm 96.44%. ¹H NMR (400 MHz, DMSO-*d*₆) δ ppm 8.19 (*d, J=8.77* Hz, 1 H) 8.05 (t, *J=*7.67 Hz, 2 H) 7.78 (*d*, *J*=2.19 Hz, 1 H) 7.75 - 7.50 (*m,* 5 H) 7.40 (*dd, J*=8.77, 2.19 Hz, 1 H), 6.93 (s, 1H), 1.41-1.32 (*m,* 1 H) 1.27 - 1.22 (*m,* 1 H) 0.95 - 0.80 (*m,* 2 H), 0.50 (*br. s.,* 1H)*.*

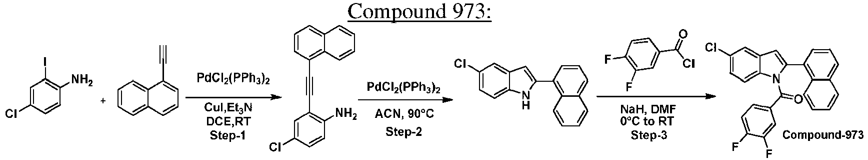

### Steps 1 and 2 were performed as described for synthesis of Compound 893

### Step 3 was performed as described for synthesis of Compound 535

Compound 973: LCMS- 418.3 (M)⁺ UPLC @ 254 nm = 99.91% and @ 220 nm 99.74%. ¹H NMR (400 MHz, DMSO-*d*₆) δ ppm 7.95 (*d*, *J*=8.77 Hz, 1 H) 7.83 - 7.88 (*m*, 2 H) 7.79 (*d, J*=7.89 Hz, 1 H) 7.70 - 7.74 *(m,* 1 H) 7.48 - 7.56 *(m,* 3 H) 7.43 (*dd, J*=8.77, 2.19 Hz, 2 H) 7.39 (*d, J*=7.89 Hz, 1 H) 7.32 (*br. s.,* 1 H) 7.09 (*br. s.,* 1 H) 6.97 (*d, J*=10.09 Hz, 1 H).

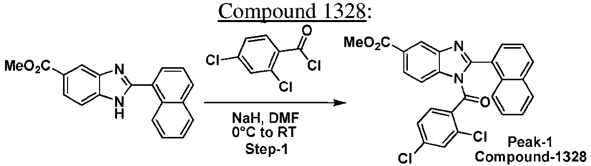

### Step 1 was performed as described for synthesis of Compound 535

Compound 1328: LCMS- (M)⁺ (475.2 ) 97.65% @220 nm ¹H NMR (400 MHz, DMSO-*d*₆) δ ppm 8.45 (s, 1 H), 8.26 - 8.13(*m*, 1 H), 7.94 - 7.85 (*m,* 2 H), 7.79 (*d, J=*8.77 Hz, 1 H), 7.76 (*d, J*=8.77 Hz, 1 H), 7.71 - 7.62 (*m,* 1 H),7.62 - 7.46 (*m,* 2 H), 7.41 (*t, J*=7.67 Hz, 1 H), 7.24 (*d,* J=7.02 Hz, 1 H), 7.13 (*br. s.,* 1 H), 6.74 (*br. s.,* 1 H), 3.94 (s, 3 H).

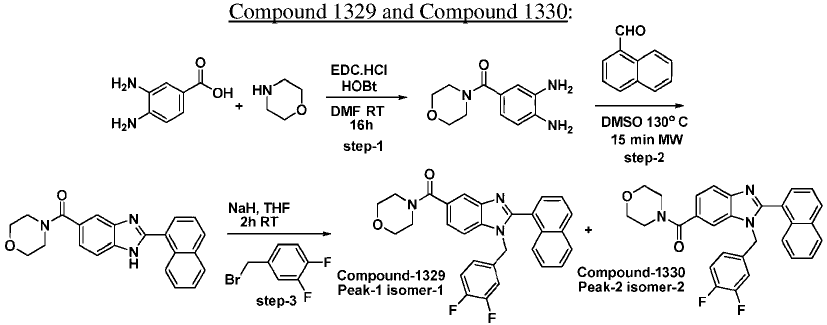

Step 1: To a stirred solution of 3,4-diaminobenzoic acid (2.0 g, 13.15 mmol) in DMF (10 mL) was added EDC.HCl (3.7g, 19.72 mmol) and HOBT (2.66g, 19.22 mmol). The resultant reaction mixture was allowed to stir for 10min followed by addition of morpholine (1.4 mL, 15.78 mmol) and then stirring for 16h at RT. After completion of reaction, the reaction mixture was diluted with water (20 ml) and extracted with (10% methanol in DCM). The combined organic layer was dried over anhydrous sodium sulfate filtered and concentrated under reduced pressure to obtain desired product which was directly used for next step.

Steps 2 and 3 were performed as described for synthesis of Compounds 579 and 533

Compound 1329: LCMS- 484.2(M+H)⁺ @ 220 nm =97.66% ¹H NMR (400 MHz, DMSO-d6) δ 8.16 (d, J = 8.2 Hz, 1H), 8.06 (d, J = 8.2 Hz, 1H), 7.84 (d, J = 8.2 Hz, 1H), 7.77-7.69 (m, 2H), 7.69-7.54 (m, 3H), 7.50 (t, J = 7.6 Hz, 1H), 7.37 (dd, J = 8.3, 1.5 Hz, 1H), 7.19 (dt, J = 10.6, 8.5 Hz, 1H), 6.97-6.79 (m, 2H), 5.35 (s, 2H), 3.59 (br.s., 8H).

Compound 1330: LCMS- 484.1(M+H)⁺ @ 220 nm =99.87% ¹H NMR (400 MHz, DMSO-d6) δ 8.16 (d, J = 8.2 Hz, 1H), 8.06 (d, J = 8.2 Hz, 1H), 7.84 (d, J = 1.4 Hz, 1H), 7.77-7.55 (m, 5H), 7.50 (t, J = 7.6 Hz, 1H), 7.39 (dd, J = 8.2, 1.6 Hz, 1H), 7.19 (dt, J = 10.7, 8.4 Hz, 2H), 6.95-6.82 (m, 1H), 5.33 (s, 2H), 3.64 (s, 8H).

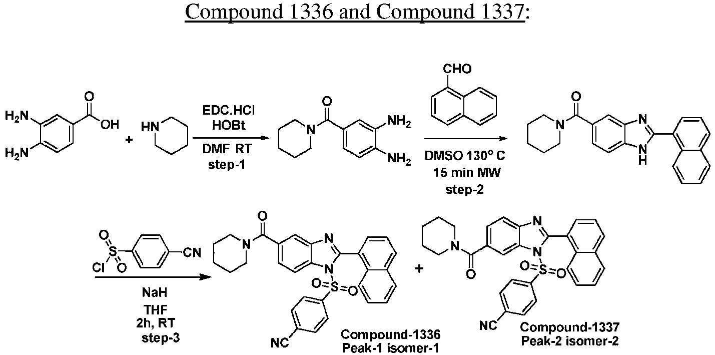

### Steps 1 and 2 were performed as described for synthesis of Compound 1329

Compound 1336: LCMS- 521.1(M+H)⁺ @ 220 nm =99.82% ¹H NMR-(400 MHz, DMSO-d6) δ 8.24 (*d, J =* 8.5 Hz, 1H), 8.18 (d*t, J =* 7.2, 3.7 Hz, 1H), 8.03 (*d,* J =8.3 Hz, 1H), 7.85 (*d, J =* 1.6 Hz, 1H), 7.82-7.75 (*m,* 2H), 7.66 (q, *J* = 3.4, 2.8 Hz, 2H), 7.62-7.50 (m,4H), 7.34 (*t, J* = 7.6 Hz, 1H), 7.17 (*d, J* = 8.5 Hz, 1H), 3.59 (br.s., 4H), 1.68-1.5(m, 6H).

Compound 1337: LCMS- 521.2(M+H)⁺ @ 220 nm =99.39% ¹H NMR-400 MHz, DMSO-*d*6) δ 8.27-8.15 (*m,* 1H), 8.13 (s, 1H), 8.03 (*d, J* = 8.3 Hz, 1H), 7.92 (*d, J =* 8.2 Hz, 1H), 7.77 (*d, J =* 7.9 Hz, 2H), 7.71-7.62 (*m,* 2H), 7.54 (*t, J* = 7.6 Hz, 4H), 7.34 (*t, J =* 7.7 Hz, 1H), 7.18 (*d, J =* 8.4 Hz, 1H), 3.63 (br.s, 4H), 1.67 (*d, J* = 7.7 Hz, 2H), 1.62-1.50 (*m,* 4H).

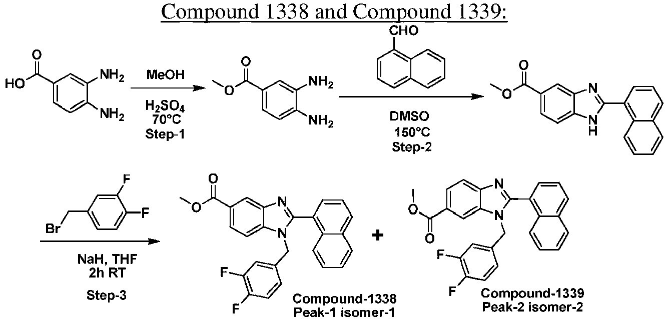

### Steps 1 and 2 were performed as described for synthesis of Compound 848

### Step 3 was performed as described for synthesis of Compound 533

Compound 1338: LCMS-429.0 (M+H)⁺ @ 220 nm =98.3% ¹H NMR (400 MHz, DMSO-d6) δ ppm 8.26 (*s,* 1 H), 8.16 (*d,* J=8.33 Hz, 1 H), 8.06 (*d,* J=8.33 Hz, 1 H), 7.96 *(d,* J=8.77 Hz, 1 H), 7.90 *(d,* J=8.33 Hz, 1 H), 7.74 *(d,* J=7.02 Hz, 1 H), 7.69 - 7.55 *(m, 3* H), 7.53 - 7.47 *(m,* 1 H), 7.24 - 7.14 *(m,* 1 H), 6.91 - 6.84 *(m,* 1 H), 6.65 *(br. s.,* 1 H), 5.43 *(s, 2* H), 3.88 *(s,* 3 H).

Compound 1339: LCMS-429.0 (M+H)⁺ @ 220 nm =97.5% ¹H NMR (400 MHz, DMSO-d6) δ 8.39 (d, J = 1.7 Hz, 1H), 8.17 (d, J = 8.3 Hz, 1H), 8.06 (d, J = 8.5 Hz, 1H), 7.97 (dd, J = 8.5, 1.6 Hz, 1H), 7.75 (dd, J = 7.8, 3.7 Hz, 2H), 7.66 (t, J = 7.6 Hz, 1H), 7.59 (dt, J = 7.6, 3.0 Hz, 2H), 7.49 (dd, J = 8.7, 6.4 Hz, 1H), 7.18 (dt, J = 10.7, 8.4 Hz, 1H), 6.87 (ddd, J = 11.2, 8.0, 2.2 Hz, 1H), 6.63 (dt, J = 7.2, 3.0 Hz, 1H), 5.36 (s, 2H), 3.90 (s, 3H).

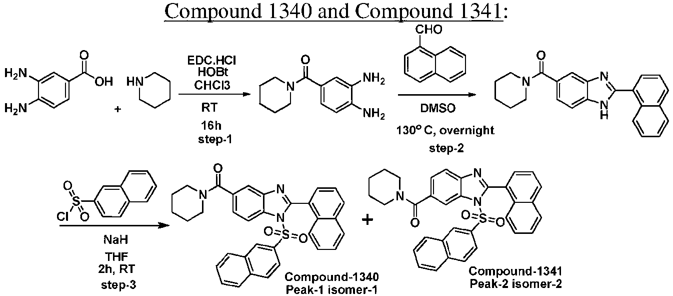

### Steps 1-3 were performed as described for synthesis of Compound 1329

Compound 1340: LCMS-546.2 (M+H)⁺ @ 220nm= 98.5% ¹H NMR (400 MHz, DMSO-d6) δ 8.18 (*d,* J = 7.4 Hz, 2H), 8.12 (*d,* J = 2.1 Hz, 1H), 7.98-7.91 (*m,* 3H), 7.87 *(dd, J* = 8.4, 6.3 Hz, 2H), 7.76-7.69 (m, 2H), 7.65 (q, J = 7.7 Hz, 2H), 7.48 (*dd,* J = 8.1, 1.5 Hz, 1H), 7.42 (*dd,* J = 8.8, 2.1 Hz, 1H), 7.36 (ddd, J = 8.1, 6.4, 1.6 Hz, 1H), 7.21-7.12 (m, 2H), 3.66 (s, 4H), 1.63 (s, 6H).

Compound 1341: LCMS-546.2 (M+H)⁺ @ 220nm= 99.3% ¹H NMR (400 MHz, DMSO-*d*₆) δ 8.31 (d, J = 8.5 Hz, 1H), 8.17 (dd, *J* = 7.0, 2.5 Hz, 1H), 8.03 (d, *J =* 2.1 Hz, 1H), 7.97-7.89 (m, 3H), 7.88-7.78 (m, 2H), 7.72 (t, *J =* 7.5 Hz, 2H), 7.68-7.59 (m, 2H), 7.58 (dd, *J* = 8.4*,* 1.6 Hz, 1H), 7.46 (dd, *J =* 8.8, 2.1 Hz, 1H), 7.33 (ddd, J = 8.2, 5.6, 2.4 Hz, 1H), 7.11 (d, *J =* 5.4 Hz, 2H), 3.61 (s, 4H), 1.58 (d, *J* = 38.2 Hz, 6H).

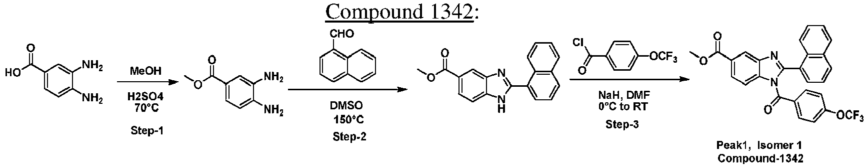

Steps 1 and 2 were performed as described for synthesis of Compounds 848 and 535

Compound 1342: ¹H NMR: ¹H NMR (400 MHz, DMSO-*d*₆) δ ppm 8.46 (s, 1 H), 8.11 (*d, J*=8.33 Hz, 1 H), 8.04-7.7 8 (*m,* 4 H), 7.66 (*d, J*=6.14 Hz, 1 H), 7.56 (*d, J=*8.77 Hz, 4 H), 7.39 (*t, J*=7.67 Hz, 1 H), 6.91 (*d, J=*8.77 Hz, 2 H), 3.94 (s, 3 H).

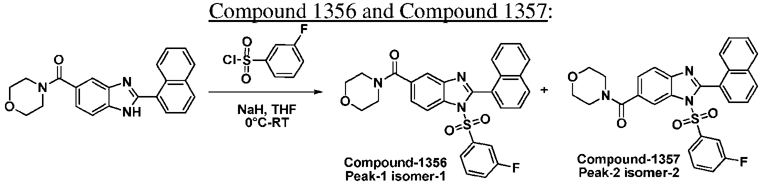

### Step 1 was performed as described for synthesis of Compound 132

Compound 1356: LCMS- (M+H)⁺ 516.5, 98.45 % @220 nm 98.71 % @254 nm ¹H NMR: 8.23-8.16 (*m,* 2H), 8.04 (*d, J =* 8.2 Hz, 1H), 7.92 (*d, J* = 8.2 Hz, 1H),7.73-7.62 (*m,* 2H), 7.55 (*t, J =* 7.5 Hz, 2H), 7.50-7.38 (*m,* 2H), 7.36 (*dd, J =* 8.9, 6.5 Hz, 2H), 7.27 -7.16 *(m,* 2H), 3.91-3.37 (*m,* 8H).
Compound 1357: LCMS- (M+H)⁺ 516.5, 94.45 % @220nm 95.47 % @254 nm

NMR: 8.26 (*d, J =* 8.5 Hz, 1H), 8.19 (*dt, J* = 7.4, 3.6 Hz, 1H), 8.04 (*d, J* =8.4 Hz, 1H), 7.91 (*d, J =* 1.4 Hz, 1H), 7.73-7.50 (*m,* 5H), 7.45 (*t, J =* 6.3 Hz, 2H), 7.40-7.30 (*m,* 2H),7.24-7.11 (*m,* 1H), 3.80-3.40 (*m,* 8H).

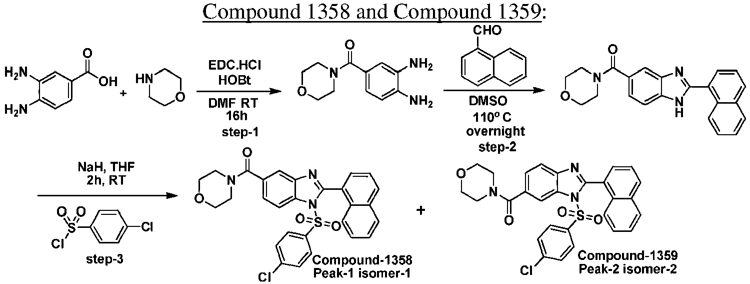

### Steps 1-3 were performed as described for synthesis of Compound 1329

Compound 1358: LCMS-532.5(M)⁺ @ 254 nm = 96.04% @ 220 nm =96.73% ¹H NMR-(400 MHz, DMSO-d6) δ 8.23-8.14 (m, 2H), 8.03 (d, J = 8.2 Hz, 1H), 7.92 (d, J = 8.2 Hz, 1H),7.71-7.62 (m, 2H), 7.60-7.50 (m, 2H), 7.44-7.30 (m, 5H), 7.18 (d, J = 8.5 Hz, 1H), 3.66 (s, 8H).

Compound 1359: LCMS-532.4 (M)⁺ @ 254 nm =98.52% @ 220 nm =97.41% ¹H NMR-(400 MHz, DMSO-d6) δ 8.24 (d, J = 8.5 Hz, 1H), 8.19 (dt, J = 7.0, 3.6 Hz, 1H), 8.03 (d, J =8.2 Hz, 1H), 7.91 (d, J = 1.5 Hz, 1H), 7.71-7.50 (m, 4H), 7.46-7.30 (m, 5H), 7.17 (d, J = 8.4 Hz, 1H), 3.55 (d, J = 65.4 Hz, 8H).

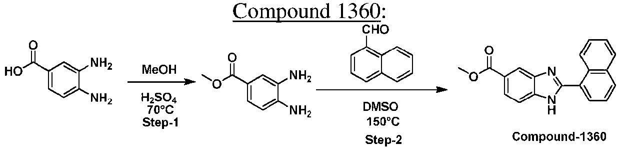

### Steps 1 and 2 were performed as described for synthesis of Compound 848

Compound 1360: LCMS- 303.2(M+H)⁺ 96.41 @220 NM 97.62 @ 254nm ¹H NMR (400 MHz, DMSO-d6) δ 13.29 (s, 1H), 9.02 (d, J = 8.0 Hz, 1H), 8.37 (s, 1H), 8.21-8.08 (m, 1H), 8.09-7.97 (m, 2H), 7.92 (dd, J = 8.2, 1.6 Hz, 1H), 7.86 (d, J = 2.6 Hz, 1H), 7.74-7.57 (m, 3H), 3.88 (s, 3H).

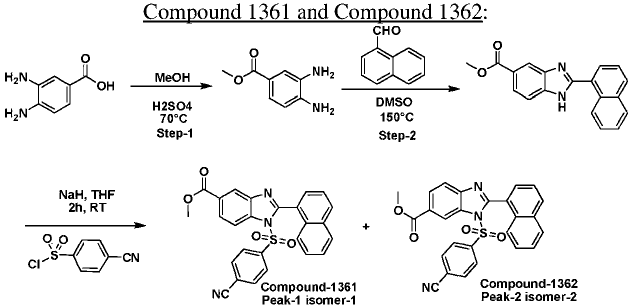

### Steps 1 and 3 were performed as described for synthesis of Compounds 848 and 631

Compound 1361: LCMS- 468.4 (M+H)⁺ @ 254 nm =97.62% @ 220 nm =96.41% ¹H NMR (400 MHz, Chloroform-d) δ 8.98 (d, J = 1.6 Hz, 1H), 8.22 (dd, J = 8.4, 1.6 Hz, 1H), 8.08 (d, J = 8.1 Hz, 1H), 7.90 (t, J = 8.3 Hz, 2H), 7.75-7.66 (m, 1H), 7.63 (t, J = 7.7 Hz, 1H), 7.50 (t, J = 7.5 Hz, 1H), 7.34-7.27 (m, 3H), 7.26 (br.s, 1H), 7.22 (dd, J = 8.6, 6.9 Hz, 1H), 7.06 (d, J = 8.5 Hz, 1H), 4.04 (s, 3H).

Compound 1362: LCMS-468.4 (M+H)⁺ @ 254 nm =96.54% @ 220 nm =94.2% 1H NMR (400 MHz, Chloroform-d) δ 8.53 (d, J = 1.6 Hz, 1H), 8.34 (d, J = 8.7 Hz, 1H), 8.25 (dd, J = 8.7, 1.6 Hz, 1H), 8.06 (t, J = 10.0 Hz, 2H), 7.90 (d, J = 8.4 Hz, 2H), 7.69 (d, J = 7.2 Hz, 1H), 7.62 (t, J = 7.6 Hz, 1H), 7.49 (dd, J = 8.4, 6.7 Hz, 1H), 7.26 (s, 2H), 7.24-7.17 (m, 1H), 7.04 (d, J = 8.4 Hz, 1H), 4.00 (s, 3H).

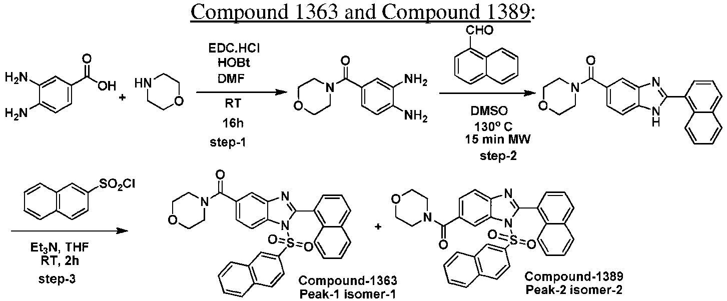

### Steps 1-3 were performed as described for synthesis of Compound 1329

Compound 1363: LCMS-548.5 (M+H)⁺ @ 254 nm =97.17% @ 220 nm =93.39% ¹H NMR (400 MHz, DMSO-d6) δ 8.24 (s, 1H), 8.17 (d, J = 8.1 Hz, 1H), 8.12 (d, J = 2.2 Hz, 1H), 8.00-7.76 (m, 5H), 7.68 (dq, J = 22.6, 7.5 Hz, 4H), 7.52 (d, J = 8.2 Hz, 1H), 7.43 (dd, J = 8.6, 2.2 Hz, 1H), 7.35 (ddd, J = 8.2, 6.1, 1.8 Hz, 1H), 7.24-7.08 (m, 2H), 3.80-3.45 (m, 8H).

Compound 1389: LCMS- 548.5 (M+H)⁺ @ 254 nm =99.42% @ 220 nm =99.49% ¹H NMR (400 MHz, DMSO-d6) δ 8.32 (d, J = 8.5 Hz, 1H), 8.17 (dt, J = 7.3, 3.6 Hz, 1H), 8.04 (d, J = 2.1 Hz, 1H), 7.94 (dd, J = 8.9, 2.8 Hz, 3H), 7.88 (d, J = 1.6 Hz, 1H), 7.84 (d, J = 8.2 Hz, 1H), 7.75-7.68 (m, 1H), 7.69-7.54 (m, 4H), 7.46 (dd, J = 8.8, 2.1 Hz, 1H), 7.33 (dq, J = 8.9, 4.9, 4.5 Hz, 1H), 7.11 (d, J = 3.9 Hz, 2H), 3.78-3.37 (m, 8H).

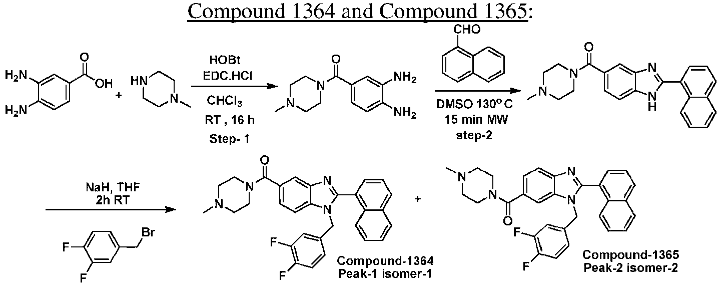

### Steps 1-3 were performed as described for synthesis of Compound 1329

Compound 1364: LCMS-497.5 (M+H)⁺ UPLC @ 254 nm = 96.82 %, @ 220 nm = 96.29%. ¹H NMR (400 MHz, Chloroform-d) δ 8.04 (dd, J=7.24, 1.97 Hz, 1 H) 7.90 - 7.98 (m, 2 H) 7.67 (d, J=7.89 Hz, 1 H) 7.53 - 7.60 (m, 3 H) 7.46 - 7.52 (m, 2 H) 7.39 - 7.45 (m, 1 H) 6.94 - 7.03 (m, 1 H) 6.60 - 6.69 (m, 2 H) 5.17 (s, 2 H), 4.01- 3.67(br. s., 4 H) 2.73 (br. s., 3 H) 2.51-2.48(br. s., 4 H)

Compound 1365: LCMS-497.5 (M+H)⁺ UPLC @ 254 nm = 96.66 %, @ 220 nm = 97.12 % ¹H NMR (400 MHz, Chloroform-d) δ 8.04 (dt, J = 7.2, 3.7 Hz, 1H), 7.96 (d, J = 7.4 Hz, 2H), 7.67 (d, J = 8.3 Hz, 1H), 7.61-7.51 (m, 3H), 7.48 (ddd, J = 8.2, 6.6, 1.3 Hz, 1H), 7.43 (dd, J = 8.4, 1.6 Hz, 1H), 7.35 (d, J = 8.3 Hz, 1H), 6.98 (dt, J = 9.8, 8.2 Hz, 1H), 6.75-6.54 (m, 2H), 5.17 (s, 2H), 3.95 (s, 4H), 2.83 (s, 4H), 2.62 (s, 3H).

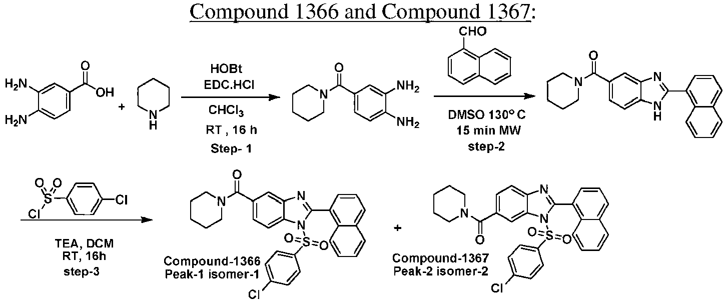

### Steps 1-3 were performed as described for synthesis of Compound 1329

Compound 1366: LCMS - 530.5 (M)⁺ UPLC @ 254 nm = 98.61 %, @ 220 nm = 98.51% ¹H NMR (400 MHz, DMSO-d6) δ 8.18 (dt, J = 7.6, 3.7 Hz, 1H), 8.11 (s, 1H), 8.03 (d, J = 8.3 Hz, 1H), 7.91 (d, J = 8.2 Hz, 1H), 7.68 (q, J = 4.5 Hz, 2H), 7.54 (q, J = 8.5, 8.0 Hz, 2H), 7.39 (s, 4H), 7.33 (d, J = 7.4 Hz, 1H), 7.19 (d, J = 8.5 Hz, 1H), 3.66 (s, 4H), 1.66 (s, 3H), 1.60-1.43 (m, 3H).

Compound 1367: LCMS-530.5 (M)⁺ UPLC @ 254 nm = 99.40 %, @ 220 nm = 98.80 %. ¹H NMR (400 MHz, DMSO-d6) δ 8.26-8.15 (m, 2H), 8.03 (d, J = 8.2 Hz, 1H), 7.84 (d, J = 1.5 Hz, 1H), 7.66 (q, J = 3.6, 3.1 Hz, 2H), 7.61-7.50 (m, 2H), 7.41 (s, 4H), 7.34 (t, J = 7.6 Hz, 1H), 7.18 (d, J = 8.4 Hz, 1H), 3.62 (br. s., 4H), 1.64 (q, J = 5.3 Hz, 3H), 1.54 (s, 3H).

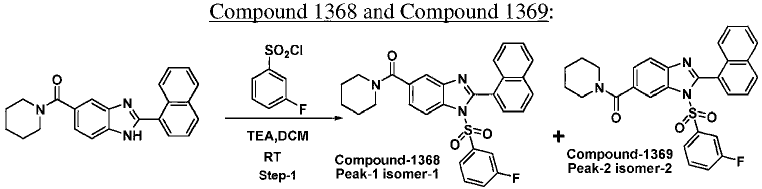

### Step 1 was performed as described for synthesis of Compound 1329

Compound 1368 - LCMS: 514.5 (M+H)⁺ 98.48 % @220 nm 99.43 % @254 nm ¹H NMR (400 MHz, DMSO-d6) δ 8.20 (d, J = 7.8 Hz, 1H), 8.16-8.11 (m, 1H), 8.04 (d, J = 8.2 Hz, 1H), 7.91 (d, J = 8.2 Hz, 1H), 7.74-7.63 (m, 2H), 7.59-7.49 (m, 2H), 7.44 (t, J = 6.0 Hz, 2H), 7.35 (q, J = 6.5, 5.1 Hz, 2H), 7.24 (d, J = 8.5 Hz, 1H), 7.18-7.12 (m, 1H), 3.66 (s, 4H), 1.59 (d, J = 51.8 Hz, 6H).

Compound 1369:-1369-LCMS:514.5 (M+H)⁺ 94.08 % @220nm 95.90 % @254 nm ¹H NMR (400 MHz, DMSO-d6) δ 8.25 (d, J = 8.5 Hz, 1H), 8.19 (dt, J = 7.2, 3.6 Hz, 1H), 8.04 (d, J = 8.4 Hz, 1H), 7.84 (d, J = 1.6 Hz, 1H), 7.67 (q, J = 4.0 Hz, 2H), 7.61-7.50 (m, 2H), 7.50-7.40 (m, 2H), 7.40-7.29 (m, 2H), 7.21 (d, J = 8.5 Hz, 1H), 7.15 (dd, J = 8.1, 2.5 Hz, 1H), 3.64 (d, J = 17.0 Hz, 4H), 1.57 (dd, J = 41.2, 10.9 Hz, 6H).

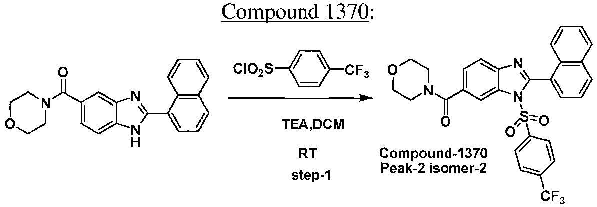

### Step 1 was performed as described for synthesis of Compound 1329

Compound 1370 -LCMS: 566.4 (M+H)⁺ 95.17 % @220 nm 97.06 % @254 nm ¹H NMR (400 MHz, DMSO-d6) δ 8.27 (d, J = 8.5 Hz, 1H), 8.19 (dd, J = 7.4, 2.1 Hz, 1H), 8.00 (d, J = 8.3 Hz, 1H), 7.92 (d, J = 1.5 Hz, 1H), 7.73-7.67 (m, 2H), 7.68-7.62 (m, 3H), 7.59 (d, J = 8.3 Hz, 2H), 7.50 (t, J = 7.5 Hz, 1H), 7.25 (t, J = 7.7 Hz, 1H), 7.09 (d, J = 8.5 Hz, 1H), 3.81 (br.s., 8H)

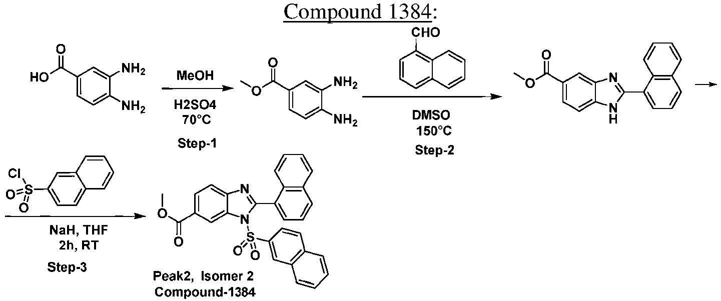

### Step 1 was performed as described for synthesis of Compounds 848 and 631

Compound 1384: LCMS: 493.4 (M+H)⁺ 88.82 % @220 nm 96.31 % @254 nm ¹H NMR (400 MHz, DMSO-d6) δ 8.41 (d, J = 8.7 Hz, 1H), 8.36 (s, 1H), 8.18 (dd, J = 8.3, 4.2 Hz, 2H), 8.03 (s, 1H), 7.99-7.88 (m, 3H), 7.82 (d, J = 8.4 Hz, 1H), 7.67 (dp, J = 22.0, 7.2 Hz, 4H), 7.44 (d, J = 8.7 Hz, 1H), 7.32 (dt, J = 8.3, 3.9 Hz, 1H), 7.09 (d, J = 4.0 Hz, 2H), 3.91 (s, 3H).

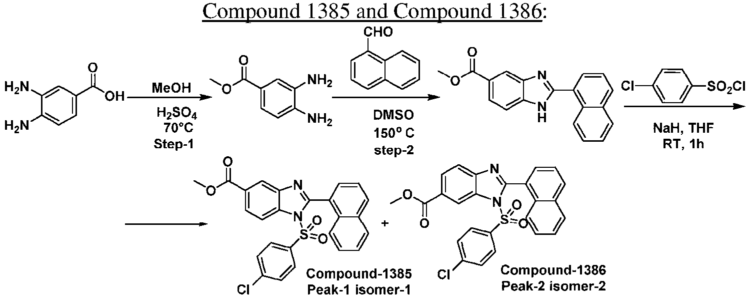

### Steps 1-3 were performed as described for synthesis of Compounds 848 and 527

Compound 1385: LCMS-477.1(M+H)⁺ @ 220 nm =99.05% ¹H NMR (400 MHz, DMSO-*d*₆) δ 8.79 (d, J = 1.6 Hz, 1H), 8.19 (q, J = 4.1, 3.3 Hz, 1H), 8.14 (dd, J = 8.5, 1.7 Hz, 1H), 8.01 (dd, J = 10.1, 8.3 Hz, 2H), 7.66 (d, J = 4.8 Hz, 2H), 7.53 (t, J = 7.5 Hz, 1H), 7.39-7.23 (m, 5H), 7.09 (d, J = 8.4 Hz, 1H), 3.97 (s, 3H).

Compound 1386: LCMS-477.1(M+H)⁺ @ 220 nm =99.5% ¹H NMR (400 MHz, DMSO-*d*₆) δ 8.39 (d, J = 1.7 Hz, 1H), 8.33 (d, J = 8.7 Hz, 1H), 8.24-8.14 (m, 2H), 8.03 (d, J = 8.2 Hz, 1H), 7.68 (q, J = 4.1 Hz, 2H), 7.55 (t, J = 7.5 Hz, 1H), 7.39 (d, J = 1.9 Hz, 4H), 7.33 (d, J = 7.4 Hz, 1H), 7.16 (d, J = 8.4 Hz, 1H), 3.93 (s, 3H).

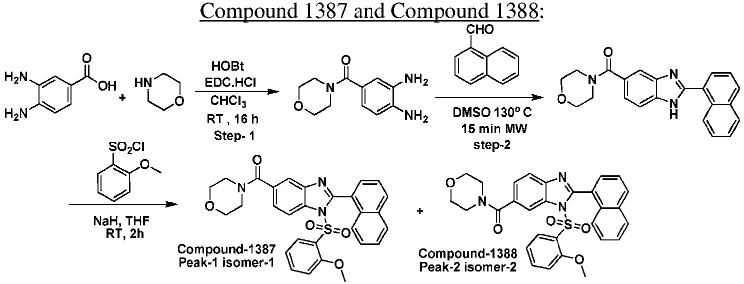

### Steps 1-3 were performed as described for synthesis of Compound 1329

Compound 1387: LCMS-527.59 (M+H)⁺ @ 200 nm = 92.0% @ 254nm =95.1% ¹H NMR (400 MHz, Chloroform-d) δ 8.36 (d, J = 1.4 Hz, 1H), 7.92 (dd, J = 11.0, 8.0 Hz, 2H), 7.77 (t, J = 7.0 Hz, 1H), 7.52 (d, J = 8.0 Hz, 1H), 7.49-7.31 (m, 4H), 7.17 (d, J = 4.0 Hz, 2H), 6.94 (dd, J = 8.0, 1.7 Hz, 1H), 6.69 (d, J = 8.4 Hz, 1H), 6.44 (q, J = 9.0, 8.4 Hz, 1H), 3.76 (s, 8H), 3.55 (s, 3H).

Compound 1388: LCMS-527.59 (M+H)⁺ @ 200 nm = 93.3% @ 254nm =95.99% ¹H NMR (400 MHz, Chloroform-d) δ 8.29 (d, J = 8.5 Hz, 1H), 7.96-7.87 (m, 2H), 7.77 (d, J = 8.2 Hz, 1H), 7.57 (dd, J = 8.5, 1.6 Hz, 1H), 7.51-7.43 (m, 2H), 7.36 (ddd, J = 8.1, 4.9, 3.0 Hz, 1H), 7.24-7.13 (m, 3H), 6.97 (dd, J = 8.0, 1.7 Hz, 1H), 6.65 (d, J = 8.4 Hz, 1H), 6.42 (t, J = 7.7 Hz, 1H), 3.76 (s, 8H), 3.51 (s, 3H).

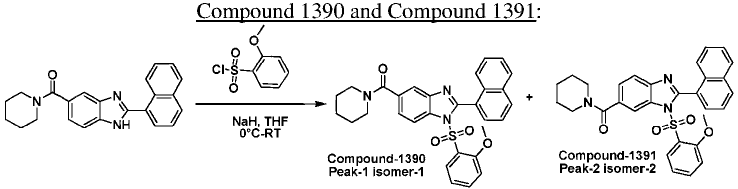

### Step 1 was performed as described for synthesis of Compounds 1329 and 631

Compound 1390: LCMS- (M+H)⁺ 526.5, 96.43 @220nm 97.46@ 254nm ¹H NMR (400 MHz, DMSO-d6) δ 8.07 (d, J = 8.3 Hz, 1H), 8.03 (d, J = 1.5 Hz, 1H), 7.91 (dd, J = 13.9, 8.2 Hz, 2H), 7.58-7.37 (m, 5H), 7.29 (t, J = 7.6 Hz, 1H), 7.17 (d, J = 8.5 Hz, 1H), 7.05-6.93 (m, 2H), 6.62 (t, J = 7.7 Hz, 1H), 3.62 (d, J = 23.6 Hz, 4H), 3.50 (s, 3H), 1.65 (s, 2H), 1.56 (s, 4H).

Compound 1391: LCMS- (M+H)⁺ 526.5, 91.79@220nm 94.64@ 254nm ¹H NMR (400 MHz, DMSO-d6) δ 8.07 (dd, J = 8.4, 5.1 Hz, 2H), 7.92 (d, J = 8.3 Hz, 1H), 7.83 (d, J = 1.6 Hz, 1H), 7.59-7.48 (m, 2H), 7.48-7.36 (m, 3H), 7.29 (t, J = 7.6 Hz, 1H), 7.18 (d, J = 8.4 Hz, 1H), 7.08-6.94 (m, 2H), 6.62 (t, J = 7.7 Hz, 1H), 3.65 (d, J = 21.4 Hz, 4H), 3.50 (s, 3H), 1.70-1.40 (m, 6H)

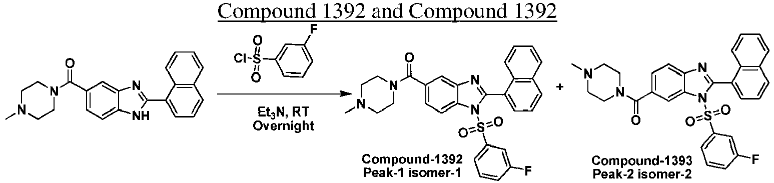

Step 1 was performed as described for synthesis of Compounds 1329 and 631

Compound 1392: LCMS- (M+H)⁺ 529.5, 95.94@220nm 97.3@ 254nm ¹H NMR (400 MHz, Chloroform-*d*) δ 8.37 (d, *J* = 1.5 Hz, 1H), 8.11-8.02 (m, 1H), 7.89 (dd, *J* = 8.7, 1.9 Hz, 2H), 7.66-7.57 (m, 2H), 7.54 (dd, *J =* 8.2, 1.5 Hz, 1H), 7.50-7.42 (m, 1H), 7.25-7.19 (m, 1H), 7.17-7.06 (m, 3H), 7.06-6.96 (m, 1H), 6.85 (dt, *J* = 7.8, 2.1 Hz, 1H), 3.90 (d, *J* = 55.7 Hz, 4H), 2.80 (s, 4H), 2.60 (s, 3H).

Compound 1393: LCMS: (M+H)⁺ 529.5, 94.96@220nm 95.9@ 254nm ¹H NMR (400 MHz, Chloroform-d) δ 8.33 (d, J = 8.5 Hz, 1H), 8.10-8.02 (m, 1H), 7.93-7.82 (m, 2H), 7.69-7.52 (m, 3H), 7.46 (ddd, J = 8.2, 6.6, 1.3 Hz, 1H), 7.25-7.20 (m, 1H), 7.17 (d, J = 8.5 Hz, 1H), 7.11 (t, J = 3.4 Hz, 2H), 7.07-6.98 (m, 1H), 6.96-6.85 (m, 1H), 3.79 (d, J = 69.0 Hz, 4H), 2.65 (s, 4H), 2.47 (s, 3H).

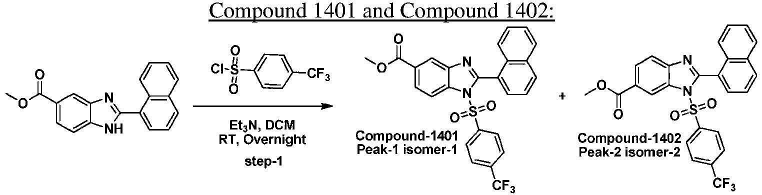

### Step 1 was performed as described for synthesis of Compound 527

Compound 1401: LCMS-511.4 (M+H)⁺ UPLC @ 254 nm = 99.48 %, @ 220 nm = 99.04%. ¹H NMR (400 MHz, DMSO-d6) δ 8.85-8.79 (m, 1H), 8.22-8.11 (m, 2H), 8.00 (dd, J = 10.4, 8.3 Hz, 2H), 7.73-7.63 (m, 2H), 7.57 (d, J = 8.4 Hz, 2H), 7.48 (d, J = 8.1 Hz, 3H), 7.20 (t, J = 7.7 Hz, 1H), 7.01 (d, J = 8.4 Hz, 1H), 3.97 (s, 3H).

Compound 1402: LCMS-511.4 (M+H)⁺ UPLC @ 254 nm = 99.54 %, @ 220 nm = 98.88 %. ¹H NMR (400 MHz, DMSO-d6) δ 8.40 (d, J = 1.7 Hz, 1H), 8.36 (d, J = 8.7 Hz, 1H), 8.26-8.15 (m, 2H), 8.00 (d, J = 8.2 Hz, 1H), 7.77-7.67 (m, 2H), 7.66-7.55 (m, 4H), 7.49 (t, J = 7.5 Hz, 1H), 7.29-7.22 (m, 1H), 7.08 (d, J = 8.4 Hz, 1H), 3.93 (s, 3H).

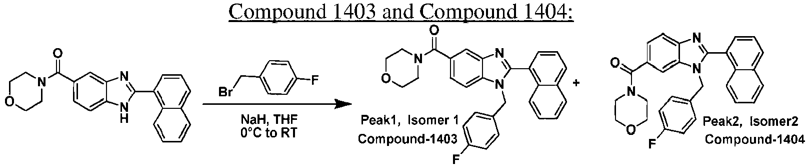

### Step 1 was performed as described for synthesis of Compounds 1329 and 533

Compound 1403: LCMS-466.4 (M+H)⁺ UPLC @ 254 nm = 97.61 %, @ 220 nm = 97.84%. ¹H NMR (400 MHz, DMSO-d6) δ 8.16 (d, J = 8.2 Hz, 1H), 8.06 (d, J = 8.2 Hz, 1H), 7.83 (d, J = 8.3 Hz, 1H), 7.72 (d, J = 6.9 Hz, 1H), 7.69-7.63 (m, 3H), 7.60 (t, J = 7.4 Hz, 1H), 7.52 (t, J = 7.5 Hz, 1H), 7.36 (d, J = 8.3 Hz, 1H), 6.98 (t, J = 8.7 Hz, 2H), 6.88 (dd, J = 8.5, 5.4 Hz, 2H), 5.35 (s, 2H), 3.51 (d, J = 53.3 Hz, 8H).

Compound 1404: LCMS-466.4 (M+H)⁺ UPLC @ 254 nm = 98.81 %, @ 220 nm = 99.01%. ¹H NMR (400 MHz, DMSO-d6) δ 8.16 (d, J = 8.1 Hz, 1H), 8.06 (d, J = 8.2 Hz, 1H), 7.83 (s, 1H), 7.71 (d, J = 6.9 Hz, 1H), 7.68-7.55 (m, 4H), 7.51 (t, J = 7.6 Hz, 1H), 7.41-7.34 (m, 1H), 6.97 (t, J = 8.9 Hz, 2H), 6.89 (dd, J = 8.5, 5.4 Hz, 2H), 5.33 (s, 2H), 3.59 (d, J = 30.9 Hz, 8H).

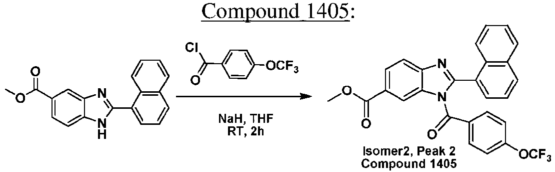

### Step 1 was performed as described for synthesis of Compound 535

Compound 1405: LCMS - 491.2 (M+H)⁺ 97.35% @ 220 nm ¹H NMR (400 MHz, DMSO-d6) δ 8.46 (d, J = 1.6 Hz, 1H), 8.11 (dd, J = 8.5, 1.7 Hz, 1H), 8.03 (d, J = 8.4 Hz, 1H), 7.95-7.89 (m, 1H), 7.85 (dd, J = 8.4, 5.6 Hz, 2H), 7.66 (d, J = 7.1 Hz, 1H), 7.55 (ddd, J = 10.1, 6.3, 3.3 Hz, 4H), 7.38 (t, J = 7.7 Hz, 1H), 6.89 (d, J = 8.3 Hz, 2H), 3.91 (s, 3H).

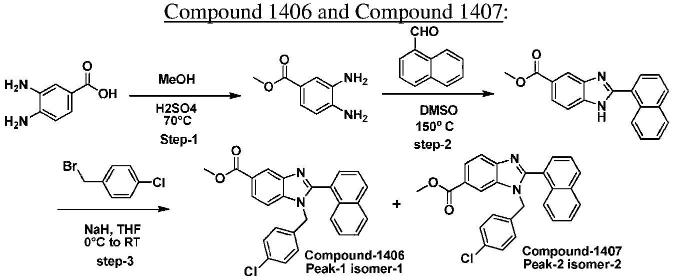

### Steps 1-3 were performed as described for synthesis of Compound 848

Compound 1406: LCMS-427.4 (M+H)⁺ UPLC @ 254 nm = 96.67 %, @ 220 nm = 98.01%. ¹H NMR (400 MHz, DMSO-d6) δ 8.19 (d, J = 1.6 Hz, 1H), 8.16 (d, J = 8.2 Hz, 1H), 8.06 (d, J = 8.1 Hz, 1H), 7.98-7.86 (m, 2H), 7.75-7.55 (m, 4H), 7.51 (t, J = 7.6 Hz, 1H), 7.22 (d, J = 8.1 Hz, 2H), 6.85 (d, J = 8.1 Hz, 2H), 5.43 (s, 2H), 3.87 (s, 3H).

Compound 1407: LCMS-427.4 (M+H)⁺ UPLC @ 254 nm = 98.05 %, @ 220 nm = 98.66%. ¹H NMR (400 MHz, DMSO-d6) δ 8.37 (d, J = 1.6 Hz, 1H), 8.14 (d, J = 8.2 Hz, 1H), 8.04 (d, J = 8.1 Hz, 1H), 7.93 (dd, J = 8.5, 1.6 Hz, 1H), 7.74-7.66 (m, 2H), 7.60 (dt, J = 18.9, 7.4 Hz, 3H), 7.49 (dd, J = 8.5, 6.7 Hz, 1H), 7.22-7.13 (m, 2H), 6.83 (d, J = 8.1 Hz, 2H), 5.35 (s, 2H), 3.88 (s, 3H).

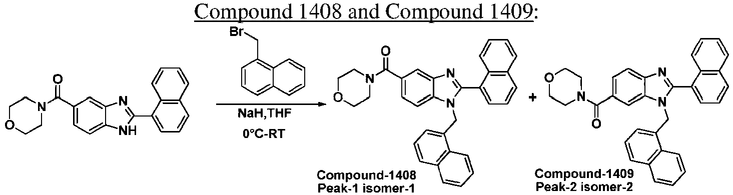

### Step 1 was performed as described for synthesis of Compounds 1329 and 533

Compound 1408: LCMS: 498.5 (M+H)⁺ 96.19 % @220 nm 96.40 % @254 nm ¹H NMR (400 MHz, DMSO-d6) δ 8.06 (d, J = 8.3 Hz, 1H), 8.03-7.97 (m, 1H), 7.88 (dd, J = 11.6, 8.1 Hz, 4H), 7.75 (d, J = 8.3 Hz, 1H), 7.68 (d, J = 7.1 Hz, 1H), 7.63-7.47 (m, 5H), 7.43 (ddd, J = 8.4, 6.8, 1.5 Hz, 1H), 7.37 (dd, J = 8.2, 1.6 Hz, 1H), 7.27 (t, J = 7.7 Hz, 1H), 6.65 (d, J = 7.2 Hz, 1H), 5.91 (s, 2H), 3.52 (br.s, 8H).

Compound 1409: LCMS: 498.5 (M+H)⁺ 99.22 % @220nm 99.09 % @254 nm ¹H NMR (400 MHz, DMSO-d6) δ 8.03 (dd, J = 21.5, 8.1 Hz, 2H), 7.92-7.82 (m, 4H), 7.71 (dd, J = 30.8, 7.7 Hz, 2H), 7.62-7.47 (m, 5H), 7.42 (t, J = 7.6 Hz, 1H), 7.33 (dd, J = 8.4, 1.5 Hz, 1H), 7.27 (t, J = 7.7 Hz, 1H), 6.68 (d, J = 7.1 Hz, 1H), 5.89 (s, 2H), 3.74-3.52 (m, 8H).

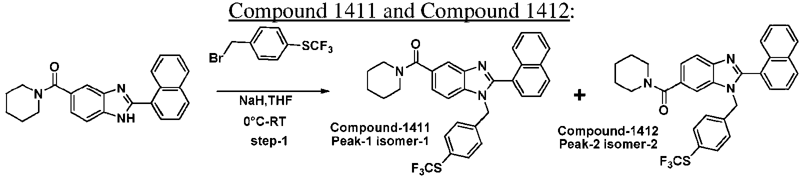

### Step 1 was performed as described for synthesis of Compounds 1329 and 533

Compound 1411: LCMS- 546.5 (M+H)⁺ 97.66 % @220nm 98.72 % @254 nm ¹H NMR (400 MHz, DMSO-d6) δ 8.14 (d, J = 8.2 Hz, 1H), 8.05 (d, J = 8.1 Hz, 1H), 7.84 (d, J = 8.3 Hz, 2H), 7.70 (t, J = 8.7 Hz, 2H), 7.64 (d, J = 7.7 Hz, 1H), 7.62-7.56 (m, 2H), 7.49 (dd, J = 7.7, 5.6 Hz, 2H), 7.32 (dd, J = 8.4, 1.5 Hz, 1H), 7.00 (d, J = 7.9 Hz, 2H), 5.45 (s, 2H), 3.56 (s, 4H), 1.49 (d, J = 81.5 Hz, 6H).

Compound 1412: LCMS- 546.5 (M+H)⁺ 97.54 % @220nm 97.39 % @254 nm ¹H NMR Compound 1412 (400 MHz, DMSO-d6) δ 8.14 (d, J = 8.1 Hz, 1H), 8.04 (d, J = 8.2 Hz, 1H), 7.79 (d, J = 1.5 Hz, 1H), 7.70 (d, J = 7.0 Hz, 1H), 7.65 (d, J = 2.7 Hz, 1H), 7.65-7.59 (m, 2H), 7.57 (d, J = 7.7 Hz, 1H), 7.48 (t, J = 7.6 Hz, 3H), 7.34 (dd, J = 8.4, 1.5 Hz, 1H), 7.00 (d, J = 8.0 Hz, 2H), 5.43 (s, 2H), 3.52 (d, J = 45.9 Hz, 4H), 1.72-1.42 (m, 6H)

**Table 1. Exemplary compounds of Formulae 1, IA, IB, IC, ID, and IE**

| No | Structure | No | Structure |
|---|---|---|---|
| 360 | 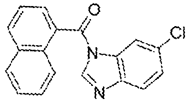 | 857 | 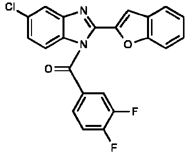 |
| 361 | 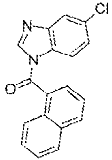 | 858 | 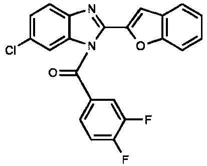 |
| 429 | 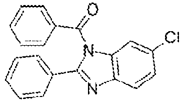 | 859 | 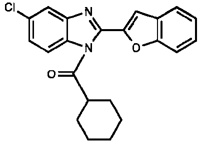 |
| 430 | 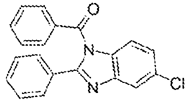 | 860 | 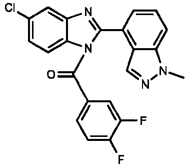 |
| 431 | 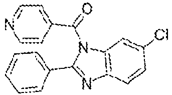 | 861 | 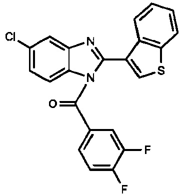 |
| 432 | 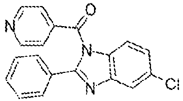 | 862 | 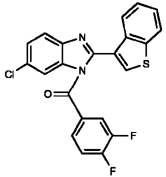 |
| 490 | 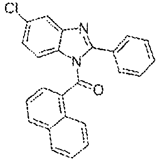 | 863 | 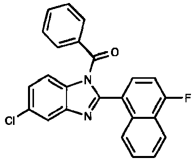 |
| 491 | 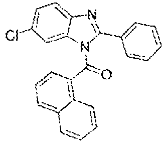 | 864 | 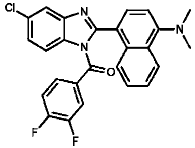 |
| 492 | 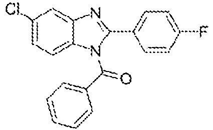 | 865 | 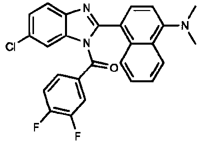 |
| 493 | 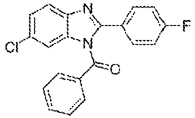 | 866 | 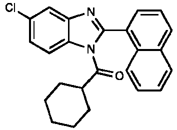 |
| 494 | 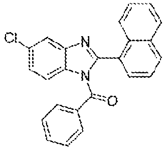 | 867 | 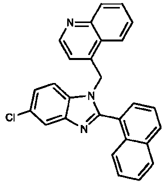 |
| 495 | 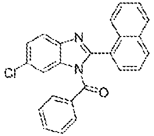 | 868 | 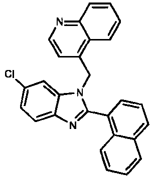 |
| 496 | 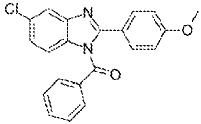 | 869 | 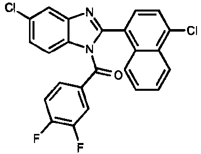 |
| 497 | 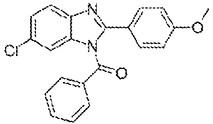 | 870 | 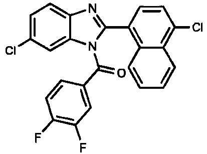 |
| 527 | 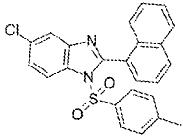 | 871 | 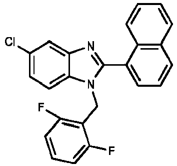 |
| 528 | 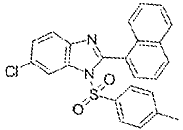 | 872 | 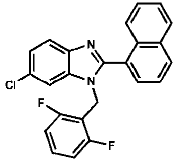 |
| 529 | 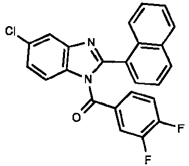 | 875 | 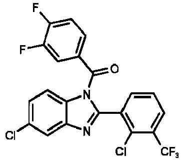 |
| 530 | 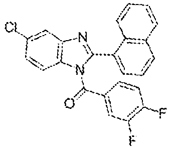 | 876 | 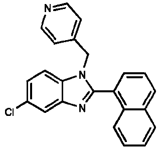 |
| 533 | 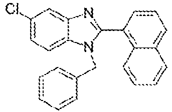 | 877 | 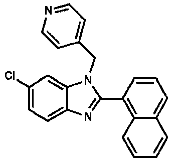 |
| 534 | 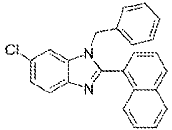 | 886 | 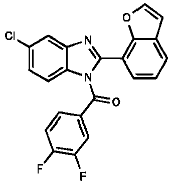 |
| 535 | 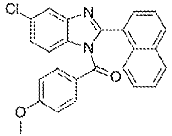 | 887 | 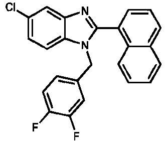 |
| 536 | 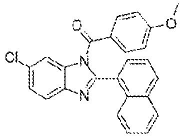 | 888 | 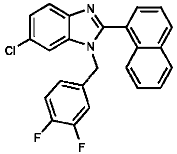 |
| 579 | 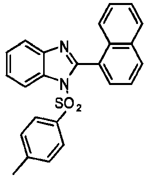 | 1328 | 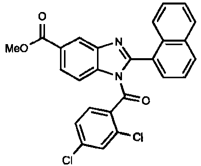 |
| 580 | 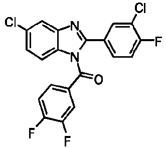 | 1329 | 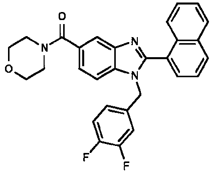 |
| 581 | 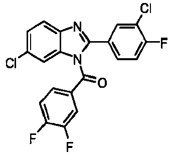 | 1330 | 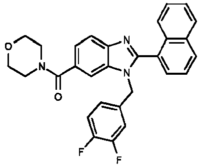 |
| 582 | 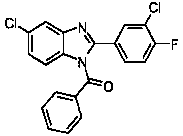 | 1336 | 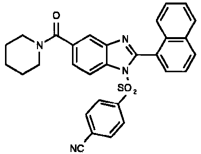 |
| 583 | 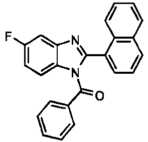 | 1337 | 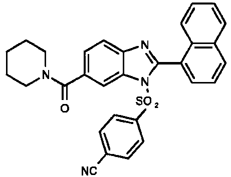 |
| 584 | 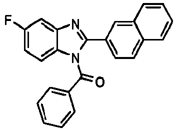 | 1338 | 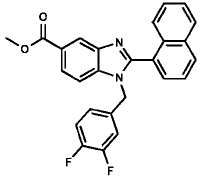 |
| 585 | 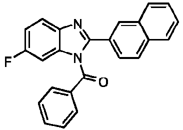 | 1339 | 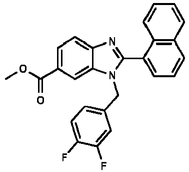 |
| 586 | 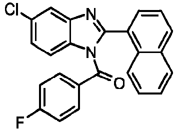 | 1340 | 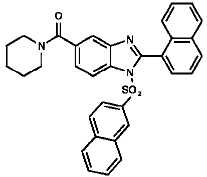 |
| 587 | 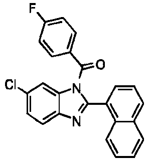 | 1341 | 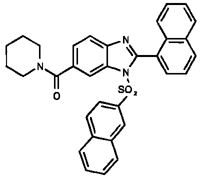 |
| 588 | 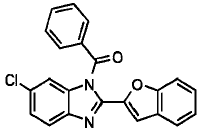 | 1342 | 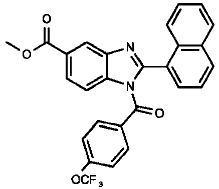 |
| 590 | 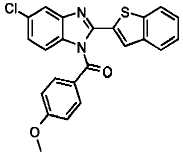 | 1356 | 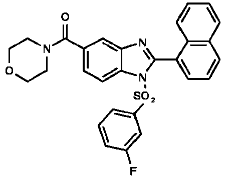 |
| 591 | 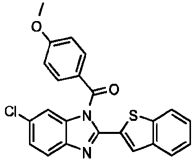 | 1357 | 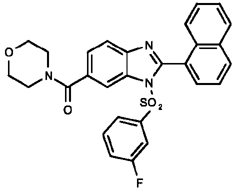 |
| 630 | 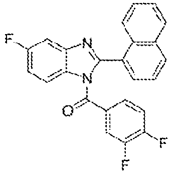 | 1358 | 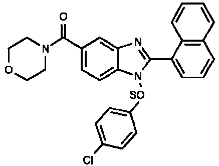 |
| 631 | 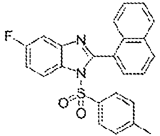 | 1359 | 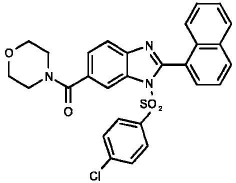 |
| 632 | 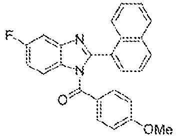 | 1360 | 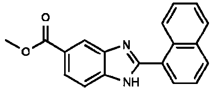 |
| 633 | 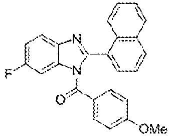 | 1361 | 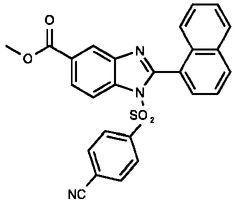 |
| 634 | 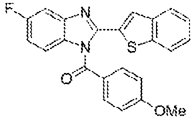 | 1362 | 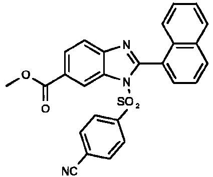 |
| 635 | 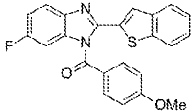 | 1363 | 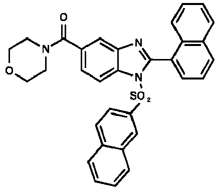 |
| 636 | 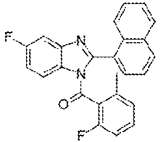 | 1364 | 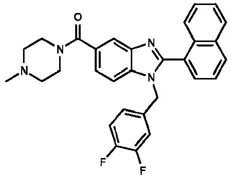 |
| 637 | 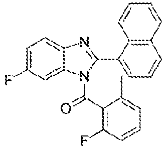 | 1365 | 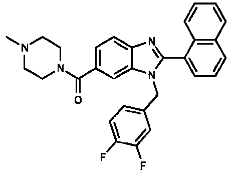 |
| 638 | 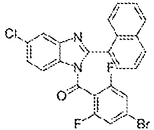 | 1366 | 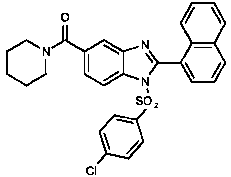 |
| 639 | 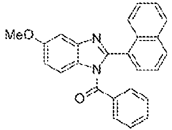 | 1367 | 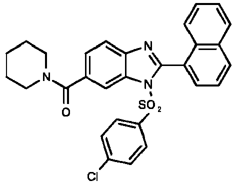 |
| 640 | 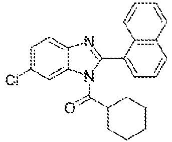 | 1368 | 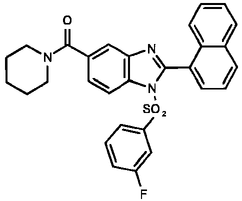 |
| 641 | 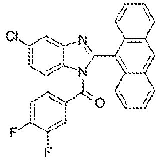 | 1369 | 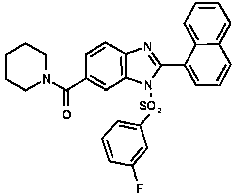 |
| 642 | 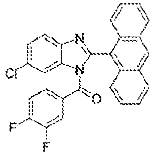 | 1370 | 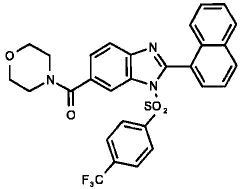 |
| 643 | 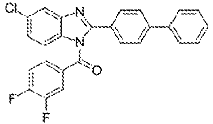 | 1384 | 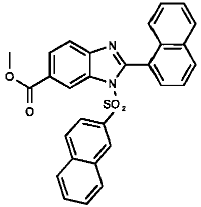 |
| 644 | 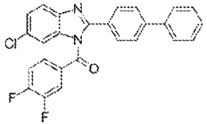 | 1385 | 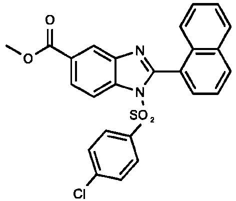 |
| 645 | 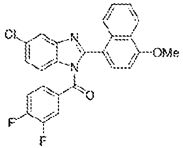 | 1386 | 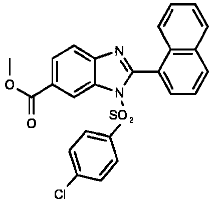 |
| 681 | 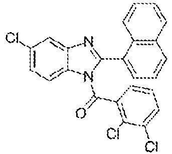 | 1387 | 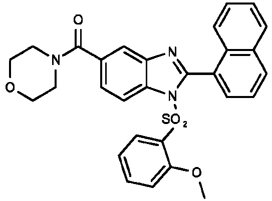 |
| 682 | 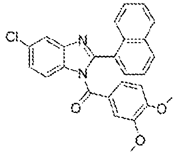 | 1388 | 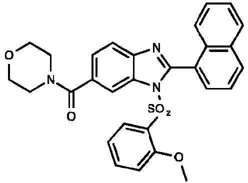 |
| 683 | 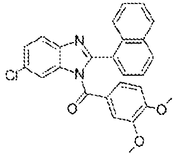 | 1389 | 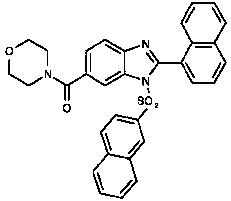 |
| 684 | 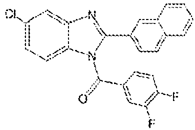 | 1390 | 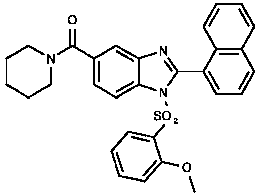 |
| 685 | 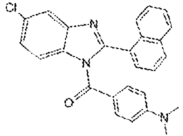 | 1391 | 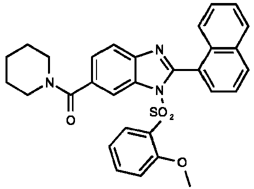 |
| 686 | 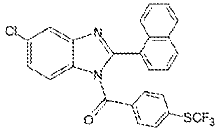 | 1392 | 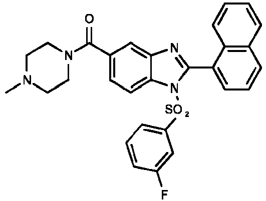 |
| 687 | 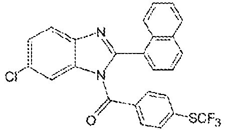 | 1393 | 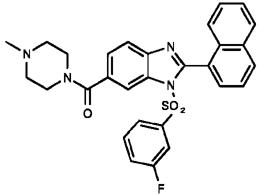 |
| 688 | 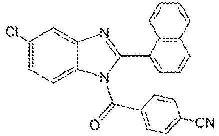 | 1401 | 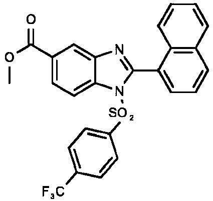 |
| 689 | 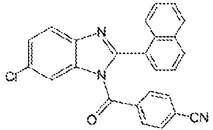 | 1402 | 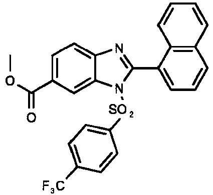 |
| 690 | 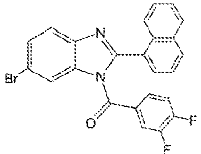 | 1403 | 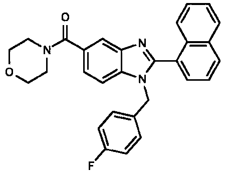 |
| 691 | 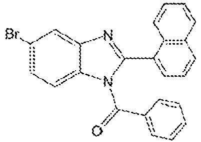 | 1404 | 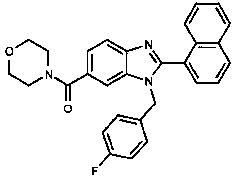 |
| 692 | 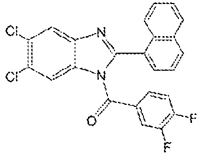 | 1405 | 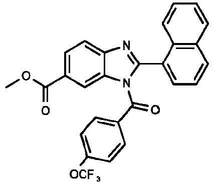 |
| 693 | 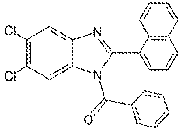 | 1406 | 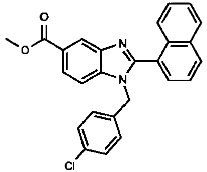 |
| 694 | 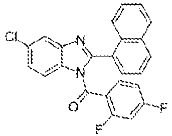 | 1407 | 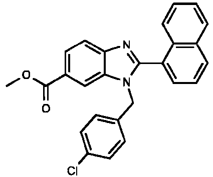 |
| 695 | 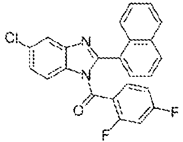 | 1408 | 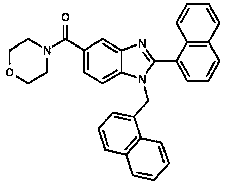 |
| 696 | 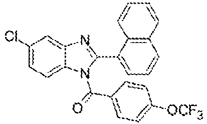 | 1409 | 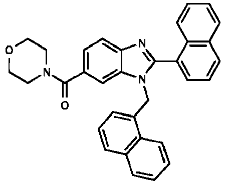 |
| 697 | 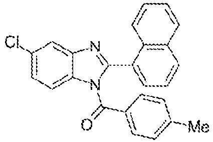 | 1411 | 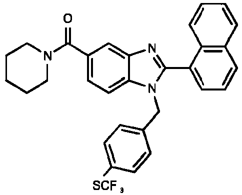 |
| 698 | 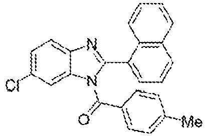 | 1412 | 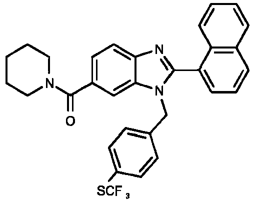 |
| 699 | 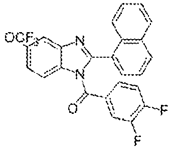 | 1460 | 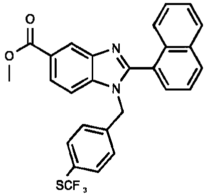 |
| 700 | 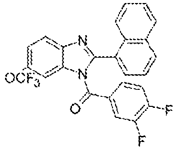 | 1461 | 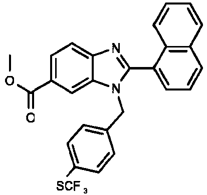 |
| 701 | 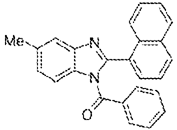 | 1462 | 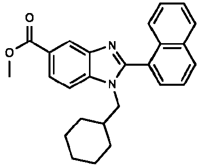 |
| 702 | 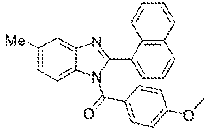 | 1463 | 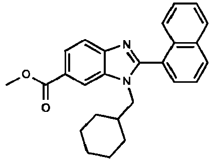 |
| 703 | 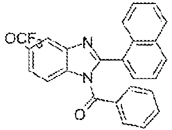 | 1464 | 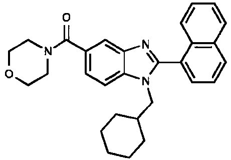 |
| 704 | 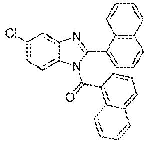 | 1465 | 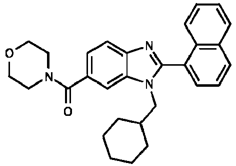 |
| 763 | 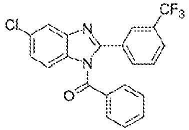 | 1466 | 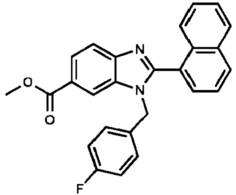 |
| 764 | 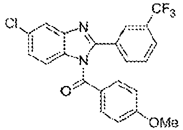 | 1467 | 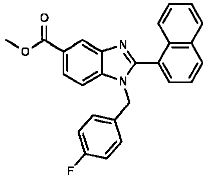 |
| 765 | 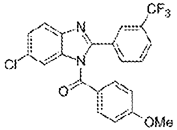 | 1468 | 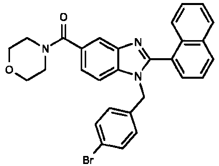 |
| 766 | 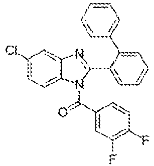 | 1469 | 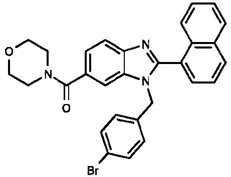 |
| 767 | 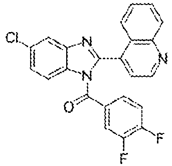 | 1470 | 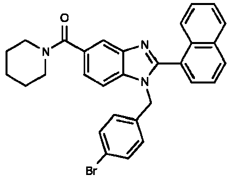 |
| 770 | 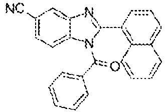 | 1471 | 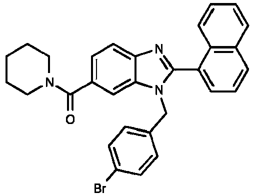 |
| 771 | 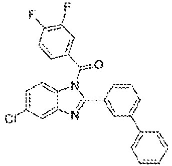 | 1472 | 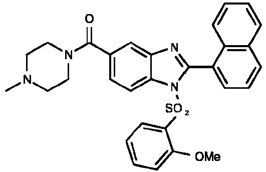 |
| 772 | 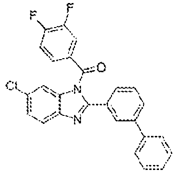 | 1473 | 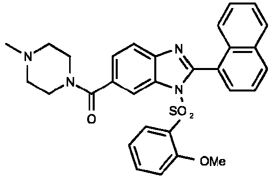 |
| 773 | 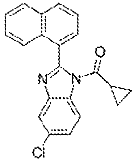 | 1474 | 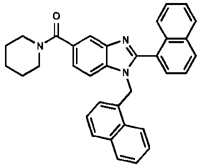 |
| 774 | 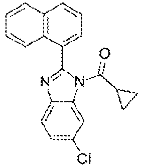 | 1475 | 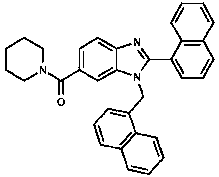 |
| 775 | 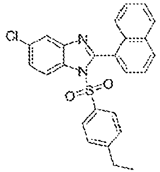 | 1476 | 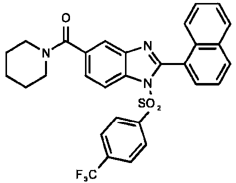 |
| 776 | 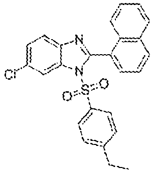 | 1477 | 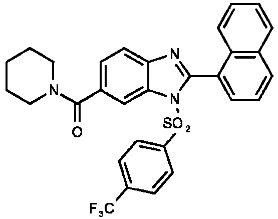 |
| 777 | 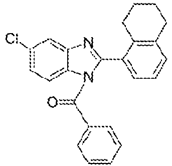 | 1478 | 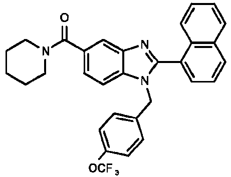 |
| 778 | 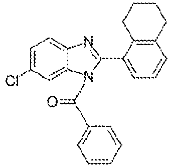 | 1479 | 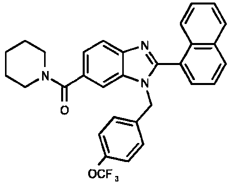 |
| 779 | 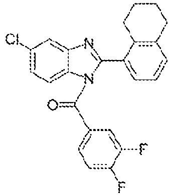 | 1481 | 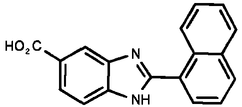 |
| 780 | 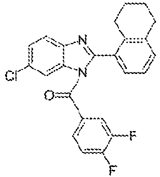 | 1490 | 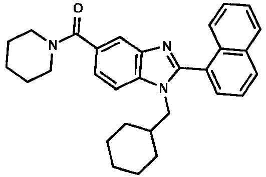 |
| 781 | 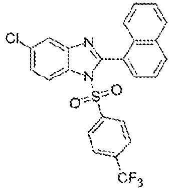 | 1491 | 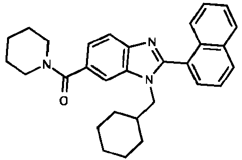 |
| 782 | 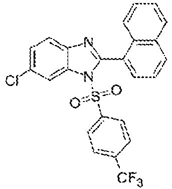 | 1492 | 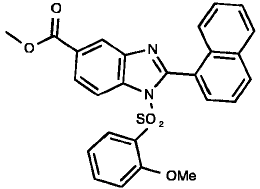 |
| 783 | 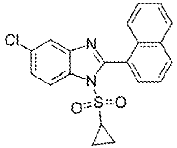 | 1493 | 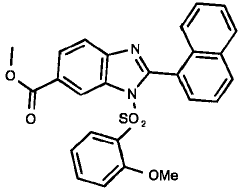 |
| 784 | 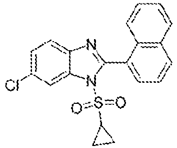 | 1494 | 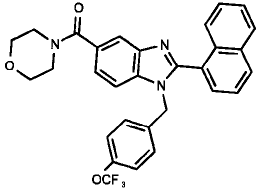 |
| 785 | 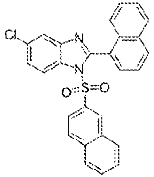 | 1495 | 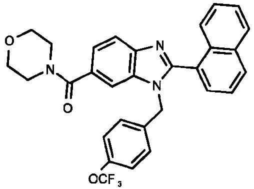 |
| 786 | 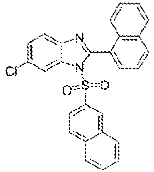 | 1496 | 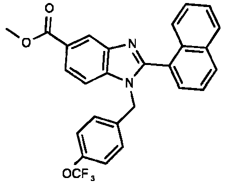 |
| 787 | 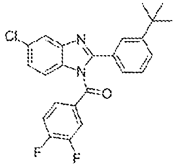 | 1497 | 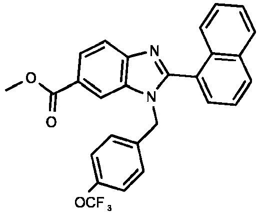 |
| 788 | 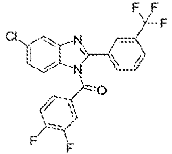 | 1498 | 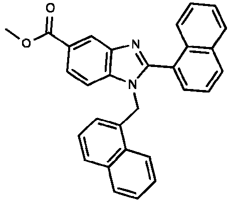 |
| 789 | 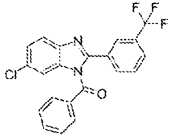 | 1499 | 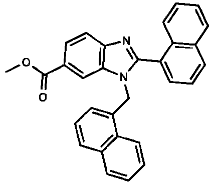 |
| 790 | 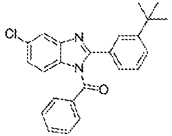 | 1500 | 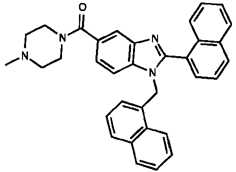 |
| 791 | 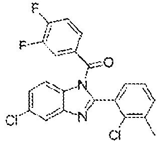 | 1501 | 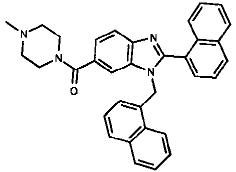 |
| 792 | 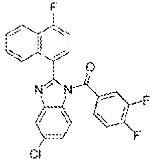 | 1502 | 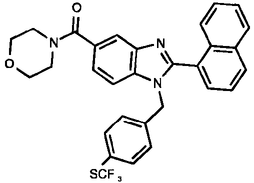 |
| 848 | 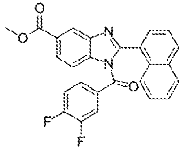 | 1503 | 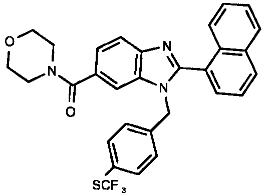 |
| 849 | 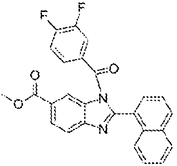 | 1504 | 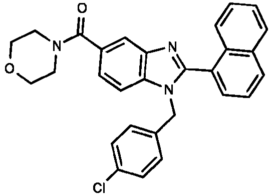 |
| 850 | 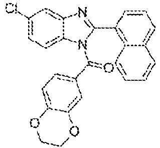 | 1505 | 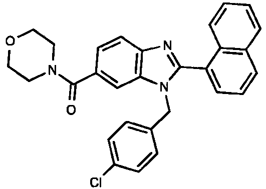 |
| 851 | 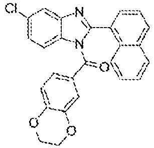 | 1506 | 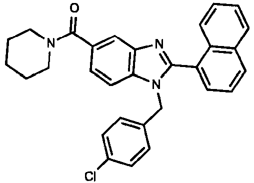 |
| 852 | 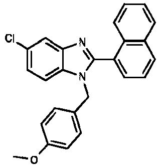 | 1507 | 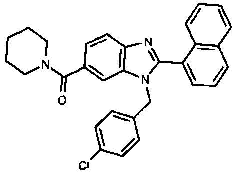 |
| 853 | 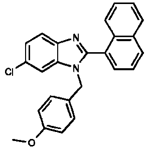 | 1508 | 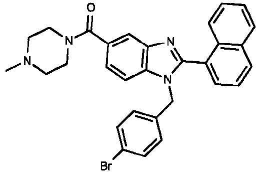 |
| 854 | 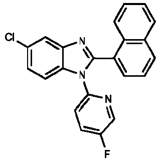 | 1509 | 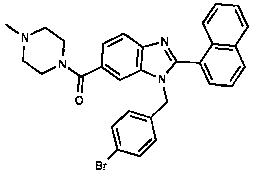 |
| 855 | 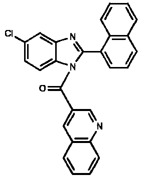 | 1510 | 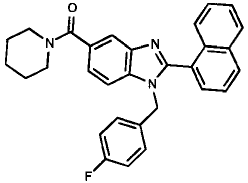 |
| 856 | 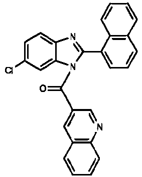 | 1511 | 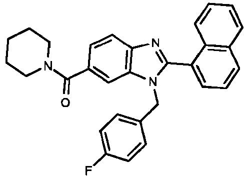 |

**Table 2. Exemplary compounds of Formula II, IIA, IIB, IIC, IID, and IIE.**

| No | Structure | No | Structure |
|---|---|---|---|
| 359 | 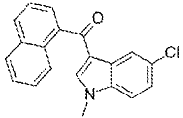 | 884 | 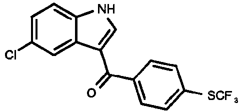 |
| 364 | 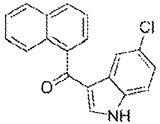 | 885 | 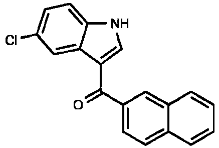 |
| 433 | 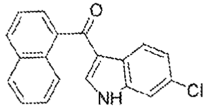 | 889 | 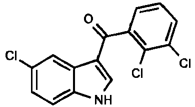 |
| 434 | 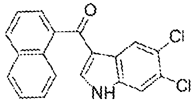 | 890 | 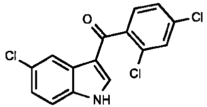 |
| 521 | 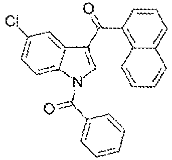 | 891 | 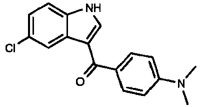 |
| 874 | 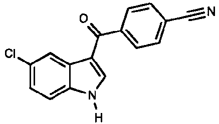 | 892 | 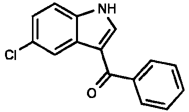 |
| 881 | 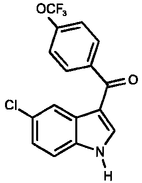 | 893 | 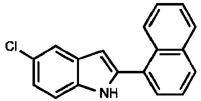 |
| 882 | 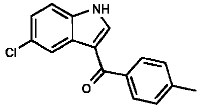 | 894 | 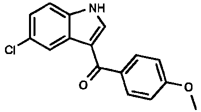 |
| 883 | 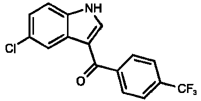 | 895 | 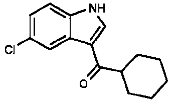 |

**Table 3. Exemplary compounds of Formula III, IIIA, and IIIB**

| No | Structure | No | Structure |
|---|---|---|---|
| 405 | 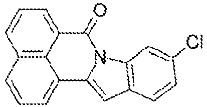 | 525 | 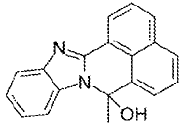 |
| 435 | 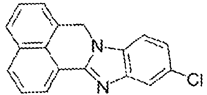 | 523 | 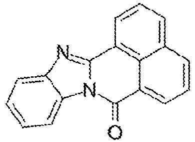 |
| 464 | 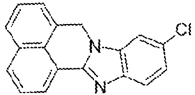 | | |

**Table 4. Exemplary compounds of Formula IV, IVA, and IVB**

| No | Structure | No | Structure |
|---|---|---|---|
| 524 | 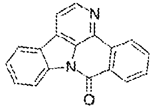 | 526 | 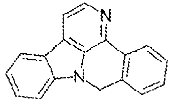 |

**Table 5 Additional exemplary Ahr ligands**

| Compound | Structure | Compound | Structure |
|---|---|---|---|
| 365 | 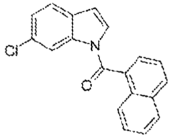 | 406 | 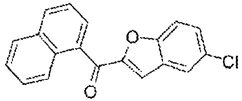 |
| 366 | 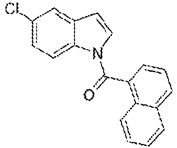 | 488 | 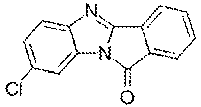 |
| 972 | 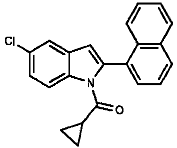 | 489 | 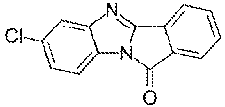 |
| 973 | 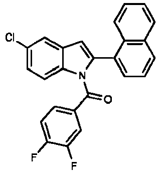 | 522 | 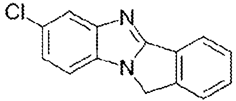 |

### AHR LIGAND SCREENING

The AhR is a ligand-activated transcription factor that dimerizes with ARNT to regulate gene expression, and genes that are regulated by AhR ligands have AhR response elements (AhRE) in their promotor regions. Activation of the AhR by novel compounds of interest was measured as previously described by O'Donnell et al. (O'Donnell, E.F.; Saili, K.S.; Koch, D.C.; Kopparapu, P.R.; Farrer, D.; Bisson, W.H.; Mathew, L.K.; Sengupta, S.; Kerkvliet, N.I.; Tanguay, R.L.; Kolluri, S.K. The Anti-Inflammatory Drug Leflunomide Is an Agonist of the Aryl Hydrocarbon Receptor. PLoS ONE 2010, 5; O'Donnell E. F., Jang H. Sang, Pearce M., Kerkvliet N. I., Kolluri S. K.The aryl hydrocarbon receptor is required for induction of p21cip1/waf1 expression and growth inhibition by SU5416 in hepatoma cells. Oncotarget. 2017; 8: 25211-25225) and Punj et al. (Punj S, Kopparapu P, Jang HS, Phillips JL, Pennington J, Rohlman D, O'Donnell E, Iversen PL, Kolluri SK, Kerkvliet NI. Benzimidazoisoquinolines: A New Class of Rapidly Metabolized Aryl Hydrocarbon Receptor (AhR) Ligands that Induce AhR-Dependent Tregs and Prevent Murine Graft-Versus-Host Disease. PLoS ONE 2014; 9(2): e887264).

Briefly, Hepa1 cells were transfected with a reporter construct consisting of AhRE linked to luciferase. The addition of AhR ligands to the transfected cells induces luciferase production that is directly proportional to the amount of AhR activation. We used this reporter system to identify novel compounds with AhR-activating properties. Transfected Hepa1 cells were plated at a density of 1×10⁴ cells/well in 100 µL of cell culture media in 96 well plates and grown overnight. The following day, cells were treated for the indicated time or 15 hours with vehicle (DMSO) or the analogs of 11-cl-BBQ. Following incubation with the compounds, the media was removed, and cells were harvested with lysis buffer. The lysates were transferred to opaque 96 well plates, where they were assayed well-by-well for luciferase activity by injection of luciferase assay substrate with a 2 sec mixing time and 15 sec integration period on a Tropix TR717 microplate luminometer. Data were expressed as fold induction of luciferase relative to vehicle (0.1% DMSO) treated cells. The reference compound (11-cl-BBQ), as well as TCDD (2,3,7,8-tetrachlorodibenzo-p-dioxin) were used as positive controls for AhR activation. Compounds that did not induce AhR activation by at least two-fold at 10 micromolar concentration were considered to lack AhR- activating properties.

Table 6 shows AhR activation activity of exemplary compounds.

**Table 6. AhR activation by exemplary compounds of the disclosure. Fold @ 1 nM, Fold @ 100 nM and Fold @ 10 uM refer to the fold change of luciferase expression after treatment of cells with 1 nM, 100 nM or 10 uM respectively of test compound relative to vehicle (0.1% DMSO) treated cells in the AhR ligand screening assay. Fold relative to benchmark @ 100 nM refers to the fold change of luciferase expression after treatment of cells with 100 nM of test compound relative to the treatment with 100 nM of a benchmark compound (11-cl-BBQ) in the AhR ligand screening assay described above.**

| Compound | Fold @ 1 nM | Fold @ 100 nM | Fold @ 10 uM | Fold relative to benchmark @ 100 nM |
|---|---|---|---|---|
| 359 | 1 | 1 | 6 | 0.03 |
| 360 | 1 | 1 | 2 | 0.03 |
| 361 | 1 | 1 | 3 | 0.03 |
| 362 | 16 | 22 | - | 1 |
| 363 | 4 | 14 | 30 | 0.69 |
| 364 | 1 | 2 | 11 | 0.05 |
| 365 | 1 | 1 | 6 | 0.03 |
| 366 | 1 | 1 | 11 | 0.03 |
| 405 | 8 | 31 | - | 0.78 |
| 406 | 1 | 1 | 7.5 | 0.03 |
| 429 | 1 | 2 | 19 | 0.05 |
| 430 | 1 | 2 | 80 | 0.05 |
| 431 | 1 | 1 | 8 | 0.03 |
| 432 | 1 | 1 | 7 | 0.03 |
| 433 | 1 | 1 | 7 | 0.03 |
| 434 | 1 | 1 | 9 | 0.03 |
| 435 | 4 | 28 | 60 | 0.7 |
| 488 | 1 | 1 | 4 | 0.03 |
| 489 | 1 | 1 | 5 | 0.03 |
| 490 | 1 | 1 | 17 | 0.03 |
| 491 | 1 | 1 | 24 | 0.03 |
| 492 | 1 | 1 | 14 | 0.03 |
| 493 | 1 | 1 | 16 | 0.03 |
| 494 | 1 | 29 | 43 | 0.94 |
| 495 | 2.3 | 25 | 59 | 0.81 |
| 496 | 1 | 1 | 3.5 | 0.03 |
| 497 | 1 | 1.5 | 3.5 | 0.05 |
| 521 | 1 | 1 | 1 | 0.07 |
| 522 | 1 | 1 | 18 | 0.07 |
| 523 | 1.8 | 11.5 | 12 | 0.82 |
| 524 | 1.2 | 4.8 | 20 | 0.34 |
| 525 | 1 | 5.6 | 20 | 0.4 |
| 526 | 2.7 | 10 | 28 | 0.71 |
| 527 | 1 | 15 | 36 | 0.38 |
| 528 | 1 | 19 | 35 | 0.48 |
| 529 | 2 | 41 | 52 | 1.03 |
| 530 | 2 | 39 | 56 | 0.98 |
| 533 | 1 | 1 | 13 | 0.03 |
| 534 | 1 | 1 | 26 | 0.03 |
| 535 | 1 | 38 | 44 | 0.95 |
| 536 | 1 | 39 | 41 | 0.98 |
| 579 | 1 | 1 | 2 | 0.07 |
| 580 | 1 | 3 | 14 | 0.2 |
| 581 | 1 | 1 | 15 | 0.07 |
| 582 | 1 | 1 | 14 | 0.07 |
| 583 | 1 | 5 | 7 | 0.33 |
| 584 | 1 | 3 | 31 | 0.2 |
| 585 | 1 | 1 | 34 | 0.07 |
| 586 | 7 | 20 | 18 | 1.33 |
| 587 | 2 | 16 | 19 | 1.07 |
| 588 | 1 | 5 | 30 | 0.33 |
| 589 | 2 | 12 | 16 | 0.8 |
| 590 | 1 | 3 | 45 | 0.2 |
| 591 | 2 | 2 | 38 | 0.13 |
| 630 | 1 | 5 | 9 | 0.56 |
| 631 | 1 | 1 | 7 | 0.11 |
| 632 | 1 | 2 | 17 | 0.22 |
| 633 | 1 | 2 | 25 | 0.22 |
| 634 | 1 | 2 | 22 | 0.22 |
| 635 | 1 | 2 | 24 | 0.22 |
| 636 | 1 | 1 | 14 | 0.11 |
| 637 | 1 | 2 | 13 | 0.22 |
| 638 | 1 | 12 | 20 | 1.17 |
| 639 | 1 | 1 | 5 | 0.11 |
| 640 | 1 | 10 | 15 | 1.21 |
| 641 | 1 | 1 | 21 | 0.11 |
| 642 | 1 | 1 | 27 | 0.11 |
| 643 | 1 | 1 | 2 | 0.11 |
| 644 | 1 | 1 | 2 | 0.11 |
| 645 | 1 | 1 | 15 | 0.11 |
| 681 | 1 | 25 | 24 | 0.96 |
| 682 | 1 | 18 | 32 | 0.69 |
| 683 | 1 | 26 | 34 | 1 |
| 684 | 1 | 2 | 56 | 0.08 |
| 685 | 1 | 13 | 28 | 0.5 |
| 686 | 1 | 24 | 31 | 0.92 |
| 687 | 1 | 28 | 32 | 1.08 |
| 688 | 2 | 28 | 44 | 1.08 |
| 689 | 1 | 28 | 47 | 1.08 |
| 690 | 2 | 22 | 30 | 0.85 |
| 691 | 2 | 26 | 34 | 1 |
| 692 | 3 | 24 | 85 | 0.92 |
| 693 | 2 | 32 | 87 | 1.23 |
| 694 | 2 | 32 | 35 | 1.23 |
| 695 | 1 | 23 | 30 | 0.88 |
| 696 | 1 | 22 | 51 | 0.85 |
| 697 | 1 | 23 | 22 | 0.88 |
| 698 | 1 | 26 | 30 | 1 |
| 699 | 1 | 22 | 24 | 0.85 |
| 700 | 1 | 16 | 22 | 0.62 |
| 701 | 1 | 2 | 35 | 0.08 |
| 702 | 1 | 1 | - | 0.04 |
| 703 | 1 | 22 | 23 | 0.85 |
| 704 | 1 | 34 | 17 | 1.31 |
| 763 | 1 | 2 | 22 | 0.08 |
| 764 | 1 | 2 | 20 | 0.08 |
| 765 | 1 | 2 | 14 | 0.08 |
| 766 | 1 | 2 | 31 | 0.08 |
| 767 | 1 | 2 | 23 | 0.08 |
| 770 | 1 | 2 | 2 | 0.08 |
| 771 | 1 | 1 | 2 | 0.04 |
| 772 | 1 | 1 | 1 | 0.04 |
| 773 | 1 | 25 | 40 | 1 |
| 774 | 1 | 21 | 28 | 0.84 |
| 775 | 1 | 19 | 25 | 0.76 |
| 776 | 1 | 15 | 20 | 0.6 |
| 777 | 1 | 2 | 6 | 0.08 |
| 778 | 1 | 1 | 8 | 0.04 |
| 779 | 1 | 1 | 5 | 0.04 |
| 780 | 1 | 1 | 7 | 0.04 |
| 781 | 1 | 20 | 24 | 0.8 |
| 782 | 1 | 23 | 19 | 0.92 |
| 783 | 1 | 1 | 24 | 0.03 |
| 784 | 1 | 2 | 34 | 0.07 |
| 785 | 1 | 16 | 44 | 0.55 |
| 786 | 1 | 31 | 40 | 1.07 |
| 787 | 1 | 1 | 4 | 0.04 |
| 788 | 1 | 2 | 19 | 0.08 |
| 789 | 1 | 3 | 8 | 0.12 |
| 790 | 1 | 1 | 2 | 0.04 |
| 791 | 1 | 1 | 26 | 0.04 |
| 792 | 1 | 2 | 19 | 0.08 |
| 848 | 1 | 1 | 5 | 0.04 |
| 849 | 1 | 1 | 1 | 0.04 |
| 850 | 1 | 22 | 33 | 0.88 |
| 851 | 1 | 21 | 29 | 0.84 |
| 852 | 1 | 1 | 29 | 0.03 |
| 853 | 1 | 1 | 11 | 0.03 |
| 854 | 1 | 1 | 4 | 0.03 |
| 855 | 1 | 33 | 44 | 1.14 |
| 856 | 1 | 38 | 46 | 1.31 |
| 857 | 1 | 12 | 75 | 0.41 |
| 858 | 1 | 10 | 73 | 0.34 |
| 859 | 1 | 12 | 80 | 0.41 |
| 860 | 1 | 3 | 88 | 0.1 |
| 861 | 1 | 2 | 90 | 0.07 |
| 862 | 1 | 2 | 82 | 0.07 |
| 863 | 1 | 2 | 34 | 0.07 |
| 864 | 1 | 2 | 47 | 0.07 |
| 865 | 1 | 7 | 47 | 0.24 |
| 866 | 1 | 34 | 50 | 1.17 |
| 867 | 1 | 2 | 32 | 0.07 |
| 868 | 1 | 2 | 28 | 0.07 |
| 869 | 1 | 12 | 68 | 0.41 |
| 870 | 1 | 9 | 74 | 0.31 |
| 871 | 1 | 1 | 12 | 0.02 |
| 872 | 1 | 1 | 34 | 0.02 |
| 874 | 1 | 7 | 52 | 0.13 |
| 875 | 1 | 3 | 48 | 0.06 |
| 876 | 1 | 3 | 32 | 0.06 |
| 877 | 1 | 3 | 42 | 0.06 |
| 881 | 1 | 2 | 18 | 0.04 |
| 882 | 1 | 1 | 2 | 0.02 |
| 883 | 1 | 1 | 2 | 0.02 |
| 884 | 1 | 2 | 16 | 0.04 |
| 885 | 1 | 1 | 1 | 0.02 |
| 886 | 1 | 14 | 300 | 0.26 |
| 887 | 1 | 1 | 14 | 0.02 |
| 888 | 1 | 1 | 9 | 0.02 |
| 889 | 1 | 1 | 5 | 0.02 |
| 890 | 1 | 2 | 17 | 0.04 |
| 891 | 1 | 1 | 21 | 0.02 |
| 892 | 1 | 1 | 15 | 0.02 |
| 893 | 1 | 49 | 49 | 0.91 |
| 894 | 1 | 1 | 7 | 0.02 |
| 895 | 1 | 1 | 10 | 0.02 |
| 972 | 1 | 2 | 45 | 0.04 |
| 973 | 1 | 1 | 1 | 0.02 |
| 1328 | 1 | 2 | 11 | 0.07 |
| 1329 | 1 | 1 | 1 | 0.03 |
| 1330 | 1 | 1 | 1 | 0.03 |
| 1336 | 1 | 1 | 6 | 0.03 |
| 1337 | 1 | 1 | 8 | 0.03 |
| 1338 | 1 | 1 | 2 | 0.03 |
| 1339 | 1 | 1 | 4 | 0.03 |
| 1340 | 1 | 1 | 8 | 0.03 |
| 1341 | 1 | 1 | 5 | 0.03 |
| 1342 | 1 | 2 | 2 | 0.07 |
| 1356 | 1 | 1 | 1 | 0.03 |
| 1357 | 1 | 1 | 1 | 0.03 |
| 1358 | 1 | 1 | 2 | 0.03 |
| 1359 | 1 | 1 | 2 | 0.03 |
| 1360 | 1 | 2 | 18 | 0.07 |
| 1361 | 1 | 2 | 2 | 0.07 |
| 1362 | 5 | 3 | 18 | 0.1 |
| 1363 | 1 | 1 | 4 | 0.03 |
| 1364 | 1 | 1 | 3 | 0.03 |
| 1365 | 1 | 1 | 2 | 0.03 |
| 1366 | 1 | 1 | 10 | 0.03 |
| 1367 | 1 | 1 | 3 | 0.03 |
| 1368 | 1 | 2 | 2 | 0.07 |
| 1369 | 1 | 1 | 2 | 0.03 |
| 1370 | 1 | 1 | 4 | 0.03 |
| 1384 | 1 | 2 | 13 | 0.07 |
| 1385 | 1 | 2 | 5 | 0.07 |
| 1386 | 1 | 2 | 6 | 0.07 |
| 1387 | 1 | 1 | 2 | 0.03 |
| 1388 | 1 | 2 | 2 | 0.07 |
| 1389 | 1 | 1 | 3 | 0.03 |
| 1390 | 1 | 1 | 3 | 0.03 |
| 1391 | 1 | 1 | 1 | 0.03 |
| 1392 | 1 | 1 | 4 | 0.03 |
| 1393 | 1 | 1 | 4 | 0.03 |
| 1401 | 1 | 1 | 2 | 0.03 |
| 1402 | 1 | 2 | 3 | 0.07 |
| 1403 | 1 | 2 | 20 | 0.07 |
| 1404 | 1 | 1 | 6 | 0.03 |
| 1405 | 1 | 2 | 30 | 0.07 |
| 1406 | 1 | 1 | 4 | 0.03 |
| 1407 | 1 | 1 | 9 | 0.03 |
| 1408 | 1 | 1 | 4 | 0.03 |
| 1409 | 1 | 1 | 4 | 0.03 |
| 1411 | 1 | 1 | 4 | 0.03 |
| 1412 | 1 | 1 | 6 | 0.03 |

### DRUG METABOLISM/DMPK

### 1. Preparation of compounds.

Compound solutions were prepared from powder as 10 mM or 1 mM stock solutions in Dimethyl Sulfoxide (DMSO; Cat. No. #D2650, Sigma Aldrich) and stored at -20°C.

### 2. Kinetic solubility.

Test articles were serially diluted in DMSO from concentration range of 10mM to 0.78 mM in 96 well V bottom dilution plate (#3363costar). 1µL of test article from each well was transferred to 96 well Flat bottom clear plates (#655101Greiner) containing 99 µL of PBS at pH-7.4 so that the DMSO concentration should not exceed >1%. Samples were incubated for one hour at 37°C followed by measurement of light scattering at 635 nm with a laser based micro plate nephelometer. Concentration (µM) was then calculated by segmental regression. Amiodarone (#A8423 Aldrich) was used as positive control.

### 3. Solubility in simulated gastric and intestinal fluids (SGF and SIF).

The following conditions were used:
- Simulated Gastric Fluid in Fed state, pH 5.0 (FeSSGF)
- Simulated Gastric Fluid in Fasted state, pH 1.2 (FaSSGF)
- Simulated Intestinal Fluid in Fasted state, pH 6.5 (FaSSIF)
- Simulated Intestinal Fluid in Fed state, pH 5.0 (FeSSIF)

Test article (1 mg) was dissolved in 1 ml of FeSSGF (pH-5.0), FaSSGF (pH-1.2), FaSSIF (pH-6.5) and FeSSIF (pH-5.0) in a transparent glass vial. Reactions were kept in reciprocating water bath at 37°C for overnight. After 12-14 hrs, all the samples were centrifuged at 10,000 rpm for 15 min. Supernatant was taken, diluted, and injected in LC-MS/MS (Shimadzu Nexera UPLC with an AB Sciex 4500 detector). Solubility was measured by plotting area of test in simulated fluids versus area of standard. Ketoconazole (#K1003 Aldrich) was used as positive control.

### 4. Liver microsomal stability.

The assessment of metabolic stability of testing compounds was performed using human, mouse, rat, dog and monkey liver microsomes (20 mg protein/ml).Each reaction mixture contained 42.5 µL of 0.1 M potassium phosphate buffer pH 7.4, containing respective LM protein (final concentration 0.5 mg/ml). 2.5 µL of the compound stock solution was added in it (1µM final concentration). The reaction was initiated by the addition of 5 µL NADPH solution (final Concentration 1 mM). At different time points (0, 5, 15, and 30 minutes), samples were quenched with 200 µL of cold acetonitrile containing ISTD Propranolol. Samples were centrifuged at 3500 rpm for 20 min at 4°C. Supernatant was subjected to LC-MS/MS analysis for quantification. Verapamil was used as a positive control.

### 5. CYP panel profile: P450 inhibition

From 10 mM stock solutions of test compounds, a dilution plate was prepared diluting serially starting from 5 mM up to 2 µM in Acetonitrile/DMSO or Methanol/DMSO Human liver Microsomes were added at required concentration as per specific CYP isoform in a deep well assay plate (1A2, 2C9, 2D6, 2B6, 2C8, 2C19, and 3A4). Compounds were spiked in all wells from dilution plate at final concentrations starting from 50 µM up to 0.02 µM, except for positive and negative control. Specific substrate were added to all wells and reactions were pre-incubated for 10 min. To start reactions, NADPH was added to all wells at 1mM final concentration. Assay plate was mixed by vortexing and incubated at 37°C for 10 min for 3A4,20 min for (1A2, 2C9, 2B6, 2C8, 2D6) and 40 min for 2C19. A quencher with chilled acetonitrile suitable internal standard was added. Samples were centrifuged and supernatants were collected and subjected to LC-MS/MS analysis for determination.

Data for exemplary compounds is presented in Tables 7-9.

**Table 7 Solubility of exemplary compounds in simulated gastric and intestinal fluids (Fasted and fed conditions)**

| Stock DMSO mM | Kinetic Solubility (µM) | FaSSGF (µg/ml) | FaSSIF (µg/ml) | FeSSGF (µg/ml) | FeSSIF (µg/ml) |
|---|---|---|---|---|---|
| <1 | <1 | 8.0 | 1.1 | 1.9 | 8.0 |
| 100 | 7.0 | 0.8 | 8.6 | 0.7 | 29.0 |
| 100 | 9.4 | 3.6 | 25.6 | 16.5 | 26.5 |
| 100 | 5.9 | - | - | - | - |
| 100 | 9.4 | 0.3 | 4.3 | 0.1 | 27.3 |
| 50 | 7.0 | 0.4 | 64.2 | 3.0 | 15.0 |
| 10 | 28.8 | - | - | - | - |
| 100 | 4.7 | 1.7 | 8.7 | 0.5 | 11.5 |
| 100 | 1.8 | 1.1 | 12.8 | 0.1 | 85.4 |
| 50 | 28.1 | - | - | - | - |
| 50 | 14.1 | - | - | - | - |

**Table 8 CYP inhibition by exemplary compounds**

| Compound | CYP-1A2 IC₅₀ (µM) | CYP-2C9 IC₅₀ (µM) | CYP-2D6 IC₅₀ (µM) | CYP-2B6 IC₅₀ (µM) | CYP-2C8 IC₅₀ (µM) | CYP-2C19 IC₅₀ (µM) | CYP-3A4 IC₅₀ (µM) |
|---|---|---|---|---|---|---|---|
| 435 | <0.02 | >50 | >50 | >50 | 19.2 | >50 | 5.3 |
| 494 | 0.7 | 4.2 | >50 | >45 | 2.5 | 5.2 | 1.2 |
| 495 | 0.3 | 5.6 | 32.6 | 19.3 | 1.9 | 1.4 | 5.3 |
| 529 | 0.6 | 3.5 | >50 | 9.6 | 9.6 | 3.6 | 1.2 |
| 530 | 0.5 | 5.0 | >50 | 9.4 | 9.4 | 3.4 | 3.7 |
| 535 | 3.9 | 2.5 | >50 | 21.5 | 21.8 | 2.1 | 0.2 |
| 536 | 0.6 | 4.8 | >50 | 16.0 | 17.1 | 2.6 | 1.7 |
| 586 | 1.7 | 7.9 | >50 | 22.0 | 9.1 | 7.1 | 1.3 |
| 587 | 0.4 | 5.3 | 26.0 | 11.6 | 3.2 | 6.7 | 6.3 |
| 638 | 0.3 | 5.2 | 14.5 | 11.6 | 1.8 | 3.4 | 8.1 |
| 640 | 0.6 | 4.5 | 29.0 | 31.1 | 2.3 | 2.4 | 3.8 |
| 643 | 20.7 | 9.2 | >50 | >50 | 12.3 | >50 | >50 |
| 644 | 4.7 | 2.7 | 8.2 | >50 | 1.7 | >50 | >50 |
| 693 | 8.6 | 11.6 | >50 | >50 | 3.5 | 24.1 | 2.0 |
| 703 | 0.8 | 5.3 | >50 | 12.0 | 4.2 | 0.5 | 10.3 |
| 704 | 1.2 | 8.9 | >50 | 8.2 | 5.6 | 4.6 | 3.9 |
| 782 | 47.9 | >50 | >50 | >50 | >50 | 6.0 | 0.4 |
| 848 | 2.6 | 6.3 | 2.9 | 16.7 | 3.5 | 1.1 | 13.7 |
| 849 | 15.1 | 7.7 | 19.3 | > 48.0 | 4.1 | 1.5 | >50 |
| 893 | 0.5 | 7.2 | 31.7 | 8.0 | 7.7 | 2.9 | 23.3 |
| 972 | 7.0 | 7.0 | >50 | 4.3 | > 48.8 | 0.2 | >50 |
| 973 | >50 | >50 | >50 | >50 | >50 | 23.1 | >50 |

**Table 9**

| Compound | Rem @ HLM x 30 min (%) | Rem @ MLM x 30 min (%) | Rem @ RLM x 30 min (%) | T ½ @ HLM (min) | T ½ @ MLM (min) | T ½ @ RLM (min) | Clint @ HLM (µl/min/mg) | Clint @ MLM (µl/min/mg) | Clint @ RLM (µl/min/mg) |
|---|---|---|---|---|---|---|---|---|---|
| 362 | - | 12.0 | 14.5 | - | 9.8 | 11.4 | - | 141.0 | 121.0 |
| 434 | 63.4 | 10.6 | 41.6 | 48.7 | 10.7 | 28.0 | 28.5 | 129.2 | 49.6 |
| 521 | 58.0 | 64.6 | 45.5 | 40.3 | 60.9 | 24.8 | 34.0 | 23 | 56 |
| 644 | 69.8 | 71.7 | 36.8 | 77.9 | 70.5 | 28.7 | 18 | 20 | 48 |
| 774 | 52.7 | 28.2 | 45.5 | 36.3 | 19.3 | 31.9 | 38.2 | 71.9 | 43.5 |
| 776 | 28.3 | 23.9 | 27.5 | 16.4 | 14.6 | 16.3 | 84.6 | 94.7 | 84.9 |
| 781 | 44.4 | 18.0 | 43.1 | 24.6 | 12.1 | 24.4 | 56.3 | 114.8 | 56.8 |
| 782 | 66.4 | 14.9 | 31.8 | 50.5 | 10.9 | 17.8 | 27.4 | 126.9 | 78.0 |
| 785 | 54.2 | 49.3 | 57.1 | 33.9 | 29.4 | 36.5 | 40.9 | 47.2 | 37.9 |
| 786 | 27.4 | 22.1 | 25.6 | 16.1 | 13.4 | 14.8 | 86.2 | 103.6 | 93.8 |
| 884 | 81.2 | 22.2 | 23.1 | 98.8 | 13.9 | 14.3 | 14 | 100 | 96.7 |
| 885 | 58.1 | 45.1 | 50.2 | 36.8 | 27.5 | 30.7 | 37.7 | 50.4 | 45.1 |
| 889 | 30.2 | 43.5 | 33.5 | 22.6 | 33.9 | 25.6 | 61.2 | 40.9 | 54.2 |
| 890 | 44.7 | 39.3 | 37.4 | 35.2 | 28.2 | 29.0 | 39.4 | 49.1 | 47.8 |
| 891 | 54.3 | 52.4 | 45.2 | 42.4 | 36.3 | 31.0 | 32.7 | 38.2 | 44.7 |
| 892 | 24.7 | 36.7 | 64.9 | 15.3 | 21.0 | 57.0 | 90.7 | 66.1 | 24.3 |
| 893 | 19.6 | 16.7 | 51.7 | 14.1 | 12.2 | 35.8 | 98.5 | 113.5 | 38.8 |
| 894 | 57.9 | 54.5 | 58.1 | 45.3 | 37.6 | 43.0 | 30.6 | 36.9 | 32.3 |
| 972 | 73.0 | 26.7 | 20.7 | 74.9 | 15.9 | 13.3 | 18.5 | 87.1 | 104.6 |
| 973 | 34.2 | 58.7 | 46.3 | 16.7 | 39.9 | 26.5 | 83.1 | 34.8 | 52.3 |

### IN VIVO PK STUDY

Pharmacokinetics of exemplary Compounds 362 and 893 following an intravenous and oral administration in male C57BL/6J mice.

Male C57BL/6J mice, approximately 8-10 weeks old, were obtained from the vivarium of Fundación Ciencia & Vida Chile (Santiago, Chile). Dosing solution of Compound 362 for PO administration was formulated in a vehicle containing 40% DMSO, 20% Kolliphor EL, 40% Propylene Glycol at 0.8 mg/mL. Dosing solution of Compound 362 for IV administration was formulated in a vehicle containing 30% DMSO, 20% Kolliphor EL, 50% PBS at 0.4 mg/Kg.

Male BalbC mice, approximately 8-11 weeks old, 22-27 grams were obtained from the vivarium Fundación Ciencia & Vida Chile (Santiago, Chile). Animals were acclimated for a minimum period of 4 days upon arrival at the testing facility. Animals were weighed, identified by marking the tail with numbers using a non-toxic permanent marker and designated into the following treatment groups on the day of dosing:
Group 1 animals received an IV administration via caudal vein of 2 mg/kg PRXS0362 dosing solution.
Group 2 animals received a PO administration via feeding tubes (20 gauge) of 8 mg/kg PRXS0362 dosing solution.

| Group | No. animals | Route | Dose [mg/Kg] | Dose conc. [mg/mL] | Dose Vol. [mL/Kg] |
|---|---|---|---|---|---|
| 1 | 30 | I.V. | 2 | 0.4 | 5 |
| 2 | 18 | P.O. | 8 | 0.8 | 10 |

Terminal whole blood was collected via cardiac puncture for group 1 at the following time points: 5, 10, 15, 30, 60, 120, 240, 360, and 480 minutes. Non-dosed mice were used to collect samples of zero time points. For the group 2 at the following time point: 15, 30, 60, 120, 240, 360, and 480 minutes.

Whole blood, approximately 300 µL per time point, was collected into microtainer tubes with EDTA (K2). Blood samples were centrifuged immediately at approximately 9,000 G at 4°C for 5 minutes and plasma separated. Plasma samples were placed into individually labeled tubes and stored in a -80°C freezer prior to LC/MS/MS analysis.

The whole brain was collected at each point only for both groups, for this the animals were euthanized with CO₂, decapitated, the brain was extracted weighed, frozen in liquid nitrogen and stored at -80 °C prior to LC/MS/MS analysis.

Compound 893 was tested by the same protocol as above.

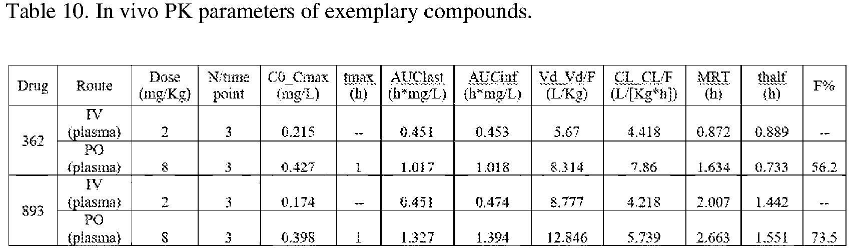

While illustrative embodiments have been illustrated and described, it will be appreciated that various changes can be made therein without departing from the spirit and scope of the invention.

### CLAUSES

1. A compound of the formula: 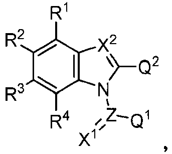 a tautomer thereof, a stereoisomer thereof, a hydrate thereof, or a pharmaceutically acceptable salt thereof, wherein:
   Q¹ is an optionally substituted C6-C10 aryl; optionally substituted C5-C10 heteroaryl; optionally substituted C5-C10 heterocyclyl; optionally substituted C1-C10 alkyl, or optionally substituted C3-C10 cycloalkyl;
   Q² is an optionally substituted C6-C14 aryl; optionally substituted C5-C10 heteroaryl; optionally substituted C5-C10 heterocyclyl; optionally substituted C1-C10 alkyl, or optionally substituted C3-C10 cycloalkyl;
   Z is C, S(O), 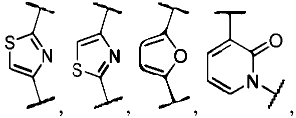 or Z is CH when X¹ is absent;
   X1 is absent, O, NH, S, or X1 is 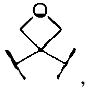 wherein the wavy lines denote points of attachment to Z;
   X2 is N, CCl, CF, CBr, CI, CCN, CCONH2, CCOOH, or CH;
   R¹, R², R³, and R⁴ are independently H, halogen, CN, optionally substituted C1-C10 alkyl, optionally substituted C3-C10 cycloalkyl, optionally substituted C1-C6 alkoxy, SO2R5, CO²R⁵, or CONR⁵R⁶, or any one of R¹ and R², R² and R³, and R³ and R⁴ pairs, together with the carbon atoms to which they are attached, forms an optionally substituted a five- or six-membered cycloalkenyl, heterocyclenyl, aryl, or heteroaryl; and
   R⁵ and R⁶ are independently H, optionally substituted C1-C10 alkyl, or optionally substituted C3-C10 cycloalkyl, or R⁵ and R⁶, together with the nitrogen atom to which they are attached, form an optionally substituted 5-membered ring or an optionally substituted 6-membered ring.
2. The compound of Clause 1, wherein X² is N.
3. The compound of Clause 1 or Clause 2, wherein Z is C.
4. The compound of Clause 1 or Clause 2, wherein X¹ is O and Z is SO.
5. The compound of any one of the preceding clauses, wherein Q¹ is selected from optionally substituted pyridyl, optionally substituted benzyl, optionally substituted phenyl, optionally substituted cyclohexyl, optionally substituted piperidinyl, optionally substituted quinolinyl, optionally substituted benzofuryl, optionally substituted benzomorpholinyl, and optionally substituted benzimidazolyl.
6. The compound of any one of the preceding clauses, wherein Q¹ is: 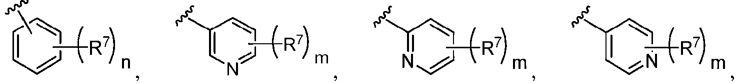 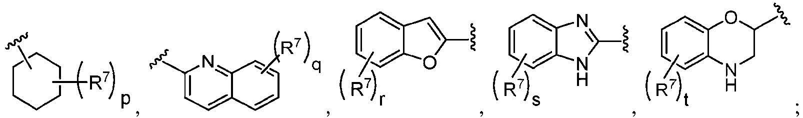 or 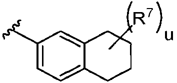
   wherein R⁷, at each occurrence, is independently H, halogen, CN, optionally substituted C1-C10 alkyl, optionally substituted C2-C6 alkenyl, optionally substituted C1-C10 heteroalkyl, optionally substituted C1-C10 heterocyclyl, optionally substituted C6-C10 aryl, optionally substituted C5-C10 heteroaryl, optionally substituted C1-C6 alkoxy, optionally substituted C1-C6 cycloalkyloxy, OCF₃, NR⁵R⁶, SCF₃, or C(O)NR⁵R⁶;
   R⁵ and R⁶ are as defined above;
   n is an integer ranging from 1 to 5; m is an integer ranging from 1 to 4; p is an integer ranging from 1 to 11; q is an integer ranging from 1 to 6; r is an integer ranging from 1 to 5; s is an integer ranging from 1 to 4; t is an integer ranging from 1 to 8; and u is an integer ranging from 1 to 11.
7. The compound of any one of Clauses 1 to 6, wherein Q¹ is a phenyl optionally substituted with one, two, or three substituents independently selected from F, Cl, OCH₃, CN, OCF₃, SCF₃, t-Bu, NMe₂, CONH₂, piperazyl, piperidyl, OCH₂CH₂OH, OCH₂CH₂NMe₂, and 1-naphthoyl.
8. The compound of any one of Clauses 1 to 7, wherein Q¹ is: 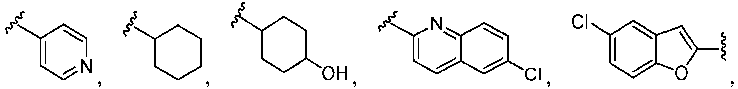 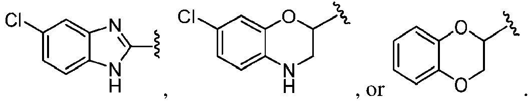
9. The compound of any one of Clauses 1 to 8, wherein Q² is selected from optionally substituted phenyl, optionally substituted quinolinyl, optionally substituted benzofuryl, optionally substituted thiophenyl, optionally substituted biphenyl, and optionally substituted indazolyl.
10. The compound of any one of the preceding clauses, wherein Q² is: 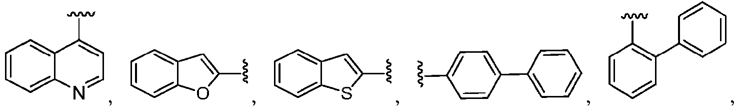 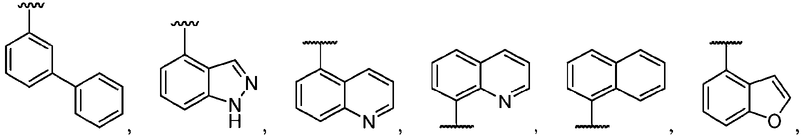 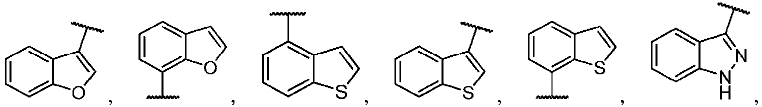 or 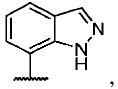 each of which is optionally substituted with one to four substituents independently selected from F, Cl, OCH₃, CN, OCF₃, CF₃, SCF₃, Me, Et, i-Pr, t-Bu, NMe₂, CONH₂, OCH₂CH₂OH, OCH₂CH₂NMe₂, CHCH₂, OMe, OEt, O(iPr), O(tBu), and OC₅H₁₁.
11. The compound of any one of Clauses 1 to 10, wherein Q² is a phenyl optionally substituted with one or two substituents independently selected from optionally substituted C1-C10 alkyl, optionally substituted C2-C6 alkenyl, optionally substituted C1-C10 heteroalkyl, optionally substituted C6-C10 aryl, optionally substituted C1-C6 alkoxy, optionally substituted C1-C6 cycloalkyloxy, CN, OCF₃, SCF₃, NMe₂, and CONH₂.
12. The compound of any one of the preceding clauses, wherein R¹ is H or halogen.
13. The compound of any one of the preceding clauses, wherein R² is H, OCF₃, or halogen.
14. The compound of any one of the preceding clauses, wherein R³ is H, OCF₃, or halogen.
15. The compound of any one of the preceding clauses, wherein R⁴ is H or halogen.
16. The compound of Clause 1, wherein the compound is a compound of the formula: 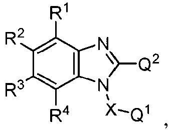 a tautomer thereof, a stereoisomer thereof, a hydrate thereof, or a pharmaceutically acceptable salt thereof, wherein:
   Q¹ is an optionally substituted C6-C10 aryl; optionally substituted C5-C10 heteroaryl; optionally substituted C5-C10 heterocyclyl; optionally substituted C1-C10 alkyl, or optionally substituted C3-C10 cycloalkyl;
   Q² is an optionally substituted C6-C14 aryl; optionally substituted C5-C10 heteroaryl; optionally substituted C5-C10 heterocyclyl; optionally substituted C1-C10 alkyl, or optionally substituted C3-C10 cycloalkyl;
   X is CO or S(O)₂;
   R1, R², R³, and R⁴ are independently H, halogen, CN, optionally substituted C1-C10 alkyl, optionally substituted C3-C10 cycloalkyl, optionally substituted C1-C6 alkoxy, SO₂R⁵, CO2R⁵, or CONR⁵R⁶, or any one of R¹ and R², R² and R³, and R³ and R⁴ pairs, together with the carbon atoms to which they are attached, forms an optionally substituted a five- or six-membered cycloalkenyl, heterocyclenyl, aryl or heteroaryl; and
   R⁵ and R⁶ are independently H, optionally substituted C1-C10 alkyl, or optionally substituted C3-C10 cycloalkyl, or R⁵ and R⁶, together with the nitrogen atom to which they are attached, form an optionally substituted 5-membered ring or an optionally substituted 6-membered ring.
17. The compound of Clause 16, wherein X is CO.
18. The compound of Clause 16, wherein X is SO₂.
19. The compound of Clause 16, wherein the compound is a compound of the formula: 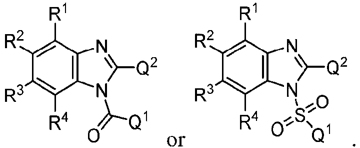
20. The compound of any one of the preceding clauses, wherein Q² is a 1-naphthyl.
21. The compound of any one of the preceding clauses, wherein the compound is: 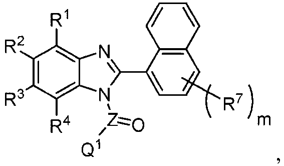 a tautomer thereof, a stereoisomer thereof, a hydrate thereof, or a pharmaceutically acceptable salt thereof, wherein:
   Q¹ is an optionally substituted C6-C10 aryl; optionally substituted C5-C10 heteroaryl; optionally substituted C5-C10 heterocyclyl; optionally substituted C1-C10 alkyl, or optionally substituted C3-C10 cycloalkyl;
   Z is C or SO;
   R¹, R², R³, and R⁴ are independently H, halogen, CN, optionally substituted C1-C10 alkyl, optionally substituted C3-C10 cycloalkyl, optionally substituted C1-C6 alkoxy, SO₂R⁵, CO₂R⁵, or CONR⁵R⁶, or any one of R¹ and R², R² and R³, and R³ and R⁴ pairs, together with the carbon atoms to which they are attached, forms an optionally substituted a five- or six-membered cycloalkenyl, heterocyclenyl, aryl or heteroaryl;
   R⁵ and R⁶ are independently H, optionally substituted C1-C10 alkyl, or optionally substituted C3-C10 cycloalkyl, or R⁵ and R⁶, together with the nitrogen atom to which they are attached, form an optionally substituted 5-membered ring or an optionally substituted 6-membered ring;
   R⁷, at each occurrence, is independently H, halogen, CN, optionally substituted C1-C10 alkyl, optionally substituted C2-C6 alkenyl, optionally substituted C1-C10 heteroalkyl, optionally substituted C1-C10 heterocyclyl, optionally substituted C6-C10 aryl, optionally substituted C5-C10 heteroaryl, optionally substituted C1-C6 alkoxy, optionally substituted C1-C6 cycloalkyloxy, OCF₃, NR⁵R⁶, SCF₃, or C(O)NR⁵R⁶; and
   m is an integer ranging from 1 to 7.
22. The compound of Clause 21, wherein Q¹ is an optionally substituted phenyl, an optionally substituted naphthyl, an optionally substituted optionally substituted C3-C6 cycloalkyl or an optionally substituted quinolinyl.
23. The compound of Clause 22, wherein Q¹ is a phenyl optionally substituted with one, two, or three substituents independently selected from F, Cl, Br, OCH₃, CN, OCF₃, SCF₃, t-Bu, NMe₂, CONH₂, piperazyl, piperidyl, OCH₂CH₂OH, OCH₂CH₂NMe₂, and 1-naphthyl.
23. The compound of any one of the preceding clauses, wherein R¹ is H or halogen.
24. The compound of any one of the preceding clauses, wherein R⁴ is H or halogen.
25. The compound of any one of the preceding clauses, wherein all R⁷ are H.
26. The compound of Clause 16, wherein the compound is: 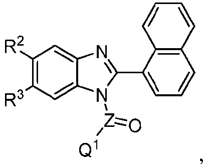
   a tautomer thereof, a stereoisomer thereof, a hydrate thereof, or a pharmaceutically acceptable salt thereof,
   wherein R² and R³, independently, F, Cl, Br, O(C1-C5 alkyl), SCF₃, OCF₃, CO₂H, CO₂(C1-C5 alkyl), or CONR⁵R⁶, wherein R⁵ and R⁶ are independently H, optionally substituted C1-C10 alkyl, optionally substituted C3-C10 cycloalkyl, or R⁵ and R⁶, together with the nitrogen atom to which they are attached, form an optionally substituted morpholinyl; and
   Q¹ is an optionally substituted C6-C10 aryl; optionally substituted C5-C10 heteroaryl; optionally substituted C5-C10 heterocyclyl; optionally substituted C1-C10 alkyl, or optionally substituted C3-C10 cycloalkyl.
27. The compound of Clause 26, wherein Q¹ is a phenyl, cyclopropyl, naphthyl, benzodioxanyl, or quinolinyl, each of which is optionally substituted with one, two, or three substituents independently selected from the group consisting of F, Cl, Br, CF₃, SCF₃, CN, and OCH₃.
28. The compound of any one of the preceding clauses, wherein the compound is a compound of Table 1.
29. A compound of the formula: 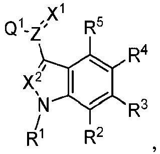 a tautomer thereof, a stereoisomer thereof, a hydrate thereof, or a pharmaceutically acceptable salt thereof, wherein:
   Q¹ is an optionally substituted C6-C10 aryl; optionally substituted C5-C10 heteroaryl; optionally substituted C5-C10 heterocyclyl; optionally substituted C1-C10 alkyl, or optionally substituted C3-C10 cycloalkyl;
   Z is C, S(O), 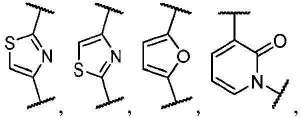 or Z is CH when X¹ is absent, or Z(X¹)Q¹ is H;
   X¹ is absent, O, NH, S, or X¹ is 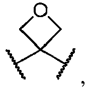 wherein the wavy lines denote points of attachment to Z;
   X² is N or CQ²;
   Q² is H, CN, CONH₂, COOH, CF₃, halogen, optionally substituted C1-C10 alkyl, optionally substituted C3-C10 cycloalkyl, optionally substituted C3-C10 heteroalkyl, optionally substituted C3-C10 hetercyclyl, optionally substituted C6-C14 aryl, or optionally substituted C5-C14 heteroaryl;
   R¹ is H, optionally substituted C1-C10 alkyl, optionally substituted C3-C10 cycloalkyl, optionally substituted C3-C10 heteroalkyl, optionally substituted C3-C10 hetercyclyl, optionally substituted C6-C10 aryl, optionally substituted C5-C10 heteroaryl, or C1-C12 acyl;
   R², R³, R⁴, and R⁵ are independently H, halogen, CN, optionally substituted C1-C10 alkyl, optionally substituted C3-C10 cycloalkyl, optionally substituted C1-C6 alkoxy, SO₂R⁷, CO₂R⁷, or CONR⁷R⁶, or any one of R² and R³, R³ and R⁴, and R⁴ and R⁵ pairs, together with the carbon atoms to which they are attached, forms an optionally substituted a five- or six-membered cycloalkenyl, heterocyclenyl, aryl or heteroaryl; and
   R⁶ and R⁷ are independently H, optionally substituted C1-C10 alkyl, or optionally substituted C3-C10 cycloalkyl, or R⁶ and R⁷, together with the nitrogen atom to which they are attached, form an optionally substituted 5-membered ring or an optionally substituted 6-membered ring.
30. The compound of Clause 29, wherein X² is CH, CF, or CCl.
31. The compound of Clause 29 or Clause 30, wherein Z is C.
32. The compound of Clause 29, wherein the compound is a compound of the formula: 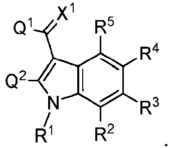
33. The compound of any one of Clauses 29 to 31, wherein X¹ is O.
34. The compound of any one of Clauses 29 to 33, wherein the compound has the formula: 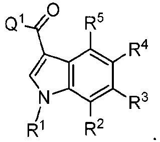
35. The compound of any one of Clauses 29 to 34, wherein R¹ is H, CH₃, or C(O)R⁸, wherein R⁸ is H, an optionally substituted C1-C10 alkyl, optionally substituted C3-C10 cycloalkyl, optionally substituted C3-C10 heteroalkyl, optionally substituted C3-C10 hetercyclyl, optionally substituted C6-C10 aryl, or optionally substituted C5-C10 heteroaryl.
36. The compound of any one of Clause 29 to Clause 35, wherein Q¹ is: 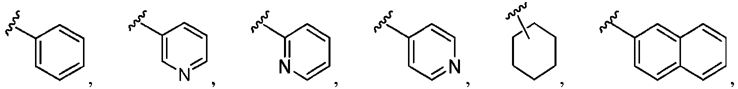 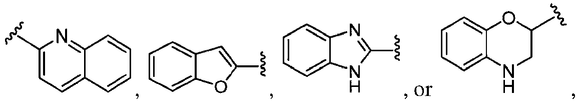 each of which is optionally substituted with one, two, three, or four substituents independently selected from H, halogen, CN, optionally substituted C1-C10 alkyl, optionally substituted C2-C6 alkenyl, optionally substituted C1-C10 heteroalkyl, optionally substituted C6-C10 aryl, optionally substituted C1-C6 alkoxy, optionally substituted C1-C6 cycloalkyloxy, OCF₃, NR⁷R⁶, SCF₃, and C(O)NR⁷R⁶, where R⁶ and R⁷ are as defined above.
37. The compound of any one of Clauses 29 to 36, wherein Q¹ is a phenyl optionally substituted with one or two substituents independently selected from F, Cl, OCH₃, CN, OCF₃, SCF₃, t-Bu, NMe₂, CONH₂, 1-piperazyl, OCH₂CH₂OH, OCH₂CH₂NMe₂, and 1-naphthoyl.
37. The compound of Clause 29, wherein Z(X¹)Q¹ is H.
38. The compound of Clause 37, wherein the compound is represented by Formula IID: 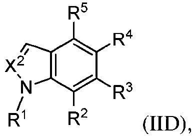 a tautomer thereof, a stereoisomer thereof, a hydrate thereof, or a pharmaceutically acceptable salt thereof, wherein:
   X² is N, CCl, CF, CBr, CI, CCN, CCONH₂, CCOOH, CH, or CQ², wherein Q² is optionally substituted C6-C10 aryl, optionally substituted C5-C10 heteroaryl, optionally substituted C6-C10 aryl, optionally substituted C3-C10 cycloalkyl, or optionally substituted C3-C10 hetercyclyl; and R¹, R², R³, R⁴, and R⁵ are independently as defined for Formula II.
39. The compound of Clause 37 or Clause 38, wherein X² is CQ², wherein Q² is optionally substituted C6-C10 aryl, optionally substituted C5-C10 heteroaryl, optionally substituted C6-C10 aryl, optionally substituted C3-C10 cycloalkyl, or optionally substituted C3-C10 hetercyclyl.
39. The compound of any one of Clauses 37-39, wherein X² is CQ², wherein Q² is optionally substituted phenyl, optionally substituted naphthyl, optionally substituted quinolinyl, optionally substituted cyclopropyl, or optionally substituted cyclohexyl.
40. The compound of Clause 37, wherein the compound represented by Formula IIE: 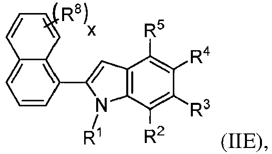 a tautomer thereof, a stereoisomer thereof, a hydrate thereof, or a pharmaceutically acceptable salt thereof, wherein:
   R¹, R², R³, R⁴, and R⁵ are independently as defined for Formula II; R⁸, at each occurrence, is independently H, halogen, CN, optionally substituted C1-C10 alkyl, optionally substituted C3-C8 cycloalkyl, optionally substituted C2-C6 alkenyl, optionally substituted C2-C6 alkynyl, optionally substituted C3-C10 heteroalkyl, optionally substituted C3-C10 heterocyclyl, optionally substituted C6-C10 aryl, optionally substituted C5-C10 heteroaryl, optionally substituted C1-C6 alkoxy, optionally substituted C3-C8 cycloalkyloxy, OCF₃, CF₃, NR'R", SCF₃, or C(O)NR^{'}R^{"};
   x is an integer ranging from 1-7; and
   R' and R" are H, optionally substituted C1-C10 alkyl, optionally substituted C3-C8 cycloalkyl, optionally substituted C2-C6 alkenyl, optionally substituted C2-C6 alkynyl, or optionally substituted C3-C10 heteroalkyl; or R^{'} and R^{"}, together with the nitrogen atom to which they are attached, form an optionally substituted 5-membered ring or an optionally substituted 6-membered ring.
41. The compound of Clause 40, wherein R¹ is H or an optionally substituted C1-C10 alkyl.
42. The compound of Clause 40, wherein R¹ is H.
43. The compound of any one of Clauses 40-42, wherein all R⁸ are H or halogen.
44. The compound of any one of Clauses 29 to 43, wherein R² is H or halogen.
45. The compound of any one of Clauses 29 to 44, wherein R³ is H or halogen.
46. The compound of any one of Clauses 29 to 45, wherein R⁴ is H or halogen.
47. The compound of any one of Clauses29 to 46, wherein R⁵ is H or halogen.
48. The compound of any one of Clauses 29 to 47, wherein the compound is a compound of Table 2.
49. The compound of any one of Clauses 1-48, wherein the compound activates AhR at 0.1 nanomolar, 1 nanomolar, 10 nanomolar, 100 nanomolar, 1 micro molar, 10 micro molar, or 100 micromolar concentrations.
50. A method of treating an autoimmune disease treatable by administering a therapeutically effective amount of an aryl hydrocarbon receptor (AhR) ligand to a subject in need thereof, wherein the aryl hydrocarbon receptor (AhR) ligand is a compound of any one of Clauses 1-49.
52. The method of Clause 50, wherein the autoimmune disease is diabetes mellitus type 1.
53. The method of Clause 50, wherein the autoimmune disease is graft versus host disease.
54. The method of Clause 50, wherein the autoimmune disease is Celiac disease, autoimmune hepatitis, autoimmune pancreatitis, Crohn's disease, interstitial cystitis, microscopic colitis, or ulcerative colitis.
55. The method of Clause 50, wherein the autoimmune disease is alopecia areata, atopic dermatitis, cicatricial pemphigoid, dermatomyositis, dermatitis herpetiformis, lichen planus, pemphigus vulgaris, or psoriasis.
56. The method of any one of Clauses 50-55, wherein the aryl hydrocarbon receptor (AhR) ligand is administered topically or systemically.
57. The method of any one of Clauses 50-55, wherein the aryl hydrocarbon receptor (AhR) ligand is administered orally, topically, intravenously, or subcutaneously.
58. The method of any one of Clauses 50-57, further including administering the AhR ligand with a pharmaceutically acceptable carrier.
59. The method of Clause 58, wherein the AhR ligand is formulated within a nanoparticle.
60. A pharmaceutical composition comprising an AhR ligand of any one of Clauses 1-49.

## Claims

1. A compound of the formula: 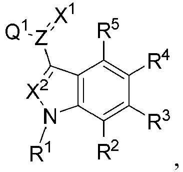 or a tautomer thereof, a stereoisomer thereof, a hydrate thereof, or a pharmaceutically acceptable salt thereof, wherein:
Q¹ is an optionally substituted C6-C10 aryl; optionally substituted C5-C10 heteroaryl; optionally substituted C5-C10 heterocyclyl; optionally substituted C1-C10 alkyl, or optionally substituted C3-C10 cycloalkyl;
Z is C, S(O), 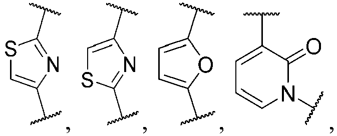 or Z is CH when X¹ is absent, or Z(X¹)Q¹ is H;
X¹ is absent, O, NH, S, or X¹ is 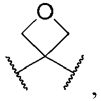 wherein the wavy lines denote points of attachment to Z;
X² is CQ²;
Q² is H, CN, CONH₂, COOH, CF₃, halogen, optionally substituted C1-C10 alkyl, optionally substituted C3-C10 cycloalkyl, optionally substituted C3-C10 heteroalkyl, optionally substituted C3-C10 hetercyclyl, optionally substituted C6-C14 aryl, or optionally substituted C5-C14 heteroaryl;
R¹ is H, optionally substituted C1-C10 alkyl, optionally substituted C3-C10 cycloalkyl, optionally substituted C3-C10 heteroalkyl, optionally substituted C3-C10 hetercyclyl, optionally substituted C6-C10 aryl, optionally substituted C5-C10 heteroaryl, or C1-C12 acyl;
R², R³, R⁴, and R⁵ are independently H, halogen, CN, optionally substituted C1-C10 alkyl, optionally substituted C3-C10 cycloalkyl, optionally substituted C1-C6 alkoxy, SO₂R⁷, CO₂R⁷, or CONR⁷R⁶, or any one of R² and R³, R³ and R⁴, and R⁴ and R⁵ pairs, together with the carbon atoms to which they are attached, forms an optionally substituted a five- or six-membered cycloalkenyl, heterocyclenyl, aryl or heteroaryl; and
R⁶ and R⁷ are independently H, optionally substituted C1-C10 alkyl, or optionally substituted C3-C10 cycloalkyl, or R⁶ and R⁷, together with the nitrogen atom to which they are attached, form an optionally substituted 5-membered ring or an optionally substituted 6-membered ring.

2. The compound of Claim 1, wherein X² is CH, CF, or CCl.

3. The compound of Claim 1, wherein the compound is a compound of the formula:

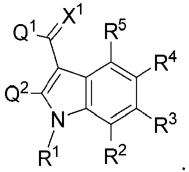

4. The compound of any one of Claims 1 to 3, wherein the compound has the formula:

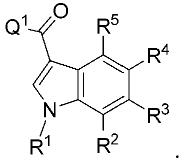

5. The compound of any one of Claims 1 to 4, wherein R¹ is H, CH₃, or C(O)R⁸, wherein R⁸ is H, an optionally substituted C1-C10 alkyl, optionally substituted C3-C10 cycloalkyl, optionally substituted C3-C10 heteroalkyl, optionally substituted C3-C10 hetercyclyl, optionally substituted C6-C10 aryl, or optionally substituted C5-C10 heteroaryl.

6. The compound of any one of Claims 1 to 5, wherein Q¹ is: 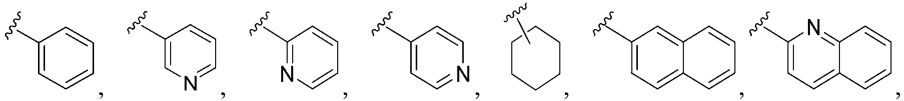 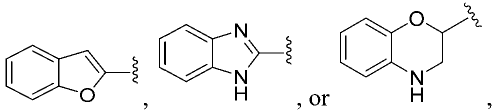
each of which is optionally substituted with one, two, three, or four substituents independently selected from H, halogen, CN, optionally substituted C1-C10 alkyl, optionally substituted C2-C6 alkenyl, optionally substituted C1-C10 heteroalkyl, optionally substituted C6-C10 aryl, optionally substituted C1-C6 alkoxy, optionally substituted C1-C6 cycloalkyloxy, OCF₃, NR⁷R⁶, SCF₃, and C(O)NR⁷R⁶;
preferably wherein Q¹ is a phenyl optionally substituted with one or two substituents independently selected from F, Cl, OCH₃, CN, OCF₃, SCF₃, t-Bu, NMe₂, CONH₂, 1-piperazyl, OCH₂CH₂OH, OCH₂CH₂NMe₂, and 1-naphthoyl.

7. The compound of Claim 1, wherein the compound is represented by Formula IID: 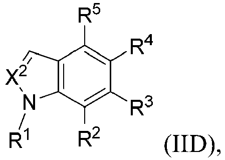 or a tautomer thereof, a stereoisomer thereof, a hydrate thereof, or a pharmaceutically acceptable salt thereof, wherein:
X² is CCl, CF, CBr, Cl, CCN, CCONH₂, CCOOH, CH, or CQ², wherein Q² is optionally substituted C6-C10 aryl, optionally substituted C5-C10 heteroaryl, optionally substituted C6-C10 aryl, optionally substituted C3-C10 cycloalkyl, or optionally substituted C3-C10 hetercyclyl.

8. The compound of Claim 1 or Claim 7, wherein X² is CQ², Q² is optionally substituted C6-C10 aryl, optionally substituted C5-C10 heteroaryl, optionally substituted C6-C10 aryl, optionally substituted C3-C10 cycloalkyl, or optionally substituted C3-C10 hetercyclyl;
preferably wherein X² is CQ², Q² is optionally substituted phenyl, optionally substituted naphthyl, optionally substituted quinolinyl, optionally substituted cyclopropyl, or optionally substituted cyclohexyl.

9. The compound of Claim 1, wherein the compound represented by Formula IIE: 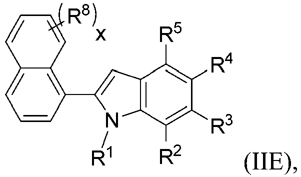 or a tautomer thereof, a stereoisomer thereof, a hydrate thereof, or a pharmaceutically acceptable salt thereof, wherein:
R⁸, at each occurrence, is independently H, halogen, CN, optionally substituted C1-C10 alkyl, optionally substituted C3-C8 cycloalkyl, optionally substituted C2-C6 alkenyl, optionally substituted C2-C6 alkynyl, optionally substituted C3-C10 heteroalkyl, optionally substituted C3-C10 heterocyclyl, optionally substituted C6-C10 aryl, optionally substituted C5-C10 heteroaryl, optionally substituted C1-C6 alkoxy, optionally substituted C3-C8 cycloalkyloxy, OCF₃, CF₃, NR'R", SCF₃, or C(O)NR'R";
x is an integer ranging from 1-7; and
R' and R" are H, optionally substituted C1-C10 alkyl, optionally substituted C3-C8 cycloalkyl, optionally substituted C2-C6 alkenyl, optionally substituted C2-C6 alkynyl, or optionally substituted C3-C10 heteroalkyl; or R^{'} and R^{"}, together with the nitrogen atom to which they are attached, form an optionally substituted 5-membered ring or an optionally substituted 6-membered ring.

10. The compound of any one of Claims 1 to 9, wherein R², R³, R⁴, and R⁵ are independently H or halogen.

11. The compound of any one of Claims 1 to 10, wherein the compound is selected from the group consisting of: 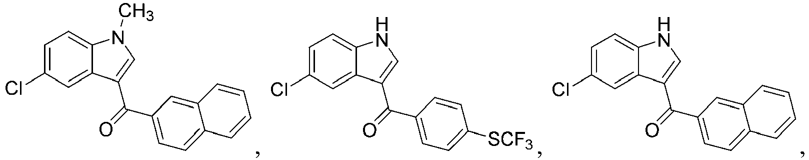 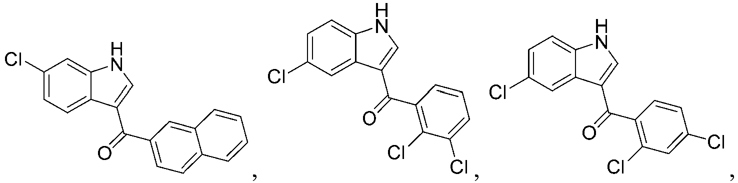 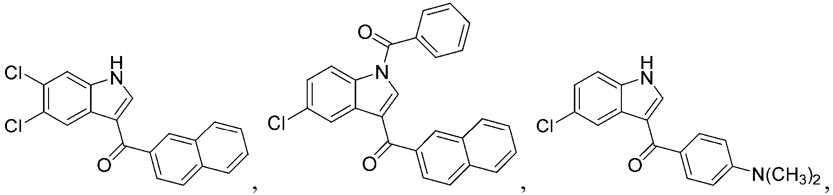 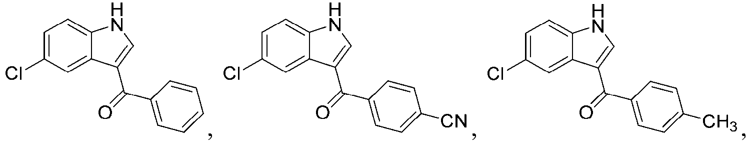 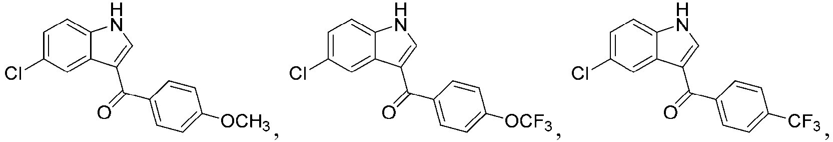 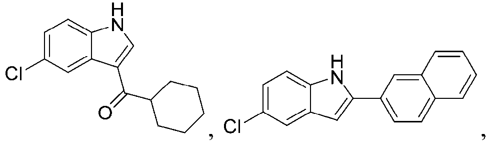 and
tautomers, stereoisomers, hydrates, or pharmaceutically acceptable salts thereof.

12. An aryl hydrocarbon receptor (AhR) ligand of any one of the preceding claims for use in a method of treating an autoimmune disease treatable by administering a therapeutically effective amount of the aryl hydrocarbon receptor (AhR) ligand to a subject in need thereof.

13. The AhR for use of Claim 12, wherein the autoimmune disease is diabetes mellitus type 1, graft versus host disease, Celiac disease, autoimmune hepatitis, autoimmune pancreatitis, Crohn's disease, interstitial cystitis, microscopic colitis, ulcerative colitis, alopecia areata, atopic dermatitis, cicatricial pemphigoid, dermatomyositis, dermatitis herpetiformis, lichen planus, pemphigus vulgaris, or psoriasis.

14. The AhR for use of any one of Claims 12-13, wherein the aryl hydrocarbon receptor (AhR) ligand is administered topically or systemically (e.g., orally, intravenously, or subcutaneously).

15. A pharmaceutical composition comprising an AhR ligand of any one of Claims 1-11.
